# EUROPÄISCHE PATENTSCHRIFT

(11) **EP 1 200 428 B1**
(45) Veröffentlichungstag und Bekanntmachung des Hinweises auf die Patenterteilung: **16.04.2003**
(21) Anmeldenummer: 00958421.0
(22) Anmeldetag: 10.08.2000
(51) Int. Cl.: C07D 413/04, C07D 263/56, A01N 43/653, A01N 43/836, A01N 43/76

(54) **SUBSTITUIERTE BENZOXAZOLE**
SUBSTITUTED BENZOXAZOLES
BENZOXAZOLES SUBSTITUES

(30) Priorität: 12.08.1999 DE 19938073
(43) Veröffentlichungstag der Anmeldung: 02.05.2002
(73) Patentinhaber: BASF AKTIENGESELLSCHAFT, 67056 Ludwigshafen (DE)
(72) Erfinder: REINHARD, Robert, 67061 Ludwigshafen (DE); HAMPRECHT, Gerhard, 69469 Weinheim (DE); MENKE, Olaf, 67317 Altleiningen (DE); PUHL, Michael, 68623 Lampertheim (DE); SAGASSER, Ingo, 67125 Dannstadt (DE); ZAGAR, Cyrill, 67061 Ludwigshafen (DE); OTTEN, Martina, 67069 Ludwigshafen (DE); WESTPHALEN, Karl-Otto, 67346 Speyer (DE); WALTER, Helmut, 67283 Obrigheim (DE)
(74) Vertreter: Kinzebach, Werner, Dr.
(86) Internationale Anmeldenummer: EP0007803
(87) Internationale Veröffentlichungsnummer: WO01012625

(56) Entgegenhaltungen:
- WO-A-00/28822
- WO-A-97/08170
- WO-A-97/08171
- WO-A-97/12886
- WO-A-98/38188
- WO-A-99/31091
- CHEMICAL ABSTRACTS, vol. 128, no. 4, 26. Januar 1998 (1998-01-26) Columbus, Ohio, US; abstract no. 34781, ITO, MINORU ET AL: "Preparation of 2H-chromenes and their use as herbicides" XP002158256 & JP 09 301973 A (KUMIAI CHEMICAL INDUSTRY CO., LTD., JAPAN;IHARA CHEMICAL INDUSTRY CO.,) 25. November 1997 (1997-11-25)
- STEVENS M.F.G. ET AL.: "Introduction of a triflate group into sterically hindered positions in 1-aryl-4,6-diamino-1,3,5- triazines and their Dimroth rearrangement products" JOURNAL OF HETEROCYCLIC CHEMISTRY., Bd. 30, Nr. 4, 1993, Seiten 849-853, XP002158746 HETEROCORPORATION. PROVO., US ISSN: 0022-152X

## Beschreibung

Die vorliegende Erfindung betrifft substituierte Benzoxazole der allgemeinen Formel I 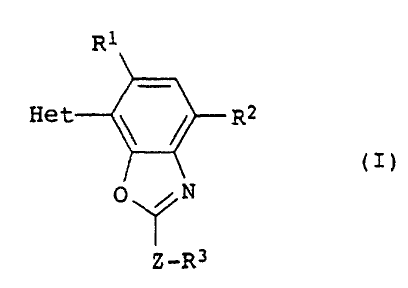 worin
- Z: eine chemische Bindung, O oder S bedeutet,
- R¹: für Wasserstoff oder Halogen,
- R²: für Halogen, Cyano, C₁-C₆-Alkyl, C₁-C₆-Halogenalkyl, C₁-C₆-Alkoxy oder C₁-C₆-Halogenalkoxy, und
- R³: für C₁-C₆-Alkyl, C₁-C₆-Halogenalkyl, Hydroxy-C₁-C₄-alkyl, Cyano-C₁-C₄-alkyl-, C₁-C₄-Alkoxy-C₁-C₄-alkyl, C₁-C₄-Halogenalkoxy-C₁-C₄-alkyl, C₃-C₄-Alkenyloxy-C₁-C₄-alkyl, C₃-C₄-Alkinyloxy-C₁-C₄-alkyl, C₃-C₈-Cycloalkoxy-C₁-C₄-alkyl,
Amino-C₁-C₄-alkyl, C₁-C₄-Alkylamino-C₁-C₄-alkyl, Di(C₁-C₄-alkyl)amino-C₁-C₄-alkyl, C₁-C₄-Alkylthio-C₁-C₄-alkyl, C₁-C₄-Halogenalkylthio-C₁-C₄-alkyl, C₃-C₆-Alkenyl, Cyano-C₃-C₆-alkenyl, C₃-C₆-Halogenalkenyl, C₃-C₆-Alkinyl, Cyano-C₃-C₆-alkinyl, C₃-C₆-Halogenalkinyl, Hydroxycarbonyl-C₁-C₄-alkyl, (C₁-C₄-Alkoxy)carbonyl-C₁-C₄-alkyl, (C₁-C₄-Alkylthio)carbonyl-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl, Di(C₁-C₄-alkyl)phosphonyl-C₁-C₄-alkyl;
Phenyl, Phenyl-C₁-C₄-alkyl, worin die Phenylringe gegebenenfalls einen, zwei oder drei Substituenten tragen, ausgewählt unter Halogen, Cyano, C₁-C₆-Alkyl, C₁-C₆-Halogenalkyl, C₁-C₆-Alkoxy oder C₁-C₆-Halogenalkoxy;
C₃-C₈-Cycloalkyl, C₃-C₈-Cycloalkyl-C₁-C₄-alkyl, 3- bis 7-gliedriges Heterocyclyl, 3- bis 7-gliedriges Heterocyclyl-C₁-C₄alkyl, wobei Heterocyclyl ein, zwei oder drei Heteroatome, ausgewählt unter Sauerstoff-, Schwefel- und Stickstoff-Atomen enthält, wobei jeder Cycloalkyl- und jeder Heterocyclyl-Ring ein Carbonyl- oder Thiocarbonyl-Ringglied enthalten kann, und worin jeder Cycloalkyl- und Heterocyclyl-Ring unsubstituiert sein oder ein, zwei, drei oder vier Substituenten tragen kann, jeweils ausgewählt unter Cyano, Nitro, Amino, Hydroxy, Halogen, C₁-C₄-Alkyl, C₁-C₄-Halogenalkyl, C₁-C₄-Cyanoalkyl, C₁-C₄-Hydroxyalkyl, C₁-C₄-Aminoalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy, Di(C₁-C₄-alkyl)amino, C₃-C₆-Alkenyl, C₃-C₆-Alkinyl, C₃-C₄-Alkenyloxy, C₃-C₄-Alkenylthio, C₃-C₄-Alkinyloxy und C₃-C₄-Alkinylthio; stehen,
oder, sofern Z eine chemische Bindung bedeutet, R³ gewünschtenfalls auch für Wasserstoff, Hydroxy, Cyano, Mercapto, Amino, C₁-C₄-Alkylamino, Di-C₁-C₄-alkylamino, gesättigtes, 5- oder 6-gliedriges, N-gebundenes Stickstoffheterocyclyl, C₃-C₆-Cycloalkylamino, Halogen, -(CH₂)ₙ-CH(OH)-CH₂-R⁹, -(CH₂)ₙ-CH(Halogen)-CH₂-R⁹, -(CH₂)ₙ-CH₂-CH(Halogen)-R⁹, -(CH₂)ₙ-CH=CH-R⁹ oder -(CH₂)ₙ-CH=C(Halogen)-R⁹ stehen kann, worin R⁹ Hydroxycarbonyl, (C₁-C₄-Alkoxy)carbonyl, (C₁-C₄-Alkylthio)carbonyl, Aminocarbonyl, (C₁-C₄-Alkylamino)carbonyl oder Di(C₁-C₄-alkyl)aminocarbonyl bedeutet und n für 0 oder 1 steht;
- Het: für einen ungesättigten, fünf- oder sechsgliedrigen, heterocylischen Rest, der mit dem Benzoxazolteil über ein Stickstoffatom verknüpft ist und der ausgewählt ist unter Resten der allgemeinen Formeln II-1 bis II-18, 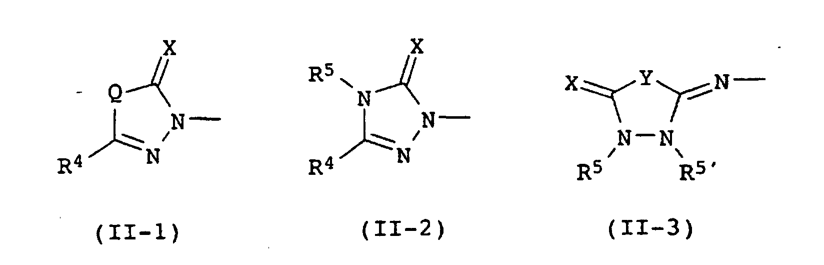 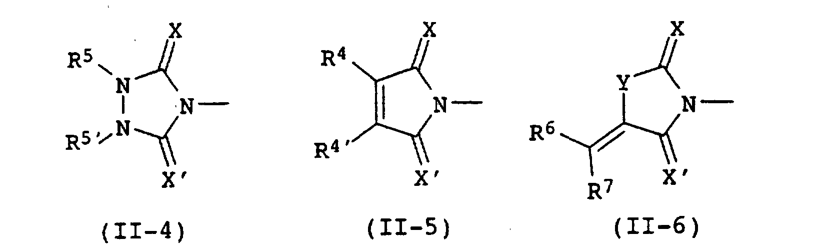 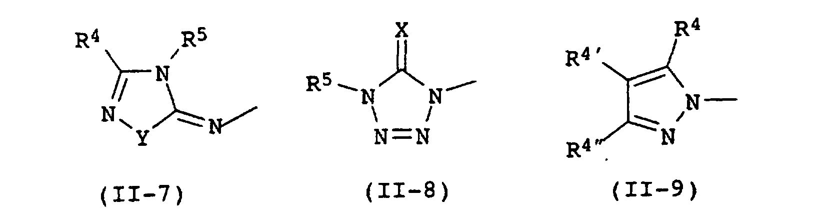 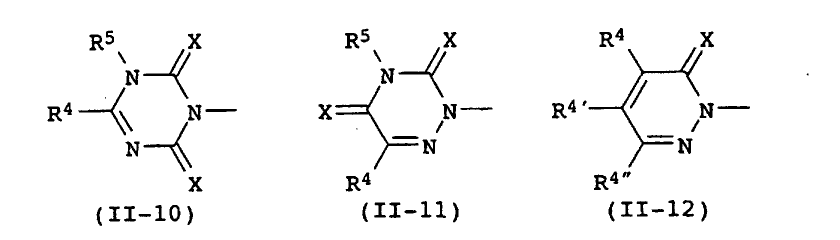 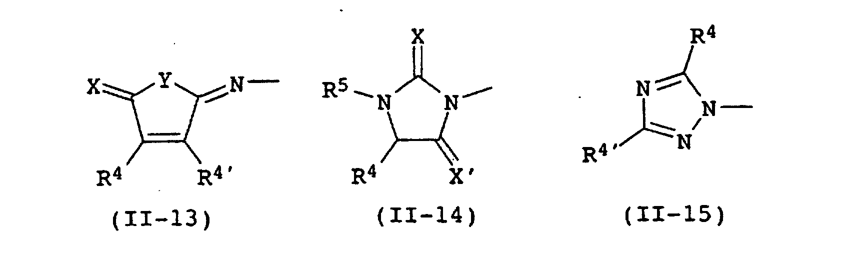 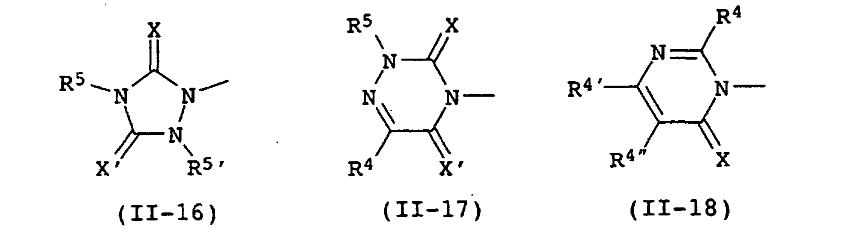
worin
- R⁴, R^{4'} und R^{4"}: unabhängig voneinander Wasserstoff, Amino, C₁-C₄-Alkylamino, Di-C₁-C₄-alkylamino, gesättigtes, 5- oder 6-gliedriges, N-gebundenes Stickstoffheterocyclyl, C₃-C₆-Cycloalkylamino, Halogen, Cyano, Carboxyl, C₁-C₆-Alkyl, C₁-C₆-Halogenalkyl, C₃-C₆-Alkenyl, C₃-C₆-Alkinyl, C₁-C₆-Alkoxy, C₁-C₆-Halogenalkoxy, C₁-C₆-Halogenalkylthio, C₁-C₆-Alkoxycarbonyl, C₃-C₆-Alkenyloxy, C₃-C₆-Alkinyloxy, C₁-C₆-Alkylthio, C₁-C₆-Alkylsulfinyl, C₁-C₆-Alkylsulfonyl, C₃-C₈-Cycloalkyl oder C₃-C₈-Cycloalkyl-C₁-C₄-alkyl bedeuten;
- R⁵ und R^{5'}: unabhängig voneinander Wasserstoff, Amino, Hydroxy, Cyano, C₁-C₆-Alkyl, C₁-C₆-Halogenalkyl, C₃-C₆-Alkenyl, C₃-C₆-Alkinyl, C₁-C₆-Alkoxycarbonyl, C₃-C₈-Cycloalkyl, C₃-C₈-Cycloalkyl-C₁-C₄-alkyl, Phenyl oder Phenyl-C₁-C₆-alkyl bedeuten;
und/oder zwei der Reste R⁴, R^{4'}, R^{4"}, R⁵ und R^{5'} gemeinsam mit dem Cyclus an den sie gebunden sind, einen 4-, 5-, 6- oder 7-gliedrigen Ring bilden, der gesättigt oder ungesättigt sein kann, der ein oder zwei Sauerstoff- und/oder Schwefelatome als Ringlied enthalten kann, und/oder der durch ein, zwei oder drei Reste ausgewählt unter C₁-C₄-Alkyl und Halogen substituiert sein kann;
- R⁶ und R⁷: unabhängig voneinander Wasserstoff, C₁-C₆-Alkyl, C₁-C₆-Halogenalkyl oder C₃-C₆-Cycloalkyl bedeuten, oder gemeinsam mit dem C-Atom, an das sie gebunden sind, einen 4-, 5-, 6-oder 7-gliedrigen Ring bilden, der gesättigt oder ungesättigt sein kann, der ein oder zwei Sauerstoff- und/oder Schwefelatome als Ringlied enthalten kann, und/oder durch ein, zwei oder drei Substituenten, ausgewählt unter C₁-C₄-Alkyl und Halogen substituiert sein kann;
- Q: xin Formel II-1 für O oder S steht,
- X und X': unabhängig voneinander für O oder S stehen, und
- Y: O, S oder eine Gruppe N-R⁸ bedeutet, worin R⁸ für Wasserstoff, C₁-C₆-Alkyl, C₁-C₆-Halogenalkyl oder C₃-C₈-Cycloalkyl steht;
sowie die landwirtschaftlich brauchbaren Salze der Verbindungen der Formel I.

Außerdem betrifft die Erfindung
- die Verwendung der Verbindungen I als Herbizide,
- Mittel, welche die Verbindungen I als herbizid wirksame Substanzen enthalten,
- Verfahren zur Bekämpfung von unerwünschtem Pflanzenwuchs mit den Verbindungen der Formel I, sowie
- Verbindungen der allgemeinen Formeln III, IV, V und X als Zwischenprodukte zur Herstellung der Verbindungen I.

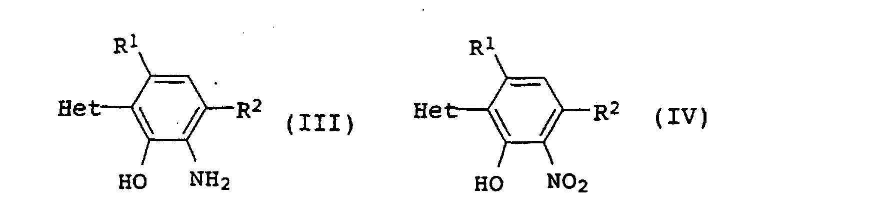

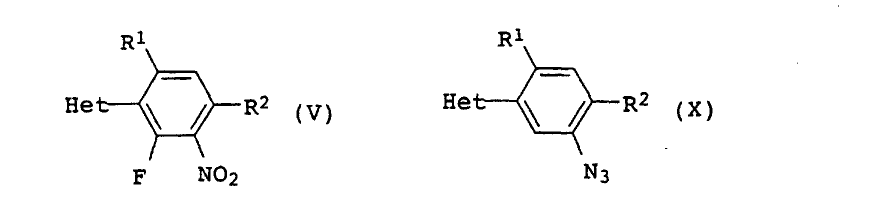

In der WO 97/08170 werden 3-(Benzoxazol-7-yl)- und 3-(Benzthiazol-7-yl)-6-(trifluormethyl)uracile als Herbizide beschrieben. Weitere 3-(Benzthiazol-7-yl)uracile sowie deren Verwendung als Herbizide und zur Desikkation/Defoliation von Pflanzen werden in der WO 97/08171 gelehrt. Gegenstand der WO 97/12886 sind u.a. bestimmte 3-Benzisoxazol-7-yl-2,4-(1H,3H)pyrimidindione, denen eine herbizide und desikkante Wirkung zugeschrieben wird. Weiterhin sind aus der DE-A 197 55 926 Benzazol-4-ylpyrimidindione und aus der älteren deutschen Patentanmeldung P 198 52 802.7 Derivate von 3-(Benzazol-7-yl)pyrimidindionen bekannt.

Der vorliegenden Erfindung liegt die Aufgabe zugrunde, neue herbizid wirksame Verbindungen bereitzustellen, mit denen sich unerwünschte Pflanzen besser als mit den bekannten gezielt bekämpfen lassen.

Diese Aufgabe wird gelöst durch die eingangs definierten Derivate des Benzoxazols, die in der 7-Position einen über ein Stickstoffatom gebundenen 5- oder 6-gliedrigen Heterocyclus aufweisen.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Die bei der Definition der Substituenten R¹ bis R⁹ oder als Reste an Cycloalkyl-, Phenyl- oder heterocyclischen Ringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Cyanoalkyl-, Hydroxyalkyl-, Aminoalkyl-, Hydroxycarbonylalkyl-, Aminocarbonylalkyl-, Phenylalkyl-, Heterocyclylalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Alkenyl-, Halogenalkenyl-, Cyanoalkenyl-, Alkenyloxy-, Alkenylthio-, Alkenylsulfinyl-, Alkenylsulfonyl-, Alkinyl-, Halogenalkinyl-, Cyanoalkinyl-, Alkinyloxy-, Alkinylthio-, Alkinylsulfinyl- und Alkinylsulfonyl-Teile können geradkettig oder verzweigt sein. Halogenierte Substituenten tragen vorzugsweise ein bis fünf gleiche oder verschiedene Halogenatome.

Die Bedeutung Halogen steht jeweils für Fluor, Chlor, Brom oder Iod.

Ferner stehen beispielsweise:
- C₁-C₄-Alkyl für: CH₃, C₂H₅, CH₂-C₂H₅, CH(CH₃)₂, n-Butyl, CH(CH₃)-C₂H₅, CH₂-CH(CH₃)₂ oder C(CH₃)₃;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂F, CHF₂, CF₃, CH₂Cl, CH(Cl)₂, C(Cl)₃, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, CH₂-C₂F₅, CF₂-C₂F₅, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Alkyl für: einen C₁-C₄-Alkylrest wie vorstehend genannt, oder z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise für CH₃, C₂H₅, CH₂-C₂H₅, CH(CH₃)₂, n-Butyl, C(CH₃)₃, n-Pentyl oder n-Hexyl;
- C₁-C₆-Halogenalkyl für: einen C₁-C₆-Alkylrest wie vorstehend genannt, der partiell oder vollständig durch Fluor, Chlor, Brom und/oder Iod substituiert ist, also z.B. einen der unter C₁-C₄-Halogenalkyl genannten Reste oder für 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl;
- Cyano-C₁-C₄-alkyl für: CH₂CN, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 4-Cyanobut-2-yl, 1-(CH₂CN)eth-1-yl, 1-(CH₂CN)-1-(CH₃)-eth-1-yl oder 1-(CH₂CN)prop-1-yl;
- Hydroxy-C₁-C₄-alkyl für: CH₂OH, 1-Hydroxyethyl, 2-Hydroxyethyl, 1-Hydroxyprop-1-yl, 2-Hydroxyprop-1-yl, 3-Hydroxyprop-1-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 3-Hydroxybut-2-yl, 4-Hydroxybut-2-yl, 1-(CH₂OH)eth-1-yl, 1-(CH₂OH)-1-(CH₃)-eth-1-yl oder 1-(CH₂OH)prop-1-yl;
- Amino-C₁-C₄-alkyl für: CH₂NH₂, 1-Aminoethyl, 2-Aminoethyl, 1-Aminoprop-1-yl, 2-Aminoprop-1-yl, 3-Aminoprop-1-yl, 1-Aminobut-1-yl, 2-Aminobut-1-yl, 3-Aminobut-1-yl, 4-Aminobut-1-yl, 1-Aminobut-2-yl, 2-Aminobut-2-yl, 3-Aminobut-2-yl, 4-Aminobut-2-yl, 1-(CH₂NH₂)eth-1-yl, 1-(CH₂NH₂)-1-(CH₃)-eth-1-yl oder 1-(CH₂NH₂)prop-1-yl;
- Hydroxycarbonyl-C₁-C₄-alkyl für: CH₂COOH, 1-(COOH)ethyl, 2-(COOH)ethyl, 1-(COOH)prop-1-yl 2-(COOH)prop-1-yl, 3-(COOH)-prop-1-yl, 1-(COOH)but-1-yl, 2-(COOH)but-1-yl, 3-(COOH)but-1-yl, 4-(COOH)but-1-yl, 1-(COOH)but-2-yl, 2-(COOH)but-2-yl, 3-(COOH)but-2-yl, 4-(COOH)but-2-yl, 1-(CH₂COOH)eth-1-yl, 1-(CH₂COOH)-1-(CH₃)-eth-1-yl oder 1-(CH₂COOH)prop-1-yl;
- Aminocarbonyl-C₁-C₄-alkyl für: CH₂CONH₂, 1-(CONH₂)ethyl, 2-(CONH₂)ethyl, 1-(CONH₂)prop-1-yl, 2-(CONH₂)prop-1-yl, 3-(CONH₂)prop-1-yl, 1-(CONH₂)but-1-yl, 2-(CONH₂)but-1-yl, 3-(CONH₂)but-1-yl, 4-(CONH₂)but-1-yl, 1-(CONH₂)but-2-yl, 2-(CONH₂)but-2-yl, 3-(CONH₂)but-2-yl, 4-(CONH₂)but-2-yl, 1-(CH₂CONH₂)eth-1-yl, 1-(CH₂CONH₂)-1-(CH₃)-eth-1-yl oder 1-(CH₂CONH₂)prop-1-yl;
- Phenyl-C₁-C₄-alkyl für: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Benzyl)-eth-1-yl, 1-(Benzyl)-1-(methyl)-eth-1-yl oder 1-(Benzyl)-prop-1-yl, vorzugsweise für Benzyl oder 2-Phenylethyl;
- Heterocyclyl-C₁-C₄-alkyl für: Heterocyclylmethyl, 1-Heterocyclyl-ethyl, 2-Heterocyclyl-ethyl, 1-Heterocyclyl-prop-1-yl, 2-Heterocyclyl-prop-1-yl, 3-Heterocyclyl-prop-1-yl, 1-Heterocyclyl-but-1-yl, 2-Heterocyclyl-but-1-yl, 3-Heterocyclylbut-1-yl, 4-Heterocyclyl-but-1-yl, 1-Heterocyclyl-but-2-yl, 2-Heterocyclyl-but-2-yl, 3-Heterocyclyl-but-2-yl, 3-Heterocyclyl-but-2-yl, 4-Heterocyclyl-but-2-yl, 1-(Heterocycly methyl)-eth-1-yl, 1-(Heterocyclylmethyl)-1-(methyl)-eth-1-yl oder 1-(Heterocyclylmethyl)-prop-1-yl, vorzugsweise Heterocyclylmethyl oder 2-Heterocyclyl-ethyl;
- C₁-C₄-Alkoxy für: OCH₃, OC₂H₅, OCH₂-C₂H₅, OCH(CH₃)₂, n-Butoxy, OCH(CH₃)-C₂H₅, OCH₂-CH(CH₃)₂ oder C(CH₃)₃, vorzugsweise für OCH₃, OC₂H₅ oder OCH(CH₃)₂;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy, vorzugsweise für OCHF₂, OCF₃, Dichlorfluormethoxy, Chlordifluormethoxy oder 2,2,2-Trifluorethoxy;
- C₁-C₆-Alkoxy für: einen C₁-C₄-Alkoxyrest wie vorstehend genannt, oder z.B. n-Pentoxy, 1-Methylbutoxy, 2-Methylbutoxy, 3-Methylbutoxy, 2,2-Dimethylpropoxy, 1-Ethylpropoxy, n-Hexoxy, 1,1-Dimethylpropoxy, 1,2-Dimethylpropoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy, vorzugsweise für OCH₃, OC₂H₅, OCH₂-C₂H₅, OCH(CH₃)₂, n-Butoxy, OC(CH₃)₃, n-Pentoxy oder n-Hexoxy;
- C₁-C₆-Halogenalkoxy für: einen C₁-C₆-Alkoxyrest wie vorstehend genannt, der partiell oder vollständig durch Fluor, Chlor, Brom und/oder Iod substituiert ist, also z.B. einen der unter C₁-C₄-Halogenalkoxy genannten Reste oder für 5-Fluor-1-pentoxy, 5-Chlor-1-pentoxy, 5-Brom-1-pentoxy, 5-Iod-1-pentoxy, 5,5,5-Trichlor-1-pentoxy, Undecafluorpentoxy, 6-Fluor-1-hexoxy, 6-Chlor-1-hexoxy, 6-Brom-1-hexoxy, 6-Iod-1-hexoxy, 6,6,6-Trichlor-1-hexoxy oder Dodecafluorhexoxy;
- C₁-C₄-Alkylthio für: SCH₃, SC₂H₅, SCH₂-C₂H₅, SCH(CH₃)₂, n-Butylthio, SCH(CH₃)-C₂H₅, SCH₂-CH(CH₃)₂ oder SC(CH₃)₃, vorzugsweise für SCH₃ oder SC₂H₅;
- C₁-C₄-Halogenalkylthio für: einen C₁-C₄-Alkylthiorest wie vorstehend genannt, der partiell oder vollständig durch Fluor, Chlor, Brom und/oder Iod substituiert ist, also z.B. SCH₂F, SCHF₂, SCF₃, SCH₂Cl, SCH(Cl)₂, SC(Cl)₃, Chlorfluormethylthio, Dichlorfluormethylthio, Chlordifluormethylthio, 2-Fluorethylthio, 2-Chlorethylthio, 2-Bromethylthio, 2-Iodethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, SC₂F₅, 2-Fluorpropylthio, 3-Fluorpropylthio, 2,2-Difluorpropylthio, 2,3-Difluorpropylthio, 2-Chlorpropylthio, 3-Chlorpropylthio, 2,3-Dichlorpropylthio, 2-Brompropylthio, 3-Brompropylthio, 3,3,3-Trifluorpropylthio, 3,3,3-Trichlorpropylthio, SCH₂-C₂F₅, SCF₂-C₂F₅, 1-(CH₂F)-2-fluorethylthio, 1-(CH₂Cl)-2-chlorethylthio, 1-(CH₂Br)-2-bromethylthio, 4-Fluorbutylthio, 4-Chlorbutylthio, 4-Brombutylthio oder SCF₂-CF₂-C₂F₅, vorzugsweise für SCHF₂, SCF₃, Dichlorfluormethylthio, Chlordifluormethylthio oder 2,2,2-Trifluorethylthio;
- C₁-C₆-Alkylthio für: einen C₁-C₄-Alkylthiorest wie vorstehend genannt, oder z.B. n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio, vorzugsweise für SCH₃, SC₂H₅, SCH₂-C₂H₅, SCH(CH₃)₂, n-Butylthio, SC(CH₃)₃, n-Pentylthio oder n-Hexylthio;
- C₁-C₄-Alkoxy-C₁-C₄-alkyl für: durch C₁-C₄-Alkoxy - wie vorstehend genannt - substituiertes C₁-C₄-Alkyl, also z.B. für CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)-propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise für CH₂-OCH₃, CH₂-OC₂H₅, 2-(OCH₃)ethyl oder 2-(OC₂H₅)ethyl;
- C₁-C₄-Halogenalkoxy-C₁-C₄-alkyl für: durch C₁-C₄-Halogenalkoxy wie vorstehend genannt substituiertes C₁-C₄-Alkyl, also z.B. für 2-(OCHF₂)ethyl, 2-(OCF₃)ethyl oder 2-(OC₂F₅)ethyl;
- C₁-C₄-Alkylthio-C₁-C₄-alkyl für: durch C₁-C₄-Alkylthio - wie vorstehend genannt - substituiertes C₁-C₄-Alkyl, also z.B. für CH₂-SCH₃, CH₂-SC₂H₅, n-Propylthiomethyl, CH₂-SCH(CH₃)₂, n-Butylthiomethyl, (1-Methylpropylthio)methyl, (2-Methylpropylthio)methyl, CH₂-SC(CH₃)₃, 2-(Methylthio)ethyl, 2-(Ethylthio)-ethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 2-(Ethylthio)propyl, 2-(n-Propylthio)propyl, 2-(1-Methylethylthio)propyl, 2-(n-Butylthio)propyl, 2-(1-Methylpropylthio)propyl, 2-(2-Methylpropylthio)propyl, 2-(1,1-Dimethylethylthio)propyl, 3-(Methylthio)propyl, 3-(Ethylthio)propyl, 3-(n-Propylthio)propyl, 3-(1-Methylethylthio)propyl, 3-(n-Butylthio)propyl, 3-(1-Methylpropylthio)-propyl, 3-(2-Methylpropylthio)propyl, 3-(1,1-Dimethylethylthio)propyl, 2-(Methylthio)butyl, 2-(Ethylthio)butyl, 2-(n-Propylthio)butyl, 2-(1-Methylethylthio)butyl, 2-(n-Butylthio)butyl, 2-(1-Methylpropylthio)butyl, 2-(2-Methylpropylthio)butyl, 2-(1,1-Dimethylethylthio)butyl, 3-(Methylthio)butyl, 3-(Ethylthio)butyl, 3-(n-Propylthio)butyl, 3-(1-Methylethylthio)butyl, 3-(n-Butylthio)butyl, 3-(1-Methylpropylthio)butyl, 3-(2-Methylpropylthio)butyl, 3-(1,1-Dimethylethylthio)butyl, 4-(Methylthio)butyl, 4-(Ethylthio)butyl, 4-(n-Propylthio)butyl, 4-(1-Methylethylthio)butyl, 4-(n-Butylthio)-butyl, 4-(1-Methylpropylthio)butyl, 4-(2-Methylpropylthio)butyl oder 4-(1,1-Dimethylethylthio)butyl, vorzugsweise CH₂-SCH₃, CH₂-SC₂H₅, 2-(SCH₃)ethyl oder 2-(SC₂H₅)ethyl;
- C₁-C₄-Halogenalkylthio-C₁-C₄-alkyl für: durch C₁-C₄-Halogenalkylthio wie vorstehend genannt substituiertes C₁-C₄-Alkyl, also z.B. für 2-(SCHF₂)ethyl, 2-(SCF₃)ethyl oder 2-(SC₂F₅)ethyl;
- (C₁-C₄-Alkyl)carbonyl für: CO-CH₃, CO-C₂H₅, CO-CH₂-C₂H₅, CO-CH(CH₃)₂, n-Butylcarbonyl, CO-CH(CH₃)-C₂H₅, CO-CH₂-CH(CH₃)₂ oder CO-C(CH₃)₃, vorzugsweise für CO-CH₃ oder CO-C₂H₅;
- (C₁-C₄-Halogenalkyl)carbonyl für: einen (C₁-C₄-Alkyl)carbonylrest - wie vorstehend genannt - der partiell oder vollständig durch Fluor, Chlor, Brom und/oder Iod substituiert ist, also z.B. CO-CH₂F, CO-CHF₂, CO-CF₃, CO-CH₂Cl, CO-CH(Cl)₂, CO-C(Cl)₃, Chlorfluormethylcarbonyl, Dichlorfluormethylcarbonyl, Chlordifluormethylcarbonyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, CO-C₂F₅, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, CO-CH₂-C₂F₅, CO-CF₂-C₂F₅, 1-(CH₂F)-2-fluorethylcarbonyl, 1-(CH₂Cl)-2-chlorethylcarbonyl, 1-(CH₂Br)-2-bromethylcarbonyl, 4-Fluorbutylcarbonyl, 4-Chlorbutylcarbonyl, 4-Brombutylcarbonyl oder Nonafluorbutylcarbonyl, vorzugsweise für CO-CF₃, CO-CH₂Cl oder 2,2,2-Trifluorethylcarbonyl;
- (C₁-C₄-Alkyl)carbonyloxy für: O-CO-CH₃, O-CO-C₂H₅, O-CO-CH₂-C₂H₅, O-CO-CH(CH₃)₂, O-CO-CH₂-CH₂-C₂H₅, O-CO-CH(CH₃)-C₂H₅, O-CO-CH₂-CH(CH₃)₂ oder O-CO-C(CH₃)₃, vorzugsweise für O-CO-CH₃ oder O-CO-C₂H₅;
- (C₁-C₄-Halogenalkyl)carbonyloxy für: einen (C₁-C₄-Alkyl)carbonylrest - wie vorstehend genannt - der partiell oder vollständig durch Fluor, Chlor, Brom und/oder Iod substituiert ist, also z.B. O-CO-CH₂F, O-CO-CHF₂, O-CO-CF₃, O-CO-CH₂Cl, O-CO-CH(Cl)₂, O-CO-C(Cl)₃, Chlorfluormethylcarbonyloxy, Dichlorfluormethylcarbonyloxy, Chlordifluormethylcarbonyloxy, 2-Fluorethylcarbonyloxy, 2-Chlorethylcarbonyloxy, 2-Bromethylcarbonyloxy, 2-Iodethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 2,2,2-Trichlorethylcarbonyloxy, O-CO-C₂F₅, 2-Fluorpropylcarbonyloxy, 3-Fluorpropylcarbonyloxy, 2,2-Difluorpropylcarbonyloxy, 2,3-Difluorpropylcarbonyloxy, 2-Chlorpropylcarbonyloxy, 3-Chlorpropylcarbonyloxy, 2,3-Dichlorpropylcarbonyloxy, 2-Brompropylcarbonyloxy, 3-Brompropylcarbonyloxy, 3,3,3-Trifluorpropylcarbonyloxy, 3,3,3-Trichlorpropylcarbonyloxy, O-CO-CH₂-C₂F₅, O-CO-CF₂-C₂F₅, 1-(CH₂F)-2-fluorethylcarbonyloxy, 1-(CH₂Cl)-2-chlorethylcarbonyloxy, 1-(CH₂Br)-2-bromethylcarbonyloxy, 4-Fluorbutylcarbonyloxy, 4-Chlorbutylcarbonyloxy, 4-Brombutylcarbonyloxy oder Nonafluorbutylcarbonyloxy, vorzugsweise für O-CO-CF₃, O-CO-CH₂Cl oder 2,2,2-Trifluorethylcarbonyloxy;
- (C₁-C₄-Alkoxy)carbonyl für: CO-OCH₃, CO-OC₂H₅, CO-OCH₂-C₂H₅, CO-OCH(CH₃)₂, n-Butoxycarbonyl, CO-OCH(CH₃)-C₂H₅, CO-OCH₂-CH(CH₃)₂ oder CO-OC(CH₃)₃, vorzugsweise für CO-OCH₃ oder CO-OC₂H₅;
- (C₁-C₆-Alkoxy)carbonyl für: einen der vorstehend genannten (C₁-C₄-Alkoxy)carbonylreste, oder z.B. n-Pentoxy-CO, 1-Methylbutoxy-CO, 2-Methylbutoxy-CO, 3-Methylbutoxy-CO, 2,2-Dimethylpropoxy-CO, 1-Ethylpropoxy-CO, n-Hexoxy-CO, 1,1-Dimethylpropoxy-CO, 1,2-Dimethylpropoxy-CO, 1-Methylpentoxy-CO, 2-Methylpentoxy-CO, 3-Methylpentoxy-CO, 4-Methylpentoxy-CO, 1,1-Dimethylbutoxy-CO, 1,2-Dimethylbutoxy-CO, 1,3-Dimethylbutoxy-CO, 2,2-Dimethylbutoxy-CO, 2,3-Dimethylbutoxy-CO, 3,3-Dimethylbutoxy-CO, 1-Ethylbutoxy-CO, 2-Ethylbutoxy-CO, 1,1,2-Trimethylpropoxy-CO, 1,2,2-Trimethylpropoxy-CO, 1-Ethyl-1-methylpropoxy-CO oder 1-Ethyl-2-methylpropoxy-CO, vorzugsweise für CO-OCH₃, CO-OC₂H₅, CO-OCH₂-C₂H₅, CO-OCH(CH₃)₂, n-Butoxy-CO, CO-OC(CH₃)₃, n-Pentoxy-CO oder n-Hexoxy-CO;
- (C₁-C₄-Alkoxy)carbonyl-C₁-C₄-alkyl für: durch (C₁-C₄-Alkoxy)-carbonyl - wie vorstehend genannt - substituiertes C₁-C₄-Alkyl, also z.B. für CH₂-CO-OCH₃, CH₂-CO-OC₂H₅, CH₂-CO-OCH₂-C₂H₅, CH₂-CO-OCH(CH₃)₂, n-Butoxycarbonylmethyl, CH₂-CO-OCH(CH₃)-C₂H₅, CH₂-CO-OCH₂-CH(CH₃)₂, CH₂-CO-OC(CH₃)₃, 1-(CO-OCH₃)ethyl, 1-(CO-OC₂H₅)ethyl, 1-(CO-OCH₂-C₂H₅)ethyl, 1-[CH(CH₃)₂]ethyl, 1-(n-Butoxycarbonyl)ethyl, 1-[1-Methylpropoxycarbonyl]ethyl, 1-[2-Methylpropoxycarbonyl]ethyl, 2-(CO-OCH₃)ethyl, 2-(CO-OC₂H₅)ethyl, 2-(CO-OCH₂-C₂H₅)ethyl, 2-[CO-OCH(CH₃)₂]ethyl, 2-(n-Butoxycarbonyl)ethyl, 2-[1-Methylpropoxycarbonyl]ethyl, 2-[2-Methylpropoxycarbonyl]ethyl, 2-[CO-OC(CH₃)₃]ethyl, 2-(CO-OCH₃)propyl, 2-(CO-OC₂H₅)propyl, 2-(CO-OCH₂-C₂H₅)propyl, 2-[CO-OCH(CH₃)₂]propyl, 2-(n-Butoxycarbonyl)propyl, 2-[1-Methylpropoxycarbonyl]propyl, 2-[2-Methylpropoxycarbonyl]propyl, 2-[CO-OC(CH₃)₃)propyl, 3-(CO-OCH₃)-propyl, 3-(CO-OC₂H₅)propyl, 3-(CO-OCH₂-C₂H₅)propyl, 3-[CO-OCH(CH₃)₂]propyl, 3-(n-Butoxycarbonyl)propyl, 3-[1-Methylpropoxycarbonyl]propyl, 3-[2-Methylpropoxycarbonyl]propyl, 3-[CO-OC(CH₃)₃]propyl, 2-(CO-OCH₃)butyl, 2-(CO-OC₂H₅)butyl, 2-(CO-OCH₂-C₂H₅)butyl, 2-[CO-OCH(CH₃)₂]butyl, 2-(n-Butoxycarbonyl)butyl, 2-[1-Methylpropoxycarbonyl]butyl, 2-[2-Methylpropoxycarbonyl]butyl, 2-[CO-OC(CH₃)₃]butyl, 3-(CO-OCH₃)butyl, 3-(CO-OC₂H₅)butyl, 3-(CO-OCH₂-C₂H₅)butyl, 3-[CO-OCH(CH₃)₂]butyl, 3-(n-Butoxycarbonyl)butyl, 3-[1-Methylpropoxycarbonyl]butyl, 3-[2-Methylpropoxycarbonyl]butyl, 3-[CO-OC(CH₃)₃]butyl, 4-(CO-OCH₃)butyl, 4-(CO-OC₂H₅)butyl, 4-(CO-OCH₂-C₂H₅)butyl, 4-[CO-OCH(CH₃)₂]butyl, 4-(n-Butoxycarbonyl)butyl, 4-[1-Methylpropoxycarbonyl]butyl, 4-[2-Methylpropoxycarbonyl]butyl oder 4-[CO-OC(CH₃)₃]butyl, vorzugsweise für CH₂-CO-OCH₃, CH₂-CO-OC₂H₅, 1-(CO-OCH₃)ethyl oder 1-(CO-OC₂H₅)ethyl;
- (C₁-C₄-Alkylthio)carbonyl für: CO-SCH₃, CO-SC₂H₅, CO-SCH₂-C₂H₅, CO-SCH(CH₃)₂, CO-SCH₂CH₂-C₂H₅, CO-SCH(CH₃)-C₂H₅, CO-SCH₂-CH(CH₃)₂ oder CO-SC(CH₃)₃, vorzugsweise für CO-SCH₃ oder CO-SC₂H₅;
- (C₁-C₄-Alkylthio)carbonyl-C₁-C₄-alkyl für: durch (C₁-C₄-Alkylthio)carbonyl - wie vorstehend genannt - substituiertes C₁-C₄-Alkyl, also z.B. für CH₂-CO-SCH₃, CH₂-CO-SC₂H₅, CH₂-CO-SCH₂-C₂H₅, CH₂-CO-SCH(CH₃)₂, CH₂-CO-SCH₂CH₂-C₂H₅, CH₂-CO-SCH(CH₃)-C₂H₅, CH₂-CO-SCH₂-CH(CH₃)₂, CH₂-CO-SC(CH₃)₃, 1-(CO-SCH₃)ethyl, 1-(CO-SC₂H₅)ethyl, 1-(CO-SCH₂-C₂H₅)ethyl, 1-[CO-SCH(CH₃)₂]ethyl, 1-(CO-SCH₂CH₂-C₂H₅)ethyl, 1-[CO-SCH(CH₃)-C₂H₅]ethyl, 1-[CO-SCH₂-CH(CH₃)₂]ethyl, 1-[CO-SC(CH₃)₃]ethyl, 2-(CO-SCH₃)ethyl, 2-(CO-SC₂H₅)ethyl, 2-(CO-SCH₂-C₂H₅)ethyl, 2-[CO-SCH(CH₃)₂]ethyl, 2-(CO-SCH₂CH₂-C₂H₅)ethyl, 2-[CO-SCH(CH₃)-C₂H₅]ethyl, 2-[CO-SCH₂-CH(CH₃)₂]ethyl, 2-[CO-SC(CH₃)₃]ethyl, 2-(CO-SCH₃)-propyl, 2-(CO-SC₂H₅)propyl, 2-(CO-SCH₂-C₂H₅)propyl, 2-[CO-SCH(CH₃)₂]propyl, 2-(CO-SCH₂CH₂-C₂H₅)propyl, 2-[CO-SCH(CH₃)-C₂H₅]propyl, 2-[CO-SCH₂-CH(CH₃)₂]propyl, 2-[CO-SC(CH₃)₃]propyl, 3-(CO-SCH₃)propyl, 3-(CO-SC₂H₅)propyl, 3-(CO-SCH₂-C₂H₅)propyl, 3-[CO-SCH(CH₃)₂]propyl, 3-(CO-SCH₂CH₂-C₂H₅)propyl, 3-[CO-SCH(CH₃)-C₂H₅]propyl, 3-[CO-SCH₂-CH(CH₃)₂]propyl, 3-[CO-SC(CH₃)₃]propyl, 2-(CO-SCH₃)butyl, 2-(CO-SC₂H₅)butyl, 2-(CO-SCH₂-C₂H₅)butyl, 2-[CO-SCH(CH₃)₂]butyl, 2-(CO-SCH₂CH₂-C₂H₅)butyl, 2-[CO-SCH(CH₃)-C₂H₅]butyl, 2-[CO-SCH₂-CH(CH₃)₂]butyl, 2-[CO-SC(CH₃)₃]butyl, 3-(CO-SCH₃)butyl, 3-(CO-SC₂H₅)butyl, 3-(CO-SCH₂-C₂H₅)butyl, 3-[CO-SCH(CH₃)₂]butyl, 3-(CO-SCH₂CH₂-C₂H₅)butyl, 3-[CO-SCH(CH₃)-C₂H₅]butyl, 3-[CO-SCH₂-CH(CH₃)₂]butyl, 3-[CO-SC(CH₃)₃]butyl, 4-(CO-SCH₃)-butyl, 4-(CO-SC₂H₅)butyl, 4-(CO-SCH₂-C₂H₅)butyl, 4-[CO-SCH(CH₃)₂]butyl, 4-(CO-SCH₂CH₂-C₂H₅)butyl, 4-[CO-SCH(CH₃)-C₂H₅]butyl, 4-[CO-SCH₂-CH(CH₃)₂)butyl oder 4-[CO-SC(CH₃)₃]butyl, vorzugsweise für CH₂-CO-SCH₃, CH₂-CO-SC₂H₅, 1-(CO-SCH₃)ethyl oder 1-(CO-SC₂H₅)ethyl;
- C₁-C₆-Alkylsulfinyl für: einen C₁-C₄-Alkylsulfinylrest wie SO-CH₃, SO-C₂H₅, SO-CH₂-C₂H₅, SO-CH(CH₃)₂, SO-(n-C₄H₉), SO-CH(CH₃)-C₂H₅, SO-CH₂-CH(CH₃)₂ oder SO-C(CH₃)₃, oder z.B. SO-(n-C₅H₁₁), 1-Methylbutyl-SO, 2-Methylbutyl-SO, 3-Methylbutyl-SO, 2,2-Dimethylpropyl-SO, 1-Ethylpropyl-SO, n-Hexyl-SO, 1,1-Dimethylpropyl-SO, 1,2-Dimethylpropyl-SO, 1-Methylpentyl-SO, 2-Methylpentyl-SO, 3-Methylpentyl-SO, 4-Methylpentyl-SO, 1,1-Dimethylbutyl-SO, 1,2-Dimethylbutyl-SO, 1,3-Dimethylbutyl-SO, 2,2-Dimethylbutyl-SO, 2,3-Dimethylbutyl-SO, 3,3-Dimethylbutyl-SO, 1-Ethylbutyl-SO, 2-Ethylbutyl-SO, 1,1,2-Trimethylpropyl-SO, 1,2,2-Trimethylpropyl-SO, 1-Ethyl-1-methylpropyl-SO oder 1-Ethyl-2-methylpropyl-SO, vorzugsweise für SO-CH₃, SO-C₂H₅, SO-CH₂-C₂H₅, SO-CH(CH₃)₂, SO-(n-C₄H₉), SO-C(CH₃)₃, SO-(n-C₅H₁₁) oder SO-(n-C₆H₁₃);
- C₁-C₄-Alkylsulfonyl für: SO₂-CH₃, SO₂-C₂H₅, SO₂-CH₂-C₂H₅, SO₂-CH(CH₃)₂, n-Butylsulfonyl, SO₂-CH(CH₃)-C₂H₅, SO₂-CH₂-CH(CH₃)₂ oder SO₂-C(CH₃)₃, vorzugsweise für SO₂-CH₃ oder SO₂-C₂H₅;
- C₁-C₄-Halogenalkylsulfonyl für: einen C₁-C₄-Alkylsulfonylrestwie vorstehend genannt - der partiell oder vollständig durch Fluor, Chlor, Brom und/oder Iod substituiert ist, also z.B. SO₂-CH₂F, SO₂-CHF₂, SO₂-CF₃, SO₂-CH₂Cl, SO₂-CH(Cl)₂, SO₂-C(Cl)₃, Chlorfluormethylsulfonyl, Dichlorfluormethylsulfonyl, Chlordifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, SO₂-C₂F₅, 2-Fluorpropylsulfonyl, 3-Fluorpropylsulfonyl, 2,2-Difluorpropylsulfonyl, 2,3-Difluorpropylsulfonyl, 2-Chlorpropylsulfonyl, 3-Chlorpropylsulfonyl, 2,3-Dichlorpropylsulfonyl, 2-Brompropylsulfonyl, 3-Brompropylsulfonyl, 3,3,3-Trifluorpropylsulfonyl, 3,3,3-Trichlorpropylsulfonyl, SO₂-CH₂-C₂F₅, SO₂-CF₂-C₂F₅, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl, vorzugsweise für SO₂-CH₂Cl, SO₂-CF₃ oder 2,2,2-Trifluorethylsulfonyl;
- C₁-C₆-Alkylsulfonyl für: einen C₁-C₄-Alkylsulfonylrest wie vorstehend genannt, oder z.B. SO₂-(n-C₅H₁₁), 1-Methylbutyl-SO₂, 2-Methylbutyl-SO₂, 3-Methylbutyl-SO₂, 2,2-Dimethylpropyl-SO₂, 1-Ethylpropyl-SO₂, n-Hexyl-SO₂, 1,1-Dimethylpropyl-SO₂, 1,2-Dimethylpropyl-SO₂, 1-Methylpentyl-SO₂, 2-Methylpentyl-SO₂, 3-Methylpentyl-SO₂, 4-Methylpentyl-SO₂, 1,1-Dimethylbutyl-SO₂, 1,2-Dimethylbutyl-SO₂, 1,3-Dimethylbutyl-SO₂, 2,2-Dimethylbutyl-SO₂, 2,3-Dimethylbutyl-SO₂, 3,3-Dimethylbutyl-SO₂, 1-Ethylbutyl-SO₂, 2-Ethylbutyl-SO₂, 1,1,2-Trimethylpropyl-SO₂, 1,2,2-Trimethylpropyl-SO₂, 1-Ethyl-1-methylpropyl-SO₂ oder 1-Ethyl-2-methylpropyl-SO₂, vorzugsweise für SO₂-CH₃, SO₂-C₂H₅, SO₂-CH₂-C₂H₅, SO₂-CH(CH₃)₂, SO₂-(n-C₄H₉), SO₂-C(CH₃)₃, SO₂-(n-C₅H₁₁) oder SO₂-(n-C₆H₁₃);
- C₁-C₄-Alkylamino für H₃C-NH-, H₅C₂-NH-, n-Propyl-NH-, 1-Methylethyl-NH-, n-Butyl-NH-, 1-Methylpropyl-NH-, 2-Methylpropyl-NHund 1,1-Dimethylethyl-NH-, vorzugsweise für H₃C-NH- oder H₅C₂-NH-,
- C₁-C₄-Alkylamino-C₁-C₄-alkyl für: durch C₁-C₄-Alkylamino wie vorstehend definiert, substituiertes C₁-C₄-Alkyl, also beispielsweise für CH₂CH₂-NH-CH₃, CH₂CH₂-N(CH₃)₂, CH₂CH₂-NH-C₂H₅ oder CH₂CH₂-N(C₂H₅)₂;
- (C₁-C₄-Alkylamino)carbonyl für: CO-NH-CH₃, CO-NH-C₂H₅, n-Propylamino, CO-NH-CH(CH₃)₂, CO-NH-CH₂CH₂-C₂H₅, CO-NH-CH(CH₃)-C₂H₅, CO-NH-CH₂-CH(CH₃)₂ oder CO-NH-C(CH₃)₃, vorzugsweise für CO-NH-CH₃ oder CO-NH-C₂H₅;
- (C₁-C₄-Alkylamino)carbonyl-C₁-C₄-alkyl für: durch (C₁-C₄-Alkylamino)carbonyl wie vorstehend genannt, vorzugsweise CO-NH-CH₃ oder CO-NH-C₂H₅, substituiertes C₁-C₄-Alkyl, als z.B. für CH₂-CO-NH-CH₃, CH₂-CO-NH-C₂H₅, CH₂-CO-NH-CH₂-C₂H₅, CH₂-CO-NH-CH(CH₃)₂, CH₂-CO-NH-CH₂CH₂-C₂H₅, CH₂-CO-NH-CH(CH₃)-C₂H₅, CH₂-CO-NH-CH₂-CH(CH₃)₂, CH₂-CO-NH-C(CH₃)₃, CH(CH₃)-CO-NH-CH₃, CH(CH₃)-CO-NH-C₂H₅, 2-(CO-NH-CH₃)ethyl, 2-(CO-NH-C₂H₅)ethyl, 2-(CO-NH-CH₂-C₂H₅)-ethyl, 2-[CH₂-CO-NH-CH(CH₃)₂]ethyl, 2-(CO-NH-CH₂CH₂-C₂H₅)ethyl, 2-[CO-NH-CH(CH₃)-C₂H₅]ethyl, 2-[CO-NH-CH₂-CH(CH₃)₂]ethyl, 2-[CO-NH-C(CH₃)₃]ethyl, 2-(CO-NH-CH₃)propyl, 2-(CO-NH-C₂H₅)propyl, 2-(CO-NH-CH₂-C₂H₅)propyl, 2-[CH₂-CO-NH-CH(CH₃)₂]propyl, 2-(CO-NH-CH₂CH₂-C₂H₅)propyl, 2-[CO-NH-CH(CH₃)-C₂H₅]propyl, 2-[CO-NH-CH₂-CH(CH₃)₂]propyl, 2-[CO-NH-C(CH₃)₃]propyl, 3-(CO-NH-CH₃)propyl, 3-(CO-NH-C₂H₅)propyl, 3-(CO-NH-CH₂-C₂H₅)propyl, 3-[CH₂-CO-NH-CH(CH₃)₂]propyl, 3-(CO-NH-CH₂CH₂-C₂H₅)propyl, 3-[CO-NH-CH(CH₃)-C₂H₅]propyl, 3-[CO-NH-CH₂-CH(CH₃)₂]propyl, 3-[CO-NH-C(CH₃)₃]propyl, 2-(CO-NH-CH₃)butyl, 2-(CO-NH-C₂H₅)-butyl, 2-(CO-NH-CH₂-C₂H₅)butyl, 2-[CH₂-CO-NH-CH(CH₃)₂]butyl, 2-(CO-NH-CH₂CH₂-C₂H₅)butyl, 2-[CO-NH-CH(CH₃)-C₂H₅]butyl, 2-[CO-NH-CH₂-CH(CH₃)₂]butyl, 2-[CO-NH-C(CH₃)₃]butyl, 3-(CO-NH-CH₃)butyl, 3-(CO-NH-C₂H₅)butyl, 3-(CO-NH-CH₂-C₂H₅)-butyl, 3-[CH₂-CO-NH-CH(CH₃)₂]butyl, 3-(CO-NH-CH₂CH₂-C₂H₅)butyl, 3-[CO-NH-CH(CH₃)-C₂H₅]butyl, 3-[CO-NH-CH₂-CH(CH₃)₂]butyl, 3-[CO-NH-C(CH₃)₃]butyl, 4-(CO-NH-CH₃)butyl, 4-(CO-NH-C₂H₅)butyl, 4-(CO-NH-CH₂-C₂H₅)butyl, 4-[CH₂-CO-NH-CH(CH₃)₂]butyl, 4-(CO-NH-CH₂CH₂-C₂H₅)butyl, 4-[CO-NH-CH(CH₃)-C₂H₅]butyl, 4-[CO-NH-CH₂-CH(CH₃)₂]butyl oder 4-[CO-NH-C(CH₃)₃]butyl, vorzugsweise für CH₂-CO-NH-CH_{3,} CH₂-CO-NH-C₂H_{5,} CH(CH₃)-CO-NH-CH₃ oder CH(CH₃)-CO-NH-C₂H₅;
- Di(C₁-C₄-alkyl)amino für: N(CH₃)₂, N(C₂H₅)₂, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)-amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino, vorzugsweise für N(CH₃)₂ oder N(C₂H₅)₂;
- Di(C₁-C₄-alkyl)amino-C₁-C₄-alkyl für: durch Di(C₁-C₄-alkyl)-amino wie vorstehend genannt substituiertes C₁-C₄-Alkyl, also z.B. für CH₂N(CH₃)₂, CH₂N(C₂H₅)₂, N,N-Dipropylaminomethyl, N,N-Di[CH(CH₃)₂]aminomethyl, N,N-Dibutylaminomethyl, N,N-Di-(1-methylpropyl)aminomethyl, N,N-Di(2-methylpropyl)aminomethyl, N,N-Di[C(CH₃)₃]aminomethyl, N-Ethyl-N-methylaminomethyl, N-Methyl-N-propylaminomethyl, N-Methyl-N-[CH(CH₃)₂]aminomethyl, N-Butyl-N-methylaminomethyl, N-Methyl-N-(1-methylpropyl)aminomethyl, N-Methyl-N-(2-methylpropyl)aminomethyl, N-[C(CH₃)₃]-N-methylaminomethyl, N-Ethyl-N-propylaminomethyl, N-Ethyl-N-[CH(CH₃)₂]aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-[C(CH₃)₃]aminomethyl, N-[CH(CH₃)₂]-N-propylaminomethyl, N-Butyl-N-propylaminomethyl, N-(1-Methylpropyl)-N-propylaminomethyl, N-(2-Methylpropyl)-N-propylaminomethyl, N-[C(CH₃)₃]-N-propylaminomethyl, N-Butyl-N-(1-methylethyl)-aminomethyl, N-[CH(CH₃)₂]-N-(1-methylpropyl)aminomethyl, N-[CH(CH₃)₂]-N-(2-methylpropyl)aminomethyl, N-[C(CH₃)₃]-N-[CH(CH₃)₂]aminomethyl, N-Butyl-N-(1-methylpropyl)aminomethyl, N-Butyl-N-(2-methylpropyl)aminomethyl, N-Butyl-N-[C(CH₃)₃]-aminomethyl, N-(1-Methylpropyl)-N-(2-methylpropyl)aminomethyl, N-[C(CH₃)₃]-N-(1-methylpropyl)aminomethyl, N-[C(CH₃)₃]-N-(2-methylpropyl)aminomethyl, N,N-Dimethylaminoethyl, N,N-Diethylaminoethyl, N,N-Di(n-propyl)aminoethyl, N,N-Di-[CH(CH₃)₂]-aminoethyl, N,N-Dibutylaminoethyl, N,N-Di(1-methylpropyl)aminoethyl, N,N-Di(2-methylpropyl)aminoethyl, N,N-Di-[C(CH₃)₃]-aminoethyl, N-Ethyl-N-methylaminoethyl, N-Methyl-N-propylaminoethyl, N-Methyl-N-[CH(CH₃)₂]aminoethyl, N-Butyl-N-methylaminoethyl, N-Methyl-N-(1-methylpropyl)aminoethyl, N-Methyl-N-(2-methylpropyl)aminoethyl, N-[C(CH₃)₃]-N-methylaminoethyl, N-Ethyl-N-propylaminoethyl, N-Ethyl-N-[CH(CH₃)₂]aminoethyl, N-Butyl-N-ethylaminoethyl, N-Ethyl-N-(1-methylpropyl)aminoethyl, N-Ethyl-N-(2-methylpropyl)aminoethyl, N-Ethyl-N-[C(CH₃)₃]aminoethyl, N-[CH(CH₃)₂]-N-propylaminoethyl, N-Butyl-N-propylaminoethyl, N-(1-Methylpropyl)-N-propylaminoethyl, N-(2-Methylpropyl)-N-propylaminoethyl, N-[C(CH₃)₃]-N-propylaminoethyl, N-Butyl-N-[CH(CH₃)₂]aminoethyl, N-[CH(CH₃)₂]-N-(1-methylpropyl)aminoethyl, N-[CH(CH₃)₂]-N-(2-methylpropyl)-aminoethyl, N-[C(CH₃)₃]-N-[CH(CH₃)₂]aminoethyl, N-Butyl-N-(1-methylpropyl)aminoethyl, N-Butyl-N-(2-methylpropyl)aminoethyl, N-Butyl-N-[C(CH₃)₃]aminoethyl, N-(1-Methylpropyl)-N-(2-methylpropyl)aminoethyl, N-[C(CH₃)₃]-N-(1-methylpropyl)-aminoethyl oder N-[C(CH₃)₃]-N-(2-methylpropyl)aminoethyl, insbesondere für N,N-Dimethylaminoethyl oder N,N-Diethylaminoethyl;
- Di(C₁-C₄-alkyl)aminocarbonyl für: CO-N(CH₃)₂, CO-N(C₂H₅), CO-N(CH₂-C₂H₅)₂, CO-N[CH(CH₃)₂]₂, N,N-Dibutylaminocarbonyl, CO-N[CH(CH₃)-C₂H₅]₂, CO-N[CH₂-CH(CH₃)₂]₂, CO-N[C(CH₃)₃]₂, N-Ethyl-N-methylaminocarbonyl, N-Methyl-N-propylaminocarbonyl, N-Methyl-N-[CH(CH₃)₂]aminocarbonyl, N-Butyl-N-methylaminocarbonyl, N-Methyl-N-(1-methylpropyl)aminocarbonyl, N-Methyl-N-(2-methylpropyl)aminocarbonyl, N-[C(CH₃)₃]-N-methylaminocarbonyl, N-Ethyl-N-propylaminocarbonyl, N-Ethyl-N-[CH(CH₃)₂]-aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-[C(CH₃)₃]aminocarbonyl, N-[CH(CH₃)₂]-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-[C(CH₃)₃]-N-propylaminocarbonyl, N-Butyl-N-[CH(CH₃)₂]aminocarbonyl, N-[CH(CH₃)₂]-N-(1-methylpropyl)aminocarbonyl, H-[CH(CH₃)₂]-N-(2-methylpropyl)aminocarbonyl, N-[C(CH₃)₃]-N-[CH(CH₃)₂]aminocarbonyl, N-Butyl-N-(1-methylpropyl)aminocarbonyl, N-Butyl-N-(2-methylpropyl)-aminocarbonyl, N-Butyl-N-[C(CH₃)₃]aminocarbonyl, N-(1-Methylpropyl)-N-(2-methylpropyl)aminocarbonyl, N-[C(CH₃)₃]-N-(1-methylpropyl)aminocarbonyl oder N-[C(CH₃)₃]-N-(2-methylpropyl)aminocarbonyl, vorzugsweise für CO-N(CH₃)₂ oder CO-N(C₂H₅)₂;
- Di(C₁-C₆-alkyl)aminocarbonyl für: einen der vorstehend genannten Di(C₁-C₄-alkyl)aminocarbonylreste oder z.B. N(CH₃)-(n-C₅H₁₁), N(C₂H₅)-(n-C₅H₁₁), N(CH₂-C₂H₅)-(n-C₅H₁₁), N(n-C₄H₉)-(n-C₅H₁₁), N(n-C₅H₁₁)-(n-C₅H₁₁), N(n-C₆H₁₃)-(n-C₅H₁₁), N(CH₃)-(n-C₆H₁₃), N(C₂H₅)-(n-C₆H₁₃), N(CH₂-C₂H₅)-(n-C₆H₁₃), N(n-C₄H₉)-(n-C₆H₁₃), N(n-C₅H₁₁)-(n-C₆H₁₃) oder N(n-C₆H₁₃)₂;
- Di(C₁-C₄-alkyl)phosphonyl-C₁-C₄-alkyl für: durch Di(C₁-C₄-alkyl)phosphonyl wie -PO(OCH₃)₂, -PO(OC₂H₅)₂, N,N-Dipropylphosphonyl, N,N-Di-(1-methylethyl)phosphonyl, N,N-Dibutylphosphonyl, N,N-Di-(1-methylpropyl)phosphonyl, N,N-Di-(2-methylpropyl)phosphonyl, N,N-Di-(1,1-dimethylethyl)phosphonyl, N-Ethyl-N-methylphosphonyl, N-Methyl-N-propylphosphonyl, N-Methyl-N-(1-methylethyl)phosphonyl, N-Butyl-N-methylphosphonyl, N-Methyl-N-(1-methylpropyl)phosphonyl, N-Methyl-N-(2-methylpropyl)phosphonyl, N-(1,1-Dimethylethyl)-N-methylphosphonyl, N-Ethyl-N-propylphosphonyl, N-Ethyl-N-(1-methylethyl)phosphonyl, N-Butyl-N-ethylphosphonyl, N-Ethyl-N-(1-methylpropyl)phosphonyl, N-Ethyl-N-(2-methylpropyl)phosphonyl, N-Ethyl-N-(1,1-dimethylethyl)phosphonyl, N-(1-Methylethyl)-N-propylphosphonyl, N-Butyl-N-propylphosphonyl, N-(1-Methylpropyl)-N-propylphosphonyl, N-(2-Methylpropyl)-N-propylphosphonyl, N-(1,1-Dimethylethyl)-N-propylphosphonyl, N-Butyl-N-(1-methylethyl)phosphonyl, N-(1-Methylethyl)-N-(1-methylpropyl)phosphonyl, N-(1-Methylethyl)-N-(2-methylpropyl)phosphonyl, N-(1,1-Dimethylethyl)-N-(1-methylethyl)phosphonyl, N-Butyl-N-(1-methylpropyl)phosphonyl, N-Butyl-N-(2-methylpropyl)phosphonyl, N-Butyl-N-(1,1-dimethylethyl)phosphonyl, N-(1-Methylpropyl)-N-(2-methylpropyl)-phosphonyl, N-(1,1-Dimethylethyl)-N-(1-methylpropyl)phosphonyl oder N-(1,1-Dimethylethyl)-N-(2-methylpropyl)phosphonyl, vorzugsweise -PO(OCH₃)₂ oder -PO(OC₂H₅)₂, substituiertes C₁-C₄-Alkyl, also z.B. für CH₂-PO(OCH₃)₂, CH₂-PO(OC₂H₅)₂, CH(CH₃)-PO(OCH₃)₂ oder CH(CH₃)-PO(OC₂H₅)₂;
- C₃-C₆-Alkenyl für: Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl, 1-Methylprop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methylbut-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methylpent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methylpent-3-en-1-yl 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethylbut-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₃-C₆-Halogenalkenyl für: C₃-C₆-Alkenyl wie vorstehend genannt, das partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- Cyano-C₃-C₆-alkenyl für: z.B. 2-Cyanoallyl, 3-Cyanoallyl, 4-Cyanobut-2-enyl, 4-Cyanobut-3-enyl oder 5-Cyanopent-4-enyl;
- C₃-C₆-Alkinyl für: Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für Prop-2-in-1-yl;
- C₃-C₆-Halogenalkinyl für: C₃-C₆-Alkinyl wie vorstehend genannt, das partiell oder vollständig durch Fluor, Chlor, Brom und/oder Iod substituiert ist, also z.B. 1,1-Difluorprop-2-in-1-yl, 4-Fluorbut-2-in-1-yl, 4-Chlorbut-2-in-1-yl, 1,1-Difluorbut-2-in-1-yl, 5-Fluorpent-3-in-1-yl oder 6-Fluorhex-4-in-1-yl;
- Cyano-C₃-C₆-alkinyl für: z.B. 3-Cyanopropargyl, 4-Cyanobut-2-in-1-yl, 5-Cyanopent-3-in-1-yl und 6-Cyanohex-4-in-1-yl;
- C₃-C₄-Alkenyloxy-C₁-C₄-alkyl für: durch C₃-C₄-Alkenyloxy wie Allyloxy, But-1-en-3-yloxy, But-1-en-4-yloxy, But-2-en-1-yloxy, 1-Methylprop-2-enyloxy oder 2-Methylprop-2-enyloxy substituiertes C₁-C₄-Alkyl, also beispielsweise für Allyloxymethyl, 2-Allyloxyethyl oder But-1-en-4-yloxymethyl, insbesondere für 2-Allyloxyethyl;
- C₃-C₄-Alkinyloxy-C₁-C₄-alkyl für: durch C₃-C₄-Alkinyloxy wie Propargyloxy, But-1-in-3-yloxy, But-1-in-4-yloxy, But-2-in-1-yloxy, 1-Methylprop-2-inyloxy oder 2-Methylprop-2-inyloxy, vorzugsweise Propargyloxy, substituiertes C₁-C₄-Alkyl, also beispielsweise für Propargyloxymethyl oder 2-Propargyloxyethyl, insbesondere für 2-Propargyloxyethyl;
- C₃-C₆-Cycloalkyl für: Cyclopropyl, Cyclobutyl, Cyclopentyl oder Cyclohexyl;
- C₃-C₆-Cycloalkylamino für: Cyclopropylamino, Cyclobutylamino, Cyclopentylamino oder Cyclohexylamino;
- C₃-C₈-Cycloalkyl für: Cyclopropyl, Cyclobutyl, Cyclopentyl, Cyclohexyl, Cycloheptyl oder Cyclooctyl;
- C₃-C₈-Cycloalkyl-C₁-C₆-alkyl für: z.B. Cyclopropylmethyl, Cyclobutylmethyl, Cyclopentylmethyl, Cyclohexylmethyl, Cycloheptylmethyl, Cyclooctylmethyl, 2-(Cyclopropyl)ethyl, 2-(Cyclobutyl)-ethyl, 2-(Cyclopentyl)ethyl, 2-(Cyclohexyl)ethyl, 2-(Cycloheptyl)ethyl, 2-(Cyclooctyl)ethyl, 3-(Cyclopropyl)propyl, 3-(Cyclobutyl)propyl, 3-(Cyclopentyl)propyl, 3-(Cyclohexyl)-propyl, 3-(Cycloheptyl)propyl, 3-(Cyclooctyl)propyl, 4-(Cyclopropyl)butyl, 4-(Cyclobutyl)butyl, 4-(Cyclopentyl)butyl, 4-(Cyclohexyl)butyl, 4-(Cycloheptyl)butyl, 4-(Cyclooctyl)butyl, 5-(cyclopropyl)pentyl, 5-(Cyclobutyl)pentyl, 5-(Cyclopentyl)-pentyl, 5-(Cyclohexyl)pentyl, 5-(Cycloheptyl)pentyl, 5-(Cyclooctyl)pentyl, 6-(Cyclopropyl)hexyl, 6-(Cyclobutyl)hexyl, 6-(Cyclopentyl)hexyl, 6-(Cyclohexyl)hexyl, 6-(Cycloheptyl)hexyl oder 6-(Cyclooctyl)hexyl;
- C₃-C₈-Cycloalkyloxy-C₁-C₄-alkyl für: Cyclopropyloxymethyl, 1-Cyclopropyloxy-ethyl, 2-Cyclopropyloxy-ethyl, 1-Cyclopropyloxy-prop-1-yl, 2-Cyclopropyloxy-prop-1-yl, 3-Cyclopropyloxyprop-1-yl, 1-Cyclopropyloxy-but-1-yl, 2-Cyclopropyloxy-but1-yl, 3-Cyclopropyloxy-but-1-yl, 4-Cyclopropyloxy-but-1-yl, 1-Cyclopropyloxy-but-2-yl, 2-Cyclopropyloxy-but-2-yl, 3-Cyclopropyloxy-but-2-yl, 3-Cyclopropyloxy-but-2-yl, 4-Cyclopropyloxy-but-2-yl, 1-(Cyclopropyloxymethyl)-eth-1-yl, 1-(Cyclopropyloxymethyl)-1-(CH₃)-eth-1-yl, 1-(Cyclopropylmethyloxy)-prop-1-yl, Cyclobutyloxymethyl, 1-Cyclobutyloxy-ethyl, 2-Cyclobutyloxy-ethyl, 1-Cyclobutyloxy-prop-1-yl, 2-Cyclobutyloxyprop-1-yl, 3-Cyclobutyloxy-prop-1-yl, 1-Cyclobutyloxy-but-1-yl, 2-Cyclobutyloxy-but-1-yl, 3-Cyclobutyloxy-but-1-yl, 4-Cyclobutyloxy-but-1-yl, 1-Cyclobutyloxy-but-2-yl, 2-Cyclobutyloxybut-2-yl, 3-Cyclobutyloxy-but-2-yl, 3-Cyclobutyloxy-but-2-yl, 4-Cyclobutyloxy-but-2-yl, 1-(Cyclobutyloxymethyl)eth-1-yl, 1-(Cyclobutyloxymethyl)-1-(CH₃)-eth-1-yl, 1-(Cyclobutyloxymethyl)prop-1-yl, Cyclopentyloxymethyl, 1-Cyclopentyloxy-ethyl, 2-Cyclopentyloxy-ethyl, 1-Cyclopentyloxy-prop-1-yl, 2-Cyclopentyloxy-prop-1-yl, 3-Cyclopentyloxy-prop-1-yl, 1-Cyclopentyloxy-but-1-yl, 2-Cyclopentyloxy-but-1-yl, 3-Cyclopentyloxy-but-1-yl, 4-Cyclopentyloxy-but-1-yl, 1-Cyclopentyloxy-but-2-yl, 2-Cyclopentyloxy-but-2-yl, 3-Cyclopentyloxy-but-2-yl, 3-Cyclopentyloxy-but-2-yl, 4-Cyclopentyloxy-but-2-yl, 1-(Cyclopentyloxymethyl)eth-1-yl, 1-(Cyclopentyloxymethyl)-1-(CH₃)-eth-1-yl, 1-(Cyclopentyloxymethyl)prop-1-yl, Cyclohexyloxymethyl, 1-Cyclohexyloxy-ethyl, 2-Cyclohexyloxy-ethyl, 1-Cyclohexyloxyprop-1-yl, 2-Cyclohexyloxy-prop-1-yl, 3-Cyclohexyloxy-prop-1-yl, 1-Cyclohexyloxy-but-1-yl, 2-Cyclohexyloxy-but-1-yl, 3-Cyclohexyloxy-but-1-yl, 4-Cyclohexyloxy-but-1-yl, 1-Cyclohexyloxy-but-2-yl, 2-Cyclohexyloxy-but-2-yl, 3-Cyclohexyloxybut-2-yl, 3-Cyclohexyloxy-but-2-yl, 4-Cyclohexyloxy-but-2-yl, 1-(Cyclohexyloxymethyl)eth-1-yl, 1-(Cyclohexyloxymethyl)-1-(CH₃)-eth-1-yl, 1-(Cyclohexyloxymethyl)-prop-1-yl, Cycloheptyloxymethyl, 1-Cycloheptyloxy-ethyl, 2-Cycloheptyloxy-ethyl, 1-Cycloheptyloxy-prop-1-yl, 2-Cycloheptyloxy-prop-1-yl, 3-Cycloheptyloxy-prop-1-yl, 1-Cycloheptyloxy-but-1-yl, 2-Cycloheptyloxy-but-1-yl, 3-Cycloheptyloxy-but-1-yl, 4-Cycloheptyloxy-but-1-yl, 1-Cycloheptyloxy-but-2-yl, 2-Cycloheptyloxy-but-2-yl, 3-Cycloheptyloxy-but-2-yl, 3-Cycloheptyloxy-but-2-yl, 4-Cycloheptyloxy-but-2-yl, 1-(Cycloheptyloxymethyl)eth-1-yl, 1-(Cycloheptyloxymethyl)-1-(CH₃)-eth-1-yl, 1-(Cycloheptyloxymethyl)prop-1-yl, Cyclooctyloxymethyl, 1-Cyclooctyloxy-ethyl, 2-Cyclooctyloxy-ethyl, 1-Cyclooctyloxy-prop-1-yl, 2-Cyclooctyloxy-prop-1-yl, 3-Cyclooctyloxy-prop-1-yl, 1-Cyclooctyloxy-but-1-yl, 2-Cyclooctyloxy-but-1-yl, 3-Cyclooctyloxy-but-1-yl, 4-Cyclooctyloxy-but-1-yl, 1-Cyclooctyloxy-but-2-yl, 2-Cyclooctyloxy-but-2-yl, 3-Cyclooctyloxy-but-2-yl, 3-Cyclooctyloxybut-2-yl, 4-Cyclooctyloxy-but-2-yl, 1-(Cyclooctyloxymethyl)-eth-1-yl, 1-(Cyclooctyloxymethyl)-1-(CH₃)-eth-1-yl oder 1-(Cyclooctyloxymethyl)prop-1-yl, insbesondere für C₃-C₆-Cycloalkoxymethyl oder 2-(C₃-C₆-Cycloalkoxy)ethyl.

Unter 3- bis 7-gliedrigem Heterocyclyl sind sowohl gesättigte, partiell oder vollständig ungesättigte als auch aromatische Heterocyclen mit ein bis drei Heteroatomen, ausgewählt unter einem, zwei oder drei Stickstoffatomen, einem oder zwei Sauerstoffund einem oder zwei Schwefelatomen, zu verstehen. Bevorzugt sind gesättigte Heterocyclen, und hierunter insbesondere solche mit einem oder zwei Heteroatomen, ausgewählt unter Sauerstoff und Schwefel. Die gesättigten Heterocylcen können ein Carbonyl- oder Thiocarbonyl-Ringglied enthalten.

Beispiele für gesättigte Heterocyclen, die ein Carbonyl- oder Thiocarbonyl-Ringglied enthalten können, sind:
Oxiranyl, Thiiranyl, Aziridin-1-yl, Aziridin-2-yl, Diaziridin-1-yl, Diaziridin-3-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl; 1,3-Dioxepan-6-yl, 1,3-Dithiepan-2-yl, 1,3-Dithiepan-2-yl, 1,3-Dithiepan-2-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl.

Gesättigtes, 5-oder 6-gliedriges, N-gebundenes Heterocyclyl steht beispielsweise für: Pyrrolidin-1-yl, Tetrahydropyrazol-1-yl, Piperidin-1-yl, Hexahydropyrimidin-1-yl, Piperazin-1-yl und Morpholin-1-yl.

Im Hinblick auf die Verwendung der erfindungsgemäßen Verbindungen der Formel I als Herbizide haben die Variablen Z, R¹, R² und R³, vorzugsweise folgende Bedeutungen, und zwar jeweils für sich alleine oder in Kombination:
- Z: O, S, oder eine Einfachbindung, insbesondere eine Einfachbindung;
- R¹: Wasserstoff, Fluor oder Chlor;
- R²: Halogen, insbesondere Fluor oder Chlor, oder Cyano;
- R³: für C₁-C₄-Alkyl, C₁-C₄-Halogenalkyl, Hydroxy-C₁-C₄-alkyl, Cyano-C₁-C₄-alkyl, C₁-C₄-Alkoxy-C₁-C₄-alkyl, Amino-C₁-C₄-alkyl, C₁-C₄-Alkylamino-C₁-C₄-alkyl, Di(C₁-C₄-alkyl)amino-C₁-C₄-alkyl, C₃-C₆-Alkenyl, C₃-C₆-Alkinyl, Hydroxycarbonyl-C₁-C₄-alkyl, (C₁-C₄-Alkoxy)carbonyl-C₁-C₄-alkyl, (C₁-C₄-Alkylthio)carbonyl-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl;
Phenyl, das gegebenenfalls einen Substituenten trägt, der ausgewählt ist unter Halogen, Cyano, C₁-C₆-Alkyl, C₁-C₆-Halogenalkyl, C₁-C₆-Alkoxy oder C₁-C₆-Halogenalkoxy;
gesättigtes C₃-C₇-Cycloalkyl, 3- bis 7-gliedriges, gesättigtes Heterocyclyl, das ein, zwei oder drei Heteroatome, ausgewählt unter Sauerstoff- und Schwefel-Atomen enthält, wobei jeder Cycloalkyl- und jeder Heterocyclyl-Ring ein Carbonyl- oder Thiocarbonyl-Ringglied enthalten kann; steht,
oder, sofern Z eine chemische Bindung bedeutet, R³ gewünschtenfalls auch für Wasserstoff, Hydroxy, Cyano, Mercapto, Amino, C₁-C₄-Alkylamino, Di-C₁-C₄-alkylamino, gesättigtes, 5- oder 6-gliedriges, N-gebundenes Stickstoffheterocyclyl, C₃-C₆-Cycloalkylamino, Halogen, -(CH₂)ₙ-CH(OH)-CH₂-R⁹, -(CH₂)ₙ-CH(Halogen)-CH₂-R⁹, -(CH₂)ₙ-CH₂-CH(Halogen)-R⁹, -(CH₂)ₙ-CH=CH-R⁹ oder -(CH₂)ₙ-CH=C(Halogen)-R⁹ stehen kann, worin R⁹ Hydroxycarbonyl, (C₁-C₄-Alkoxy)carbonyl, (C₁-C₄-Alkylthio)carbonyl, Aminocarbonyl, (C₁-C₄-Alkylamino)carbonyl oder Di(C₁-C₄-alkyl)aminocarbonyl bedeutet und n für 0 oder 1 steht;

Beipiele für besonders bevorzugte Reste R³ sind Wasserstoff, Methyl, Ethyl, n-Propyl, Isopropyl, n-Butyl, 2-Butyl, Isobutyl, tert.-Butyl, Propen-3-yl, Propargyl, 2-Buten-1-yl, 3-Buten-1-yl, Methoxymethyl, Methoxyethyl, Cyanomethyl, 2-Cyanoethyl, 2,2,2-Trifluorethyl, Chlormethyl, Fluormethyl, 2-Chlorethyl, 2-Chlormethyl, Methoxycarbonylmethyl, Ethoxycarbonylmethyl, n-Propoxycarbonylmethyl, Dimethylaminocarbonylmethyl, Hydroxymethyl, Aminomethyl, Methylaminomethyl, Dimethylaminomethyl, Diethylaminomethyl, 2-(Methylamino)ethyl, 2-(Dimethylamino)ethyl, 2-(Diethylamino)ethyl, (Methylthio)carbonylmethyl, (Ethylthio)carbonylmethyl, Phenyl, Cyclopropyl, Cyclobutyl, Cyclopentyl, Cyclohexyl, Cycloheptyl, Oxiran-2-yl, Oxetan-2-yl, Tetrahydrofuran-2-yl, Tetrahydropyran-2-yl, Oxepan-2-yl, Thiiran-2-yl, Thiethan-2-yl, Tetrahydrothiophen-2-yl, Tetrahydrothiopyran-2-yl, Thiepan-2-yl, Oxetan-3-yl, Tetrahydrofuran-3-yl, Tetrahydropyran-3-yl, Oxepan-3-yl, Thiethan-3-yl, Tetrahydrothiophen-3-yl, Tetrahydrothiopyran-3-yl, Thiepan-3-yl, Tetrahydropyran-4-yl, Oxepan-4-yl, Tetrahydrothiopyran-4-yl, Thiepan-4-yl, 2-Oxocyclopropyl, 2-Oxocyclobutyl, 2-Oxocyclopentyl, 2-Oxocyclohexyl, 2-Oxocycloheptyl, 2-Thioxocyclopropyl, 2-Thioxocyclobutyl, 2-Thioxocyclopentyl, 2-Thioxocyclohexyl, 2-Thioxocycloheptyl, 2-Oxooxetan-3-yl, 2-Oxothiethan-2-yl, 2-Oxotetrahydrothien-3-yl, 2-Oxotetrahydrothiopyran-3-yl, 2-Oxothiepan-3-yl, 2-Oxotetrahydrofuran-3-yl, 2-Oxotetrahydropyran-2-yl, 2-Oxooxepan2-yl, 2-Thioxooxetan-3-yl, 2-Thioxothiethan-2-yl, 2-Thioxotetrahydrothien-3-yl, 2-Thioxotetrahydrothiopyran-3-yl, 2-Thioxothiepan-3-yl, 2-Thioxotetrahydrofuran-3-yl, 2-Thioxotetrahydropyran-2-yl, 2-Thioxooxepan-2-yl, -CH₂-CH(Cl)-COOH, -CH₂-CH(Cl)-COOCH₃, -CH₂-CH(Cl)-COOCH₂CH₃, -CH₂-CH(Cl)-COOCH(CH₃)₂, -CH₂-CH(Cl)-COOCH₂CH(CH₃)₂, -CH₂-CH(Cl)-COOCH(CH₃)CH₂CH₃, -CH₂-CH(Cl)-COOC(CH₃)₃, -CH₂-CH(Cl)-COO-n-C₃H₇, -CH₂-CH(Cl)-COOn-C₄H₉, -CH₂-CH(Br)-COOH, -CH₂-CH(Br)-COOCH₃, -CH₂-CH(Br)-COOCH₂CH₃, -CH₂-CH(Br)-COOCH(CH₃)₂, -CH₂-CH(Br)-COOCH₂CH(CH₃)₂, -CH₂-CH(Br)-COOCH(CH₃)CH₂CH₃, -CH₂-CH(Br)-COOC(CH₃)₃, -CH₂-CH(Br)-COO-n-C₃H₇, -CH₂-CH(Br)-COO-n-C₄H₉, -CH=CH-COOH, -CH=CH-COOCH₃, -CH=CH-COOCH₂CH₃, -CH=CH-COOCH(CH₃)₂, -CH=CH-COOCH₂CH(CH₃)₂, -CH=CH-COOCH(CH₃)CH₂CH₃, -CH=CH-COOC(CH₃)₃, -CH=CH-COO-n-C₃H₇, -CH=CH-COO-n-C₄H₉, -CH=CH(Cl)-COOH, -CH=CH(Cl)-COOCH₃, -CH=CH(Cl)-COOCH₂CH₃, -CH=CH(Cl)-COOCH(CH₃)₂, -CH=CH(Cl)-COOCH₂CH(CH₃)₂, -CH=CH(Cl)-COOCH(CH₃)CH₂CH₃, -CH=CH(Cl)-COOC(CH₃)₃, -CH=CH(Cl)-COO-n-C₃H₇, -CH=CH(Cl)-COO-n-C₄H₉, -CH=CH(Br)-COOH, -CH=CH(Br)-COOCH₃, -CH=CH(Br)-COOCH₂CH₃, -CH=CH(Br)-COOCH(CH₃)₂, -CH=CH(Br)-COOCH₂CH(CH₃)₂, -CH=CH(Br)-COOCH(CH₃)CH₂CH₃, -CH=CH(Br)-COOC(CH₃)₃, -CH=CH(Br)-COO-n-C₃H₇, -CH=CH(Br)-COO-n-C₄H₉, -CH₂-CH(Cl)-CONH₂, -CH₂-CH(Cl)-CONHCH₃, -CH₂-CH(Cl)-CON(CH₃)₂, -CH₂-CH(Cl)-CONHC₂H₅, -CH₂-CH(Cl)-CON(CH₂CH₃)₂, -CH₂-CH(Cl)-CONH-n-C₃H₇, -CH₂-CH(Cl)-CON(n-C₃H₇)₂, -CH₂-CH(Cl)-CONH-n-C₄H₉, -CH₂-CH(Cl)-CON(n-C₄H₉)₂ , -CH₂-CH(Br)-CONH₂, -CH₂-CH(Br)-CONHCH₃, -CH₂-CH(Br)-CON(CH₃)₂, -CH₂-CH(Br)-CONH-C₂H₅, -CH₂-CH(Br)-CON(CH₂CH₃)₂, -CH₂-CH(Br)-CONH-n-C₃H₇, -CH₂-CH(Br)-CON(n-C₃H₇)₂, -CH₂-CH(Br)-CONH-n-C₄H₉, -CH₂-CH(Br)-CON(n-C₄H₉)₂ , -CH=CH-CONH₂, -CH=CH-CONHCH₃, -CH=CH-COON(CH₃)₂, -CH=CH-COONHC₂H₅, -CH=CH-CON(CH₂CH₃)₂, -CH=CH-CONH-n-C₃H₇, -CH=CH-CON(n-C₃H₇)₂, -CH=CH-CONH-n-C₄H₉, -CH=CH-CON(n-C₄H₉)₂, -CH=CH(Cl)-CONH₂, -CH=CH(Cl)-CONHCH₃, -CH=CH(Cl)-CON(CH₃)₂, -CH=CH(Cl)-CONHC₂H₅, -CH=CH(Cl)-CON(CH₂CH₃)₂, -CH=CH(Cl)-CONH-n-C₃H₇, -CH=CH(Cl)-CON(n-C₃H₇)₂, -CH=CH(Cl)-CONH-n-C₄H₉, -CH=CH(Cl)-CON(n-C₄H₉)₂ , -CH=CH(Br)-CONH₂, -CH=CH(Br)-CONHCH₃, -CH=CH(Br)-CON(CH₃)₂, -CH=CH(Br)-CONH-C₂H₅, -CH=CH(Br)-CON(CH₂CH₃)₂, -CH=CH(Br)-CONH-n-C₃H₇, -CH=CH(Br)-CON(n-C₃H₇)₂, -CH=CH(Br)-CONH-n-C₄H₉, -CH=CH(Br)-CON(n-C₄H₉)₂ .

Ganz besonders bevorzugt steht R³ für C₁-C₄-Alkyl, C₁-C₄-Halogenalkyl oder Phenyl, das gegebenenfalls einen Substituenten ausgewählt unter Halogen, C₁-C₄-Alkyl oder C₁-C₄-Halogenalkyl aufweist, gesättigtes C₃-C₇-Cycloalkyl, insbesondere Cyclopropyl, gesättigtes C₃-C₇-Cycloalkyl-C₁-C₄-alkyl, insbesondere Cyclopropylmethyl, 3- bis 7-gliedriges, gesättigtes Heterocyclyl, das ein, zwei oder drei Heteroatome, ausgewählt unter Sauerstoff- und Schwefel-Atomen enthält, wobei jeder Cycloalkyl- und jeder Heterocyclyl-Ring ein Carbonyl- oder Thiocarbonyl-Ringglied enthalten kann.

In anderen bevorzugten Verbindungen der Formel I steht Het für einen über einen Imidstickstoff gebundenen Cyclus der Formel II-4, II-5, II-6, II-10, II-14 oder II-17, insbesondere II-5 oder II-6.

In weiteren bevorzugten Verbindungen der Formel I steht Het für einen Stickstoffhetercyclus, der wenigstens eine Carbonyl- oder Thiocarbonylfunktion und wenigstens eine endocyclische Hydrazonstruktur aufweist und der ausgewählt ist unter den Resten der allgemeinen Formel II-1, II-2, II-11 und II-12.

Im Hinblick auf die herbizide und/oder desikkante oder defoliante Wirksamkeit steht Het vorzugsweise für einen Rest der allgemeinen Formel II-1, II-2, II-5, II-6 oder II-12 und insbesondere für einen Rest der Formel II-2, II-5 und II-12.

In Het stehen X und X' vorzugsweise für Sauerstoff. Y steht ebenfalls bevorzugt für Sauerstoff. Q in Formel II-1 steht ebenfalls bevorzugt für Sauerstoff.

In den Resten Het haben die Variablen R⁴, R⁴', R⁴" unabhängig voneinander vorzugsweise die folgenden Bedeutungen: Wasserstoff, C₁-C₆-Alkyl, Halogen, Amino, C₁-C₆-Halogenalkyl, C₁-C₆-Halogenalkoxy, C₁-C₆-Halogenalkylthio oder C₁-C₆-Alkylsulfonyl.

In den Resten Het haben die Variablen R⁵ und R^{5'} unabhängig voneinander vorzugsweise die folgenden Bedeutungen: Wasserstoff, C₁-C₆-Alkyl, Amino oder C₁-C₆-Halogenalkyl.

Bevorzugte Verbindungen der allgemeinen Formel I können auch Reste Het aufweisen, worin zwei der Reste R⁴, R^{4'}, R^{4"}, R⁵ und R^{5'} gemeinsam mit dem Cyclus an den sie gebunden sind, einen 5-, 6-oder 7-gliedrigen Ring bilden, der ein- oder zweifach ungesättigt oder gesättigt sein kann und der ein oder zwei Heteroatome, ausgewählt unter Sauerstoff und Schwefel als Ringlied enthalten kann.

Unter den Verbindungen der Formel I, worin Het für einen Cyclus der Formel II-1 steht, sind solche Verbindungen bevorzugt, in denen X für Sauerstoff steht. In diesen Verbindungen steht Q vorzugsweise ebenfalls für Sauerstoff. In diesen Verbindungen steht R⁴ vorzugsweise für C₁-C₄-Alkyl und insbesondere für tert.-Butyl.

Unter den Verbindungen der Formel I, worin Het für einen Cyclus der Formel II-2 steht, sind solche Verbindungen bevorzugt, in denen X für Sauerstoff steht. In diesen Verbindungen steht R⁵ vorzugsweise für Wasserstoff oder C₁-C₄-Alkyl und insbesondere für Methyl. R⁵ steht vorzugsweise für C₁-C₄-Halogenalkyl und insbesondere für Trifluormethyl.

Unter den Verbindungen der Formel I, worin Het für einen Cyclus der Formel II-5 steht, sind solche Verbindungen bevorzugt, in denen X und X' für Sauerstoff stehen. In diesen Verbindungen stehen R⁴ und R^{4'} vorzugsweise für Wasserstoff oder C₁-C₄-Alkyl oder bilden insbesondere mit den Kohlenstoffatomen, an die sie gebunden sind, einen 6-gliedrigen Carbocyclus und besonders bevorzugt einen Cyclohexen- oder Benzol-Ring.

Unter den Verbindungen der Formel I, worin Het für einen Cyclus der Formel II-6 steht, sind solche Verbindungen bevorzugt, in denen X und X' für Sauerstoff stehen. Y steht ebenfalls vorzugsweise für Sauerstoff. In diesen Verbindungen stehen R⁶ und R⁷ vor-zugsweise für C₁-C₄-Alkyl, insbesondere Methyl oder bilden vorzugsweise mit dem Kohlenstoffatom an das sie gebunden sind, einen 5- oder 6-gliedrigen, gesättigten Carbocyclus.

Unter den Verbindungen der Formel I, worin Het für einen, Cyclus der Formel II-12 steht, sind solche Verbindungen bevorzugt, in denen X für Sauerstoff steht. In diesen Verbindungen steht R⁴ vorzugsweise für Wasserstoff, Amino, C₁-C₄-Alkyl und insbesondere für Methyl. R^{4'} steht vorzugsweise für C₁-C₄-Halogenalkyl und insbesondere für Trifluormethyl, oder für C₁-C₄-Alkylsulfonyl, insbesondere Methylsulfonyl. R^{4''} steht insbesondere für Wasserstoff.

Bevorzugt sind insbesondere Verbindungen der allgemeinen Formel I-A., worin R² Chlor, Z eine Einfachbindung und Het einen Rest der Formel II-1 bedeuten, worin X und Q für O und R⁴ für tert.-Butyl stehen, und R¹ und R³ die in Tabelle 1 angegebenen Bedeutungen aufweisen (Verbindungen I-Aa.1 bis I-Aa.558).

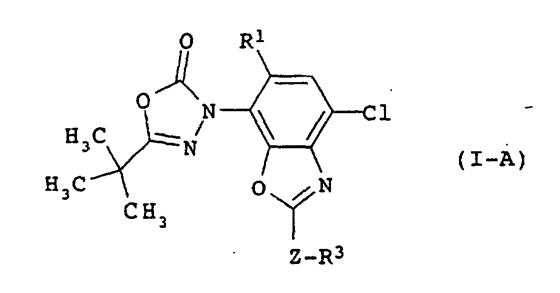

**Tabelle 1:**

| Nr. | R¹ | **R**^{**3**} bzw. **R**^{**3'**} |
|---|---|---|
| 1 | H | H |
| 2 | H | Me |
| 3 | H | Et |
| 4 | H | n-Pr |
| 5 | H | n-Bu |
| 6 | H | i-Pr |
| 7 | H | CH₂-CH(CH₃)₂ |
| 8 | H | CH(Me)-C₂H₅ |
| 9 | H | C(CH₃)₃ |
| 10 | H | CH₂-CH=CH₂ |
| 11 | H | CH₂-CH=CH-CH₃ |
| 12 | H | CH₂CH₂-CH=CH₂ |
| 13 | H | CH₂-C≡CH |
| 14 | H | CHMe-C≡CH |
| 15 | H | CH₂OMe |
| 16 | H | CH₂CH₂OMe |
| 17 | H | CH₂OCH₂CH₃ |
| 18 | H | CH₂CH₂OCH₂CH₃ |
| 19 | H | CH₂CN |
| 20 | H | CH₂CH₂CN |
| 21 | H | CH₂CF₃ |
| 22 | H | CH₂Cl |
| 23 | H | CH₂F |
| 24 | H | CH₂CH₂Cl |
| 25 | H | CH₂CH₂F |
| 26 | H | CH₂COOMe |
| 27 | H | CH₂COOEt |
| 28 | H | CH₂COO n-Pr |
| 29 | H | CH₂CONMe₂ |
| 30 | H | CH₂OH |
| 31 | H | CH₂NH₂ |
| 32 | H | CH₂NHCH₃ |
| 33 | H | CH₂N(CH₃)₂ |
| 34 | H | CH₂N(C₂H₅)₂ |
| 35 | H | CH₂CH₂NHCH₃ |
| 36 | H | CH₂CH₂N(CH₃)₂ |
| 37 | H | CH₂CH₂N(C₂H₅)₂ |
| 38 | H | CH₂C(O)SMe |
| 39 | H | CH₂C(O)SC₂H₅ |
| 40 | H | Phenyl |
| 41 | H | 4-Me-Phenyl |
| 42 | H | 4-Cl-Phenyl |
| 43 | H | 3-Me-Phenyl |
| 44 | H | 2-Cl-Phenyl |
| 45 | H | 2-Me-Phenyl |
| 46 | H | 2-Cl-Phenyl |
| 47 | H | Cyclopropyl |
| 48 | H | Cyclobutyl |
| 49 | H | Cyclopentyl |
| 50 | H | Cyclohexyl |
| 51 | H | Cycloheptyl |
| 52 | H | Oxiran-2-yl |
| 53 | H | Oxetan-2-yl |
| 54 | H | Tetrahydrofuran-2-yl |
| 55 | H | Tetrahydropyran-2-yl |
| 56 | H | Oxepan-2-yl |
| 57 | H | Thiiran-2-yl |
| 58 | H | Thietan-2-yl |
| 59 | H | Tetrahydrothiofuran-2-yl |
| 60 | H | Tetrahydrothiopyran-2-yl |
| 61 | H | Thiepan-2-yl |
| 62 | H | Oxetan-3-yl |
| 63 | H | Tetrahydrofuran-3-yl |
| 64 | H | Tetrahydropyran-3-yl |
| 65 | H | Oxepan-3-yl |
| 66 | H | Thietan-3-yl |
| 67 | H | Tetrahydrothiofuran-3-yl |
| 68 | H | Tetrahydrothiopyran-3-yl |
| 69 | H | Thiepan-3-yl- |
| 70 | H | Tetrahydropyran-4-yl |
| 71 | H | Oxepan-4-yl |
| 72 | H | Tetrahydrothiopyran-4-yl |
| 73 | H | Thiepan-4-yl |
| 74 | H | 2-Oxocyclopropyl |
| 75 | H | 2-Oxocyclobutyl |
| 76 | H | 2-Oxocyclopentyl |
| 77 | H | 2-Oxocyclohexyl |
| 78 | H | 2-Oxocycloheptyl |
| 79 | H | 2-Thioxo-cyclopropyl |
| 80 | H | 2-Thioxo-cyclobutyl |
| 81 | H | 2-Oxo-oxetan-3-yl |
| 82 | H | 2-Oxo-tetrahydrofuran-3-yl |
| 83 | H | 2-Oxo-tetrahydropyran-3-yl |
| 84 | H | 2-Oxo-oxepan-3-yl |
| 85 | H | 2-Thioxo-thietan-3-yl |
| 86 | H | 2-Thioxo-tetrahydrothien-3-yl |
| 87 | H | 2-Thioxo-tetrahydrothiopyran-3-yl |
| 88 | H | 2-Thioxo-thiepan-3-yl |
| 89 | H | 2-Thioxo-oxetan-3-yl |
| 90 | H | 2-Thioxo-tetrahydrofuran-3-yl |
| 91 | H | 2-Thioxo-tetrahydropyran-3-yl |
| 92 | H | 2-Thioxo-oxepan-3-yl |
| 93 | H | 2-Oxo-thietan-3-yl |
| 94 | H | 2-Oxo-tetrahydrothien-3-yl |
| 95 | H | 2-Oxo-tetrahydrothiopyran-3-yl |
| 96 | H | 2-Oxo-thiepan-3-yl |
| 97 | H | -CH₂-CH(Cl)-CO-OCH₃ |
| 98 | H | -CH₂-CH(Cl)-CO-OC₂H₅ |
| 99 | H | -CH₂-CH(Cl)-CO-O(n-C₃H₇) |
| 100 | H | -CH₂-CH(Cl)-CO-O(n-C₄H₉) |
| 101 | H | -CH₂-CH(Cl)-CO-OCH(CH₃)₂ |
| 102 | H | -CH₂-CH(Cl)-CO-OCH₂-CH(CH₃)₂ |
| 103 | H | -CH₂-CH(Cl)-CO-OCH(CH₃)-C₂H₅ |
| 104 | H | -CH₂-CH(Cl)-CO-OC(CH₃)₃ |
| 105 | H | -CH₂-CH(Br)-CO-OH |
| 106 | H | -CH₂-CH(Br)-CO-OCH₃ |
| 107 | H | -CH₂-CH(Br)-CO-OC₂H₅ |
| 108 | H | -CH₂-CH(Cl)-CO-OH |
| 109 | H | -CH₂-CH(Br)-CO-O(n-C₃H₇) |
| 110 | H | -CH₂-CH(Br)-CO-O(n-C₄H₉) |
| 111 | H | -CH₂-CH(Br)-CO-OCH(CH₃)₂ |
| 112 | H | -CH₂-CH(Br)-CO-OCH₂-CH(CH₃)₂ |
| 113 | H | -CH₂-CH(Br)-CO-OCH(CH₃)-C₂H₅ |
| 114 | H | -CH₂-CH(Br)-CO-OC(CH₃)₃ |
| 115 | H | -CH=CH-CO-OH |
| 116 | H | -CH=CH-CO-OCH₃ |
| 117 | H | -CH=CH-CO-OC₂H₅ |
| 118 | H | -CH=CH-CO-O(n-C₃H₇) |
| 119 | H | -CH=CH-CO-O(n-C₄H₉) |
| 120 | H | -CH=CH-CO-OCH(CH₃)₂ |
| 121 | H | -CH=CH-CO-OCH₂-CH(CH₃)₂ |
| 122 | H | -CH=CH-CO-OCH(CH₃)-C₂H₅ |
| 123 | H | -CH=CH-CO-OC(CH₃)₃ |
| 124 | H | -CH=C(Cl)-CO-OH |
| 125 | H | -CH=C(Cl)-CO-OCH₃ |
| 126 | H | -CH=C(Cl)-CO-OC₂H₅ |
| 127 | H | -CH=C(Cl)-CO-O(n-C₃H₇) |
| 128 | H | -CH=C(Cl)-CO-O(n-C₄H₉) |
| 129 | H | -CH=C(Cl)-CO-OCH(CH₃)₂ |
| 130 | H | -CH=C(Cl)-CO-OCH₂-CH(CH₃)₂ |
| 131 | H | -CH=C(Cl)-CO-OCH(CH₃)-C₂H₅ |
| 132 | H | -CH=C(Cl)-CO-OC(CH₃)₃ |
| 133 | H | -CH=C(Br)-CO-OH |
| 134 | H | -CH=C(Br)-CO-OCH₃ |
| 135 | H | -CH=C(Br)-CO-OC₂H₅ |
| 136 | H | -CH=C(Br)-CO-O(n-C₃H₇) |
| 137 | H | -CH=C(Br)-CO-O(n-C₄H₉) |
| 138 | H | -CH=C(Br)-CO-OCH(CH₃)₂ |
| 139 | H | -CH=C(Br)-CO-OCH₂-CH(CH₃)₂ |
| 140 | H | -CH=C(Br)-CO-OCH(CH₃)-C₂H₅ |
| 141 | H | -CH=C(Br)-CO-OC(CH₃)₃ |
| 142 | H | -CH₂-CH(Cl)-CO-NH₂ |
| 143 | H | -CH₂-CH(Cl)-CO-NH-CH₃ |
| 144 | H | -CH₂-CH(Cl)-CO-N(CH₃)₂ |
| 145 | H | -CH₂-CH(Cl)-CO-NH-C₂H₅ |
| 146 | H | -CH₂-CH(Cl)-CO-N(C₂H₅)₂ |
| 147 | H | -CH₂-CH(Cl)-CO-NH-(n-C₃H₇) |
| 148 | H | -CH₂-CH(Cl)-CO-N(n-C₃H₇)₂ |
| 149 | H | -CH₂-CH(Cl)-CO-NH-(n-C₄H₉) |
| 150 | H | -CH₂-CH(Cl)-CO-N(n-C₄H₉)₂ |
| 151 | H | -CH₂-CH(Br)-CO-NH₂ |
| 152 | H | -CH₂-CH(Br)-CO-NH-CH₃ |
| 153 | H | -CH₂-CH(Br)-CO-N(CH₃)₂ |
| 154 | H | -CH₂-CH(Br)-CO-NH-C₂H₅ |
| 155 | H | -CH₂-CH(Br)-CO-N(C₂H₅)₂ |
| 156 | H | -CH₂-CH(Br)-CO-NH-(n-C₃H₇) |
| 157 | H | -CH₂-CH(Br)-CO-N(n-C₃H₇)₂ |
| 158 | H | -CH₂-CH(Br)-CO-NH-(n-C₄H₉) |
| 159 | H | -CH₂-CH(Br)-CO-N(n-C₄H₉)₂ |
| 160 | H | -CH=CH-CO-NH₂ |
| 161 | H | -CH=CH-CO-NH-CH₃ |
| 162 | H | -CH=CH-CO-N(CH₃)₂ |
| 163 | H | -CH=CH-CO-NH-C₂H₅ |
| 164 | H | -CH=CH-CO-N(C₂H₅)₂ |
| 165 | H | -CH=CH-CO-NH-(n-C₃H₇) |
| 166 | H | -CH=CH-CO-N(n-C₃H₇)₂ |
| 167 | H | -CH=CH-CO-NH-(n-C₄H₉) |
| 168 | H | -CH=CH-CO-N(n-C₄H₉)₂ |
| 169 | H | -CH=C(Cl)-CO-NH₂ |
| 170 | H | -CH=C(Cl)-CO-NH-CH₃ |
| 171 | H | -CH=C(Cl)-CO-N(CH₃)₂ |
| 172 | H | -CH=C(Cl)-CO-NH-C₂H₅ |
| 173 | H | -CH=C(Cl)-CO-N(C₂H₅)₂ |
| 174 | H | -CH=C(Cl)-CO-NH-(n-C₃H₇) |
| 175 | H | -CH=C(Cl)-CO-N(n-C₃H₇)₂ |
| 176 | H | -CH=C(Cl)-CO-NH-(n-C₄H₉) |
| 177 | H | -CH=C(Cl)-CO-N(n-C₄H₉)₂ |
| 178 | H | -CH=C(Br)-CO-NH₂ |
| 179 | H | -CH=C(Br)-CO-NH-CH₃ |
| 180 | H | -CH=C(Br)-CO-NH(CH₃)₂ |
| 181 | H | -CH=C(Br)-CO-NH-C₂H₅ |
| 182 | H | -CH=C(Br)-CO-N(C₂H₅)₂ |
| 183 | H | -CH=C(Br)-CO-NH-(n-C₃H₇) |
| 184 | H | -CH=C(Br)-CO-N(n-C₃H₇)₂ |
| 185 | H | -CH=C(Br)-CO-NH-(n-C₄H₉) |
| 186 | H | -CH=C(Br)-CO-N(n-C₄H₉)₂ |
| 187 | Cl | H |
| 188 | Cl | Me |
| 189 | Cl | Et |
| 190 | Cl | n-Pr |
| 191 | Cl | n-Bu |
| 192 | Cl | i-Pr |
| 193 | Cl | CH₂-CH(CH₃)₂ |
| 194 | Cl | CH(Me)-C₂H₅ |
| 195 | Cl | C(CH₃)₃ |
| 196 | Cl | CH₂-CH=CH₂ |
| 197 | Cl | CH₂-CH=CH-CH₃ |
| 198 | Cl | CH₂CH₂-CH=CH₂ |
| 199 | Cl | CH₂-C≡CH |
| 200 | Cl | CHMe-C≡CH |
| 201 | Cl | CH₂OMe |
| 202 | Cl | CH₂CH₂OMe |
| 203 | Cl | CH₂OCH₂CH₃ |
| 204 | Cl | CH₂CH₂OCH₂CH₃ |
| 205 | Cl | CH₂CN |
| 206 | Cl | CH₂CH₂CN |
| 207 | Cl | CH₂CF₃ |
| 208 | Cl | CH₂Cl |
| 209 | Cl | CH₂F |
| 210 | Cl | CH₂CH₂Cl |
| 211 | Cl | CH₂CH₂F |
| 212 | Cl | CH₂COOMe |
| 213 | Cl | CH₂COOEt |
| 214 | Cl | CH₂COO n-Pr |
| 215 | Cl | CH₂CONMe₂ |
| 216 | Cl | CH₂OH |
| 217 | Cl | CH₂NH₂ |
| 218 | Cl | CH₂NHCH₃ |
| 219 | Cl | CH₂N(CH₃)₂ |
| 220 | Cl | CH₂N(C₂H₅)₂ |
| 221 | Cl | CH₂CH₂NHCH₃ |
| 222 | Cl | CH₂CH₂N(CH₃)₂ |
| 223 | Cl | CH₂CH₂N(C₂Cl₅)₂ |
| 224 | Cl | CH₂C(O)SMe |
| 225 | Cl | CH₂C(O)SC₂H₅ |
| 226 | Cl | Phenyl |
| 227 | Cl | 4-Me-Phenyl |
| 228 | Cl | 4-Cl-Phenyl |
| 229 | Cl | 3-Me-Phenyl |
| 230 | Cl | 2-Cl-Phenyl |
| 231 | Cl | 2-Me-Phenyl |
| 232 | Cl | 2-Cl-Phenyl |
| 233 | Cl | Cyclopropyl |
| 234 | Cl | Cyclobutyl |
| 235 | Cl | Cyclopentyl |
| 236 | Cl | Cyclohexyl |
| 237 | Cl | Cycloheptyl |
| 238 | Cl | Oxiran-2-yl |
| 239 | Cl | Oxetan-2-yl |
| 240 | Cl | Tetrahydrofuran-2-yl |
| 241 | Cl | Tetrahydropyran-2-yl |
| 242 | Cl | Oxepan-2-yl |
| 243 | Cl | Thiiran-2-yl |
| 244 | Cl | Thietan-2-yl |
| 245 | Cl | Tetrahydrothiofuran-2-yl |
| 246 | Cl | Tetrahydrothiopyran-2-yl |
| 247 | Cl | Thiepan-2-yl |
| 248 | Cl | Oxetan-3-yl |
| 249 | Cl | Tetrahydrofuran-3-yl |
| 250 | Cl | Tetrahydropyran-3-yl |
| 251 | Cl | Oxepan-3-yl |
| 252 | Cl | Thietan-3-yl |
| 253 | Cl | Tetrahydrothiofuran-3-yl |
| 254 | Cl | Tetrahydrothiopyran-3-yl |
| 255 | Cl | Thiepan-3-yl- |
| 256 | Cl | Tetrahydropyran-4-yl |
| 257 | Cl | Oxepan-4-yl |
| 258 | Cl | Tetrahydrothiopyran-4-yl |
| 259 | Cl | Thiepan-4-yl |
| 260 | Cl | 2-Oxocyclopropyl |
| 261 | Cl | 2-Oxocyclobutyl |
| 262 | Cl | 2-Oxocyclopentyl |
| 263 | Cl | 2-Oxocyclohexyl |
| 264 | Cl | 2-Oxocycloheptyl |
| 265 | Cl | 2-Thioxo-cyclopropyl |
| 266 | Cl | 2-Thioxo-cyclobutyl |
| 267 | Cl | 2-Oxo-oxetan-3-yl |
| 268 | Cl | 2-Oxo-tetrahydrofuran-3-yl |
| 269 | Cl | 2-Oxo-tetrahydropyran-3-yl |
| 270 | Cl | 2-Oxo-oxepan-3-yl |
| 271 | Cl | 2-Thioxo-thietan-3-yl |
| 272 | Cl | 2-Thioxo-tetrahydrothien-3-yl |
| 273 | Cl | 2-Thioxo-tetrahydrothiopyran-3-yl |
| 274 | Cl | 2-Thioxo-thiepan-3-yl |
| 275 | Cl | 2-Thioxo-oxetan-3-yl |
| 276 | Cl | 2-Thioxo-tetrahydrofuran-3-yl |
| 277 | Cl | 2-Thioxo-tetrahydropyran-3-yl |
| 278 | Cl | 2-Thioxo-oxepan-3-yl |
| 279 | Cl | 2-Oxo-thietan-3-yl |
| 280 | Cl | 2-Oxo-tetrahydrothien-3-yl |
| 281 | Cl | 2-Oxo-tetrahydrothiopyran-3-yl |
| 282 | Cl | 2-Oxo-thiepan-3-yl |
| 283 | Cl | -CH₂-CH(Cl)-CO-OCH₃ |
| 284 | Cl | -CH₂-CH(Cl)-CO-OC₂H₅ |
| 285 | Cl | -CH₂-CH(Cl)-CO-O(n-C₃H₇) |
| 286 | Cl | -CH₂-CH(Cl)-CO-O(n-C₄H₉) |
| 287 | Cl | -CH₂-CH(Cl)-CO-OCH(CH₃)₂ |
| 288 | Cl | -CH₂-CH(Cl)-CO-OCH₂-CH(CH₃)₂ |
| 289 | Cl | -CH₂-CH(Cl)-CO-OCH(CH₃)-C₂H₅ |
| 290 | Cl | -CH₂-CH(Cl)-CO-OC(CH₃)₃ |
| 291 | Cl | -CH₂-CH(Br)-CO-OH |
| 292 | Cl | -CH₂-CH(Br)-CO-OCH₃ |
| 293 | Cl | -CH₂-CH(Br)-CO-OC₂H₅ |
| 294 | Cl | -CH₂-CH(Cl)-CO-OH |
| 295 | Cl | -CH₂-CH(Br)-CO-O(n-C₃H₇) |
| 296 | Cl | -CH₂-CH(Br)-CO-O(n-C₄H₉) |
| 297 | Cl | -CH₂-CH(Br)-CO-OCH(CH₃)₂ |
| 298 | Cl | -CH₂-CH(Br)-CO-OCH₂-CH(CH₃)₂ |
| 299 | Cl | -CH₂-CH(Br)-CO-OCH(CH₃)-C₂H₅ |
| 300 | Cl | -CH₂-CH(Br)-CO-OC(CH₃)₃ |
| 301 | Cl | -CH=CH-CO-OH |
| 302 | Cl | -CH=CH-CO-OCH₃ |
| 303 | Cl | -CH=CH-CO-OC₂H₅ |
| 304 | Cl | -CH=CH-CO-O(n-C₃H₇) |
| 305 | Cl | -CH=CH-CO-O(n-C₄H₉) |
| 306 | Cl | -CH=CH-CO-OCH(CH₃)₂ |
| 307 | Cl | -CH=CH-CO-OCH₂-CH(CH₃)₂ |
| 308 | Cl | -CH=CH-CO-OCH(CH₃)-C₂H₅ |
| 309 | Cl | -CH=CH-CO-OC(CH₃)₃ |
| 310 | Cl | -CH=C(Cl)-CO-OH |
| 311 | Cl | -CH=C(Cl)-CO-OCH₃ |
| 312 | Cl | -CH=C(Cl)-CO-OC₂H₅ |
| 313 | Cl | -CH=C(Cl)-CO-O(n-C₃H₇) |
| 314 | Cl | -CH=C(Cl)-CO-O(n-C₄H₉) |
| 315 | Cl | -CH=C(Cl)-CO-OCH(CH₃)₂ |
| 316 | Cl | -CH=C(Cl)-CO-OCH₂-CH(CH₃)₂ |
| 317 | Cl | -CH=C(Cl)-CO-OCH(CH₃)-C₂H₅ |
| 318 | Cl | -CH=C(Cl)-CO-OC(CH₃)₃ |
| 319 | Cl | -CH=C(Br)-CO-OH |
| 320 | Cl | -CH=C(Br)-CO-OCH₃ |
| 321 | Cl | -CH=C(Br)-CO-OC₂H₅ |
| 322 | Cl | -CH=C(Br)-CO-O(n-C₃H₇) |
| 323 | Cl | -CH=C(Br)-CO-O(n-C₄H₉) |
| 324 | Cl | -CH=C(Br)-CO-OCH(CH₃)₂ |
| 325 | Cl | -CH=C(Br)-CO-OCH₂-CH(CH₃)₂ |
| 326 | Cl | -CH=C(Br)-CO-OCH(CH₃)-C₂H₅ |
| 327 | Cl | -CH=C(Br)-CO-OC(CH₃)₃ |
| 328 | Cl | -CH₂-CH(Cl)-CO-NH₂ |
| 329 | Cl | -CH₂-CH(Cl)-CO-NH-CH₃ |
| 330 | Cl | -CH₂-CH(Cl)-CO-N(CH₃)₂ |
| 331 | Cl | -CH₂-CH(Cl)-CO-NH-C₂H₅ |
| 332 | Cl | -CH₂-CH(Cl)-CO-N(C₂H₅)₂ |
| 333 | Cl | -CH₂-CH(Cl)-CO-NH-(n-C₃H₇) |
| 334 | Cl | -CH₂-CH(Cl)-CO-N(n-C₃H₇)₂ |
| 335 | Cl | -CH₂-CH(Cl)-CO-NH-(n-C₄H₉) |
| 336 | Cl | -CH₂-CH(Cl)-CO-N(n-C₄H₉)₂ |
| 337 | Cl | -CH₂-CH(Br)-CO-NH₂ |
| 338 | Cl | -CH₂-CH(Br)-CO-NH-CH₃ |
| 339 | Cl | -CH₂-CH(Br)-CO-N(CH₃)₂ |
| 340 | Cl | -CH₂-CH(Br)-CO-NH-C₂H₅ |
| 341 | Cl | -CH₂-CH(Br)-CO-N(C₂H₅)₂ |
| 342 | Cl | -CH₂-CH(Br)-CO-NH-(n-C₃H₇) |
| 343 | Cl | -CH₂-CH(Br)-CO-N(n-C₃H₇)₂ |
| 344 | Cl | -CH₂-CH(Br)-CO-NH-(n-C₄H₉) |
| 345 | Cl | -CH₂-CH(Br)-CO-N(n-C₄H₉)₂ |
| 346 | Cl | -CH=CH-CO-NH₂ |
| 347 | Cl | -CH=CH-CO-NH-CH₃ |
| 348 | Cl | -CH=CO-CO-N(CH₃)₂ |
| 349 | Cl | -CH=CH-CO-NH-C₂H₅ |
| 350 | Cl | -CH=CH-CO-N(C₂H₅)₂ |
| 351 | Cl | -CH=CH-CO-NH-(n-C₃H₇) |
| 352 | Cl | -CH=CH-CO-N(n-C₃H₇)₂ |
| 353 | Cl | -CH=CH-CO-NH-(n-C₄H₉) |
| 354 | Cl | -CH=CH-CO-N(n-C₄H₉)₂ |
| 355 | Cl | -CH=C(Cl)-CO-NH₂ |
| 356 | Cl | -CH=C(Cl)-CO-NH-CH₃ |
| 357 | Cl | -CH=C(Cl)-CO-N(CH₃)₂ |
| 358 | Cl | -CH=C(Cl)-CO-NH-C₂H₅ |
| 359 | Cl | -CH=C(Cl)-CO-N(C₂H₅)₂ |
| 360 | Cl | -CH=C(Cl)-CO-NH-(n-C₃H₇) |
| 361 | Cl | -CH=C(Cl)-CO-N(n-C₃H₇)₂ |
| 362 | Cl | -CH=C(Cl)-CO-NH-(n-C₄H₉) |
| 363 | Cl | -CH=C(Cl)-CO-N(n-C₄H₉)₂ |
| 364 | Cl | -CH=C(Br)-CO-NH₂ |
| 365 | Cl | -CH=C(Br)-CO-NH-CH₃ |
| 366 | Cl | -CH=C(Br)-CO-NH(CH₃)₂ |
| 367 | Cl | -CH=C(Br)-CO-NH-C₂H₅ |
| 368 | Cl | -CH=C(Br)-CO-N(C₂H₅)₂ |
| 369 | Cl | -CH=C(Br)-CO-NH-(n-C₃H₇) |
| 370 | Cl | -CH=C(Br)-CO-N(n-C₃H₇)₂ |
| 371 | Cl | -CH=C(Br)-CO-NH-(n-C₄H₉) |
| 372 | Cl | -CH=C(Br)-CO-N(n-C₄H₉)₂ |
| 373 | F | H |
| 374 | F | Me |
| 375 | F | Et |
| 376 | F | n-Pr |
| 377 | F | n-Bu |
| 378 | F | i-Pr |
| 379 | F | CH₂-CH(CH₃)₂ |
| 380 | F | CH(Me)-C₂H₅ |
| 381 | F | C(CH₃)₃ |
| 382 | F | CH₂-CH=CH₂ |
| 383 | F | CH₂-CH=CH-CH₃ |
| 384 | F | CH₂CH₂-CH=CH₂ |
| 385 | F | CH₂-C≡CH |
| 386 | F | CHMe-C≡CH |
| 387 | F | CH₂OMe |
| 388 | F | CH₂CH₂OMe |
| 389 | F | CH₂OCH₂CH₃ |
| 390 | F | CH₂CH₂OCH₂CH₃ |
| 391 | F | CH₂CN |
| 392 | F | CH₂CH₂CN |
| 393 | F | CH₂CF₃ |
| 394 | F | CH₂Cl |
| 395 | F | CH₂F |
| 396 | F | CH₂CH₂Cl |
| 397 | F | CH₂CH₂F |
| 398 | F | CH₂COOMe |
| 399 | F | CH₂COOEt |
| 400 | F | CH₂COO n-Pr |
| 401 | F | CH₂CONMe₂ |
| 402 | F | CH₂OH |
| 403 | F | CH₂NH₂ |
| 404 | F | CH₂NHCH₃ |
| 405 | F | CH₂N(CH₃)₂ |
| 406 | F | CH₂N(C₂H₅)₂ |
| 407 | F | CH₂CH₂NHCH₃ |
| 408 | F | CH₂CH₂N(CH₃)₂ |
| 409 | F | CH₂CH₂N(C₂H₅)₂ |
| 410 | F | CH₂C(O)SMe |
| 411 | F | CH₂C(O)SC₂H₅ |
| 412 | F | Phenyl |
| 413 | F | 4-Me-Phenyl |
| 414 | F | 4-Cl-Phenyl |
| 415 | F | 3-Me-Phenyl |
| 416 | F | 2-Cl-Phenyl |
| 417 | F | 2-Me-Phenyl |
| 418 | F | 2-Cl-Phenyl |
| 419 | F | Cyclopropyl |
| 420 | F | Cyclobutyl |
| 421 | F | Cyclopentyl |
| 422 | F | Cyclohexyl |
| 423 | F | Cycloheptyl |
| 424 | F | Oxiran-2-yl |
| 425 | F | Oxetan-2-yl |
| 426 | F | Tetrahydrofuran-2-yl |
| 427 | F | Tetrahydropyran-2-yl |
| 428 | F | Oxepan-2-yl |
| 429 | F | Thiiran-2-yl |
| 430 | F | Thietan-2-yl |
| 431 | F | Tetrahydrothiofuran-2-yl |
| 432 | F | Tetrahydrothiopyran-2-yl |
| 433 | F | Thiepan-2-yl |
| 434 | F | Oxetan-3-yl |
| 435 | F | Tetrahydrofuran-3-yl |
| 436 | F | Tetrahydropyran-3-yl |
| 437 | F | Oxepan-3-yl |
| 438 | F | Thietan-3-yl |
| 439 | F | Tetrahydrothiofuran-3-yl |
| 440 | F | Tetrahydrothiopyran-3-yl |
| 441 | F | Thiepan-3-yl- |
| 442 | F | Tetrahydropyran-4-yl |
| 443 | F | Oxepan-4-yl |
| 444 | F | Tetrahydrothiopyran-4-yl |
| 445 | F | Thiepan-4-yl |
| 446 | F | 2-Oxocyclopropyl |
| 447 | F | 2-Oxocyclobutyl |
| 448 | F | 2-Oxocyclopentyl |
| 449 | F | 2-Oxocyclohexyl |
| 450 | F | 2-Oxocycloheptyl |
| 451 | F | 2-Thioxo-cyclopropyl |
| 452 | F | 2-Thioxo-cyclobutyl |
| 453 | F | 2-Oxo-oxetan-3-yl |
| 454 | F | 2-Oxo-tetrahydrofuran-3-yl |
| 455 | F | 2-Oxo-tetrahydropyran-3-yl |
| 456 | F | 2-Oxo-oxepan-3-yl |
| 457 | F | 2-Thioxo-thietan-3-yl |
| 458 | F | 2-Thioxo-tetrahydrothien-3-yl |
| 459 | F | 2-Thioxo-tetrahydrothiopyran-3-yl |
| 460 | F | 2-Thioxo-thiepan-3-yl |
| 461 | F | 2-Thioxo-oxetan-3-yl |
| 462 | F | 2-Thioxo-tetrahydrofuran-3-yl |
| 463 | F | 2-Thioxo-tetrahydropyran-3-yl |
| 464 | F | 2-Thioxo-oxepan-3-yl |
| 465 | F | 2-Oxo-thietan-3-yl |
| 466 | F | 2-oxo-tetrahydrothien-3-yl |
| 467 | F | 2-Oxo-tetrahydrothiopyran-3-yl |
| 468 | F | 2-Oxo-thiepan-3-yl |
| 469 | F | -CH₂-CH(Cl)-CO-OCH₃ |
| 470 | F | -CH₂-CH(Cl)-CO-OC₂H₅ |
| 471 | F | -CH₂-CH(Cl)-CO-O(n-C₃H₇) |
| 472 | F | -CH₂-CH(Cl)-CO-O(n-C₄H₉) |
| 473 | F | -CH₂-CH(Cl)-CO-OCH(CH₃)₂ |
| 474 | F | -CH₂-CH(Cl)-CO-OCH₂-CH(CH₃)₂ |
| 475 | F | -CH₂-CH(Cl)-CO-OCH(CH₃)-C₂H₅ |
| 476 | F | -CH₂-CH(Cl)-CO-OC(CH₃)₃ |
| 477 | F | -CH₂-CH(Br)-CO-OH |
| 478 | F | -CH₂-CH(Br)-CO-OCH₃ |
| 479 | F | -CH₂-CH(Br)-CO-OC₂H₅ |
| 480 | F | -CH₂-CH(Cl)-CO-OH |
| 481 | F | -CH₂-CH(Br)-CO-O(n-C₃H₇) |
| 482 | F | -CH₂-CH(Br)-CO-O(n-C₄H₉) |
| 483 | F | -CH₂-CH(Br)-CO-OCH(CH₃)₂ |
| 484 | F | -CH₂-CH(Br)-CO-OCH₂-CH(CH₃)₂ |
| 485 | F | -CH₂-CH(Br)-CO-OCH(CH₃)-C₂H₅ |
| 486 | F | -CH₂-CH(Br)-CO-OC(CH₃)₃ |
| 487 | F | -CH=CH-CO-OH |
| 488 | F | -CH=CH-CO-OCH₃ |
| 489 | F | -CH=CH-CO-OC₂H₅ |
| 490 | F | -CH=CH-CO-O(n-C₃H₇) |
| 491 | F | -CH=CH-CO-O(n-C₄H₉) |
| 492 | F | -CH=CH-CO-OCH(CH₃)₂ |
| 493 | F | -CH=CH-CO-OCH₂-CH(CH₃)₂ |
| 494 | F | -CH=CH-CO-OCH(CH₃)-C₂H₅ |
| 495 | F | -CH=CH-CO-OC(CH₃)₃ |
| 496 | F | -CH=C(Cl)-CO-OH |
| 497 | F | -CH=C(Cl)-CO-OCH₃ |
| 498 | F | -CH=C(Cl)-CO-OC₂H₅ |
| 499 | F | -CH=C(Cl)-CO-O(n-C₃H₇) |
| 500 | F | -CH=C(Cl)-CO-O(n-C₄H₉) |
| 501 | F | -CH=C(Cl)-CO-OCH(CH₃)₂ |
| 502 | F | -CH=C(Cl)-CO-OCH₂-CH(CH₃)₂ |
| 503 | F | -CH=C(Cl)-CO-OCH(CH₃)-C₂H₅ |
| 504 | F | -CH=C(Cl)-CO-OC(CH₃)₃ |
| 505 | F | -CH=C(Br)-CO-OH |
| 506 | F | -CH=C(Br)-CO-OCH₃ |
| 507 | F | -CH=C(Br)-CO-OC₂H₅ |
| 508 | F | -CH=C(Br)-CO-O(n-C₃H₇) |
| 509 | F | -CH=C(Br)-CO-O(n-C₄H₉) |
| 510 | F | -CH=C(Br)-CO-OCH(CH₃)₂ |
| 511 | F | -CH=C(Br)-CO-OCH₂-CH(CH₃)₂ |
| 512 | F | -CH=C(Br)-CO-OCH(CH₃)-C₂H₅ |
| 513 | F | -CH=C(Br)-CO-OC(CH₃)₃ |
| 514 | F | -CH₂-CH(Cl)-CO-NH₂ |
| 515 | F | -CH₂-CH(Cl)-CO-NH-CH₃ |
| 516 | F | -CH₂-CH(Cl)-CO-N(CH₃)₂ |
| 517 | F | -CH₂-CH(Cl)-CO-NH-C₂H₅ |
| 518 | F | -CH₂-CH(Cl)-CO-N(C₂H₅)₂ |
| 519 | F | -CH₂-CH(Cl)-CO-NH-(n-C₃H₇) |
| 520 | F | -CH₂-CH(Cl)-CO-N(n-C₃H₇)₂ |
| 521 | F | -CH₂-CH(Cl)-CO-NH-(n-C₄H₉) |
| 522 | F | -CH₂-CH(Cl)-CO-N(n-C₄H₉)₂ |
| 523 | F | -CH₂-CH(Br)-CO-NH₂ |
| 524 | F | -CH₂-CH(Br)-CO-NH-CH₃ |
| 525 | F | -CH₂-CH(Br)-CO-N(CH₃)₂ |
| 526 | F | -CH₂-CH(Br)-CO-NH-C₂H₅ |
| 527 | F | -CH₂-CH(Hr)-CO-N(C₂H₅)₂ |
| 528 | F | -CH₂-CH(Br)-CO-NH-(n-C₃H₇) |
| 529 | F | -CH₂-CH(Br)-CO-N(n-C₃H₇)₂ |
| 530 | F | -CH₂-CH(Br)-CO-NH-(n-C₄H₉) |
| 531 | F | -CH₂-CH(Br)-CO-N(n-C₄H₉)₂ |
| 532 | F | -CH=CH-CO-NH₂ |
| 533 | F | -CH=CH-CO-NH-CH₃ |
| 534 | F | -CH=CH-CO-N(CH₃)₂ |
| 535 | F | -CH=CH-CO-NH-C₂H₅ |
| 536 | F | -CH=CH-CO-N(C₂H₅)₂ |
| 537 | F | -CH=CH-CO-NH-(n-C₃H₇) |
| 538 | F | -CH=CH-CO-N(n-C₃H₇)₂ |
| 539 | F | -CH=CH-CO-NH-(n-C₄H₉) |
| 540 | F | -CH=CH-CO-N(n-C₄H₉)₂ |
| 541 | F | -CH=C(Cl)-CO-NH₂ |
| 542 | F | -CH=C(Cl)-CO-NH-CH₃ |
| 543 | F | -CH=C(Cl)-CO-N(CH₃)₂ |
| 544 | F | -CH=C(Cl)-CO-NH-C₂H₅ |
| 545 | F | -CH=C(Cl)-CO-N(C₂H₅)₂ |
| 546 | F | -CH=C(Cl)-CO-NH-(n-C₃H₇) |
| 547 | F | -CH=C(Cl)-CO-N(n-C₃H₇)₂ |
| 548 | F | -CH=C(Cl)-CO-NH-(n-C₄H₉) |
| 549 | F | -CH=C(Cl)-CO-N(n-C₄H₉)₂ |
| 550 | F | -CH=C(Br)-CO-NH₂ |
| 551 | F | -CH=C(Br)-CO-N_{H}-CH₃ |
| 552 | F | -CH=C(Br)-CO-NH(CH₃)₂ |
| 553 | F | -CH=C(Br)-CO-NH-C₂H₅ |
| 554 | F | -CH=C(Br)-CO-N(C₂H₅)₂ |
| 555 | F | -CH=C(Br)-CO-NH-(n-C₃H₇) |
| 556 | F | -CH=C(Br)-CO-N(n-C₃H₇)₂ |
| 557 | F | -CH=C(Br)-CO-NH-(n-C₄H₉) |
| 558 | F | -CH=C(Br)-CO-N(n-C₄H₉)₂ |

Bevorzugt sind weiterhin Verbindungen der allgemeinen Formel I-A, worin Z eine Einfachbindung bedeutet, R³ für eine Gruppe CH₂-R^{3'} steht, worin R^{3'} und R¹ die in Tabelle 1 angegebenen Bedeutungen aufweist (Verbindungen I-Ab.1 bis I-Ab.558).

Bevorzugt sind weiterhin Verbindungen der allgemeinen Formel I-A, worin Z ein Sauerstoffatom bedeutet und R¹ und R³ die in Tabelle 1 angegebenen Bedeutungen aufweisen (Verbindungen I-Ac.1 bis I-Ac.558).

Bevorzugt sind weiterhin Verbindungen der allgemeinen Formel I-A, worin Z ein Schwefelatom bedeutet und R¹ und R³ die in Tabelle 1 angegebenen Bedeutungen aufweisen (Verbindungen I-Ad.1 bis I-Ad.558).

Bevorzugt sind ausserdem Verbindungen der allgemeinen Formel I-B, worin Z eine Einfachbindung bedeutet, Het für einen Rest der allgemeinen Formel II-2 mit X = Sauerstoff, R⁴ = Trifluormethyl und R⁵ = Methyl steht, R² Chlor bedeutet und R¹ und R³ die in Tabelle 1 angegebenen Bedeutungen aufweisen (Verbindungen I-Ba.1 bis I-Ba.558).

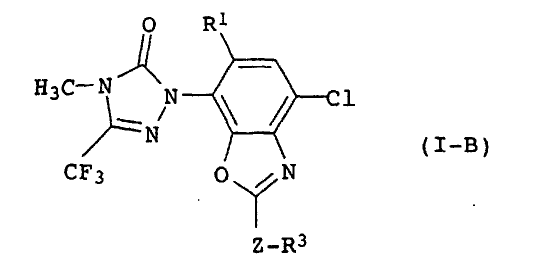

Bevorzugt sind weiterhin Verbindungen der allgemeinen Formel I-B, worin Z eine Einfachbindung bedeutet, R³ für eine Gruppe CH₂-R^{3'} steht, worin R^{3'} und R¹ die in Tabelle 1 angegebenen Bedeutungen aufweisen (Verbindungen I-Bb.1 bis I-Bb.558).

Bevorzugt sind weiterhin Verbindungen der allgemeinen Formel I-B, worin Z ein Sauerstoffatom bedeutet und R¹ und R³ die in Tabelle 1 angegebenen Bedeutungen aufweisen (Verbindungen I-Bc.1 bis I-Bc.558).

Bevorzugt sind weiterhin Verbindungen der allgemeinen Formel I-B, worin Z ein Schwefelatom bedeutet und R¹ und R³ die in Tabelle 1 angegebenen Bedeutungen aufweisen (Verbindungen I-Bd.1 bis I-Bd.558).

Bevorzugt sind ausserdem Verbindungen der allgemeinen Formel I-C, worin Z eine Einfachbindung bedeutet, Het für einen Rest der allgemeinen Formel II-5 mit X = X' = Sauerstoff, worin R⁴ und R4' gemeinsam mit den Kohlenstoffatomen, an die sie gebunden sind, einen Cyclohexenring bilden, R² Chlor bedeutet und R¹ und R³ die in Tabelle 1 angegebenen Bedeutungen aufweisen (Verbindungen I-Ca.1 bis I-Ca.558).

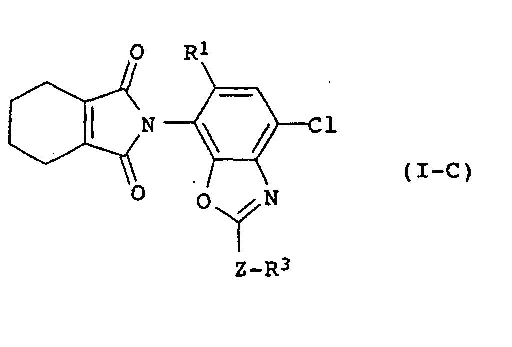

Bevorzugt sind weiterhin Verbindungen der allgemeinen Formel I-C, worin Z eine Einfachbindung bedeutet, R³ für eine Gruppe CH₂-R^{3'} steht, worin R^{3'} und R¹ die in Tabelle 1 angegebenen Bedeutungen aufweisen (Verbindungen I-Cb.1 bis I-Cb.558).

Bevorzugt sind weiterhin Verbindungen der allgemeinen Formel I-C, worin Z ein Sauerstoffatom bedeutet und R¹ und R³ die in Tabelle 1 angegebenen Bedeutungen aufweisen (Verbindungen I-Cc.1 bis I-Cc.558).

Bevorzugt sind weiterhin Verbindungen der allgemeinen Formel I-C, worin Z ein Schwefelatom bedeutet und R¹ und R³ die in Tabelle 1 angegebenen Bedeutungen aufweisen (Verbindungen I-Cd.1 bis I-Cd.558).

Bevorzugt sind ausserdem Verbindungen der allgemeinen Formel I-C', worin Z eine Einfachbindung bedeutet, Het für einen Rest der allgemeinen Formel II-5 mit X = X' = Schwefel, worin R⁴ und R4' gemeinsam mit den Kohlenstoffatomen, an die sie gebunden sind, einen Cyclohexenring bilden, R² Chlor bedeutet und R¹ und R³ die in Tabelle 1 angegebenen Bedeutungen aufweisen (Verbindungen I-C'a.1 bis I-C'a.558).

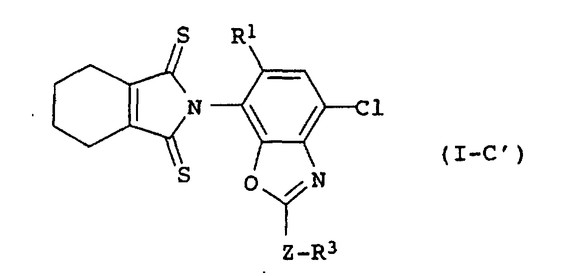

Bevorzugt sind weiterhin Verbindungen der allgemeinen Formel I-C', worin Z eine Einfachbindung bedeutet, R³ für eine Gruppe CH₂-R^{3'} steht, worin R^{3'} und R¹ die in Tabelle 1 angegebenen Bedeutungen aufweisen (verbindungen I-C'b.1 bis I-C'b.558).

Bevorzugt sind weiterhin Verbindungen der allgemeinen Formel I-C', worin Z ein Sauerstoffatom bedeutet und R¹ und R³ die in Tabelle 1 angegebenen Bedeutungen aufweisen (Verbindungen I-C'c.1 bis I-C'c.558).

Bevorzugt sind weiterhin Verbindungen der allgemeinen Formel I-C', worin Z ein Schwefelatom bedeutet und R¹ und R³ die in Tabelle 1 angegebenen Bedeutungen aufweisen (Verbindungen I-C'd.1 bis I-C'd.558).

Bevorzugt sind ausserdem Verbindungen der allgemeinen Formel I-D, worin Z eine Einfachbindung bedeutet, Het für einen Rest der allgemeinen Formel II-6 mit X = X' = Y = Sauerstoff, R⁶ und R⁷ = Methyl steht, R² Chlor bedeutet und R¹ und R³ die in Tabelle 1 angegebenen Bedeutungen aufweisen (Verbindungen I-Da.1 bis I-Da.558).

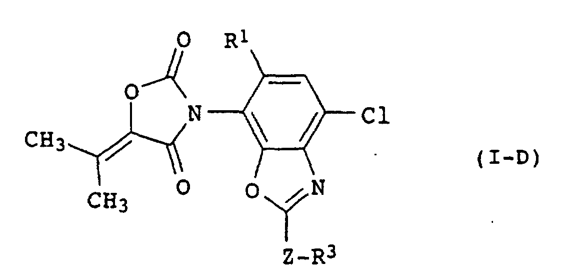

Bevorzugt sind weiterhin Verbindungen der allgemeinen Formel I-D, worin Z eine Einfachbindung bedeutet, R³ für eine Gruppe CH₂-R^{3'} steht, worin R^{3'} und R¹ die in Tabelle 1 angegebenen Bedeutungen aufweisen (Verbindungen I-Db.1 bis I-Db.558).

Bevorzugt sind weiterhin Verbindungen der allgemeinen Formel I-D, worin Z ein Sauerstoffatom bedeutet und R¹ und R³ die in Tabelle 1 angegebenen Bedeutungen aufweisen (Verbindungen I-Dc.1 bis I-Dc.558).

Bevorzugt sind weiterhin Verbindungen der allgemeinen Formel I-D, worin Z ein Schwefelatom bedeutet und R¹ und R³ die in Tabelle 1 angegebenen Bedeutungen aufweisen (Verbindungen I-Dd.1 bis I-Dd.558).

Bevorzugt sind ausserdem Verbindungen der allgemeinen Formel I-E, worin Z eine Einfachbindung bedeutet, Het für einen Rest der allgemeinen Formel II-12 mit X = Sauerstoff, R⁴ = Methyl, R^{4'} = Trifluormethyl und R^{4''} = Wasserstoff steht, R² Chlor bedeutet und R¹ und R³ die in Tabelle 1 angegebenen Bedeutungen aufweisen (Verbindungen I-Ea.1 bis I-Ea.558).

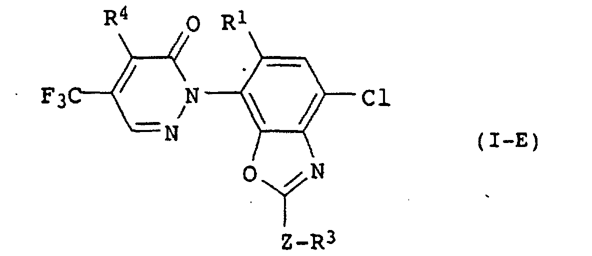

Bevorzugt sind weiterhin Verbindungen der allgemeinen Formel I-E, worin Z eine Einfachbindung bedeutet, R³ für eine Gruppe CH₂-R^{3'} steht, worin R^{3'} und R¹ die in Tabelle 1 angegebenen Bedeutungen aufweisen (Verbindungen I-Eb.1 bis I-Eb.558).

Bevorzugt sind weiterhin Verbindungen der allgemeinen Formel I-E, worin Z ein Sauerstoffatom bedeutet und R¹ und R³ die in Tabelle 1 angegebenen Bedeutungen aufweisen (Verbindungen I-Ec.1 bis I-Ec.558).

Bevorzugt sind weiterhin Verbindungen der allgemeinen Formel I-E, worin Z ein Schwefelatom bedeutet und R¹ und R³ die in Tabelle 1 angegebenen Bedeutungen aufweisen (Verbindungen I-Ed.1 bis I-Ed.558).

Bevorzugt sind ausserdem Verbindungen der allgemeinen Formel I-E', worin Z eine Einfachbindung bedeutet, Het für einen Rest der allgemeinen Formel II-12 mit X = Sauerstoff, R⁴ = Amino, R^{4'} = Methylsulfonyl und R⁴" = Wasserstoff steht, R² Chlor bedeutet und R¹ und R³ die in Tabelle 1 angegebenen Bedeutungen aufweisen (Verbindungen I-E'a.1 bis I-E'a.558).

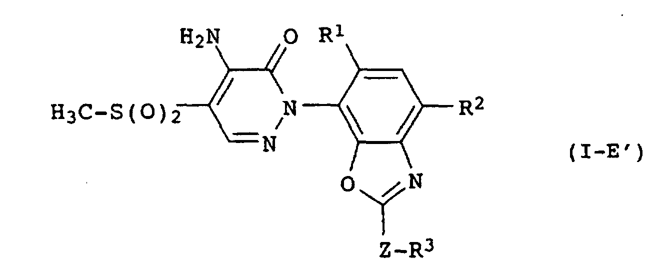

Bevorzugt sind weiterhin verbindungen der allgemeinen Formel I-E', worin Z eine Einfachbindung bedeutet, R³ für eine Gruppe CH₂-R^{3'} steht, worin R^{3'} und R¹ die in Tabelle 1 angegebenen Bedeutungen aufweisen (Verbindungen I-E'b.1 bis I-E'b.558).

Bevorzugt sind weiterhin Verbindungen der allgemeinen Formel I-E', worin Z ein Sauerstoffatom bedeutet und R¹ und R³ die in Tabelle 1 angegebenen Bedeutungen aufweisen (Verbindungen I-E'c.1 bis I-E'c.558).

Bevorzugt sind weiterhin Verbindungen der allgemeinen Formel I-E', worin Z ein Schwefelatom bedeutet und R¹ und R³ die in Tabelle 1 angegebenen Bedeutungen aufweisen (Verbindungen I-E'd.1 bis I-E'd.558).

Bevorzugt sind ausserdem Verbindungen der allgemeinen Formel I-E", bei denen es sich um Verbindungen der Formel I handelt, worin Z eine Einfachbindung bedeutet, Het für einen Rest der allgemeinen Formel II-12 mit X = Sauerstoff, R⁴ = Wasserstoff, R⁴' = Trifluormethyl und R⁴" = Wasserstoff steht, R² Chlor bedeutet und R¹ und R³ die in Tabelle 1 angegebenen Bedeutungen aufweisen (Verbindungen I-E"a.1 bis I-E"a.558).

Bevorzugt sind weiterhin Verbindungen der allgemeinen Formel I-E", worin Z eine Einfachbindung bedeutet, R³ für eine Gruppe CH₂-R^{3'} steht, worin R^{3'} und R¹ die in Tabelle 1 angegebenen Bedeutungen aufweisen (Verbindungen I-E"b.1 bis I-E"b.558).

Bevorzugt sind weiterhin Verbindungen der allgemeinen Formel I-E", worin Z ein Sauerstoffatom bedeutet und R¹ und R³ die in Tabelle 1 angegebenen Bedeutungen aufweisen (Verbindungen I-Ec".1 bis I-E"c.558).

Bevorzugt sind weiterhin Verbindungen der allgemeinen Formel I-E", worin Z ein Schwefelatom bedeutet und R¹ und R³ die in Tabelle 1 angegebenen Bedeutungen aufweisen (Verbindungen I-E"d.1 bis I-E"d.558).

Die erfindungsgemäßen Benzoxazole der Formel I sind auf verschiedene Weise erhältlich, beispielsweise durch Kondensation eines substituierten 2-Aminophenols der allgemeinen Formel III mit Kohlensäure- oder Carbonsäurederivaten: 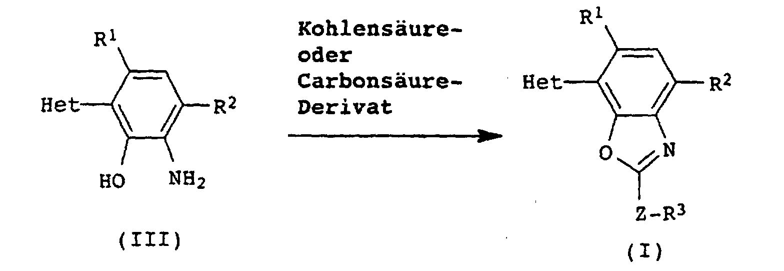 Die Kondensationsreaktion von bifunktionellen 2-Aminophenolen mit Kohlensäure- oder Carbonsäurederivaten können nach an sich bekannte weise durchgeführt werden (vgl. z.B. Houben-Weyl, Methoden der organischen Chemie, Georg Thieme Verlag Stuttgart, Bd. E8c, 1. Auflage 1994, S. 247-284; Bd. E8b, 1. Auflage 1994, S. 881-901; Bd. E8a, 1. Auflage 1993, S. 1032-1078). Bevorzugte Kohlensäure- oder Carbonsäurederivate sind die entsprechenden Ester, Anhydride, Säurechloride, Orthoester, Diimide, Nitrile, Imidoester, Trichlormethyl-substituierte Verbindungen, Isocyanate und deren Thioanaloga.
Als Lösungs-/Verdünnungsmittel kommen insbesondere organische Lösungsmittel in Betracht, beispielsweise aromatische Kohlenwasserstoffe wie Benzol, Toluol und o-, m-, p-Xylol, halogenierte Kohlenwasserstoffe wie Methylenchlorid, Chloroform und Dichlorethan, niedere Alkohole wie Methanol und Ethanol, aliphatische oder cyclische Ether wie Dimethoxyethan, Tetrahydrofuran und Dioxan, Carbonsäureester wie Essigsäureethylester oder aprotisch polare Solventien wie Dimethylformamid und Dimethylsulfoxid.

Die Reaktion kann gewünschtenfalls durch Zusatz katalytischer Mengen einer Säure beschleunigt werden. Als Säuren eignen sich insbesondere Mineralsäuren wie Salzsäuren oder Sulfonsäuren wie p-Toluolsulfonsäure bzw. deren Salze mit Stickstoffbasen wie z.B. Pyridin. Die Mengen an Säure liegen bevorzugt bei 0,01 bis 5 Molprozenten, bezogen auf die Menge an III.

Die Reaktionstemperaturen liegen bevorzugt bei 20°C bis Rückflußtemperatur des jeweiligen Reaktionsgemisches, insbesondere bei 60°C bis Rückflußtemperatur.

Das Kohlensäure- oder Carbonsäurederivat wird entweder in ca. stöchiometrischer Menge oder im Überschuß angewandt. In geeigneten Fällen kann auch ein sehr großer Überschuß eingesetzt oder ohne Lösungsmittel gearbeitet werden. Bevorzugt sind etwa stöchiometische Mengen oder ein Überschuß von bis zu 10 Moläquivalenten, bezogen auf die Menge an III.

Die substituierten 2-Aminophenole III erhält man zweckmäßigerweise durch Reduktion entsprechender 2-Nitrophenole IV (vgl. z.B. Houben-Weyl, Methoden der organischen Chemie, Georg Thieme Verlag Stuttgart, Bd. XI/1, 4. Auflage 1957, S. 431ff.):

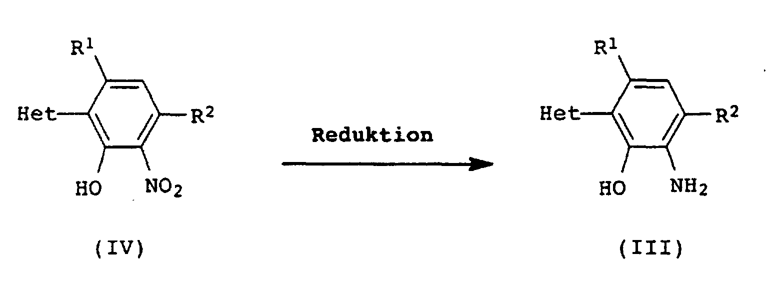

Als Reduktionsmittel kommen insbesondere
- elementare Metalle wie Eisen, Zinn und Zink,
- Wasserstoff in Gegenwart von geeigneten Katalysatoren wie Palladium oder Platin auf Kohle oder Raney-Nickel, oder
- komplexe Hydride wie LiAlH₄ und NaBH₄, ggf. in Gegenwart von Katalysatoren,
in Betracht.

Als Lösungsmittel eignen sich üblicherweise - je nach Reduktionsmittel - Carbonsäuren wie Essigsäure und Propionsäure, Mineralsäuren wie Salzsäure oder Schwefelsäure, Alkohole wie Methanol und Ethanol, Ether wie Diethylether, Methyl-tert.-butylether, Tetrahydrofuran und Dioxan, Aromaten wie Benzol und Toluol, sowie Gemische derartiger Solventien.

Die Umsetzungen können bei Temperaturen von (-100)°C bis zur Siedetemperatur des jeweiligen Reaktionsgemisches vorgenommen werden.

Üblicherweise werden die Ausgangsverbindungen in etwa stöchiometrischen Mengen eingesetzt; in Einzelfällen kann jedoch auch ein vielfacher Überschuß der einen oder anderen Komponente vorteilhaft sein.

Die 2-Nitrophenole der Formel IV können aus geschützten Vorläufern freigesetzt werden, die am Phenol-Sauerstoff einen abspaltbaren Rest R aufweisen. Geeignete Reste R sind solche Gruppen, die die Phenolgruppe als Ether, Ester oder Kohlensäurederivat schützen, z.B. Alkyl-, O-Heterocyclyl, Benzyl-, Alkylcarbonyl-, Arylcarbonyl- oder Alkoxycarbonyl-Gruppen. Geeignete Reste R und Verfahren zu ihrer Abspaltung sind dem Fachmann bekannt, beispielsweise aus Greene/Wuts: Protective Groups in Organic Synthesis, John Wiley & Sons, 2. Auflage 1991, S. 145 ff. und S. 279 ff.

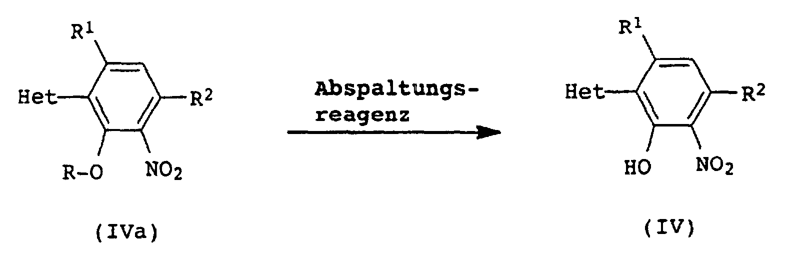

Als Abspaltungsreagenzien kommen insbesondere in Betracht:
- bei gegebenenfalls substituierten Alkylphenolen: Trimethylsilyliodid, Bortribromid, Bortrichlorid, Aluminiumtrichlorid, Lithiumchlorid oder Bromwasserstoff;
- bei gegebenenfalls substituierten Benzylphenolen: Bortrifluorid, Fluorwasserstoffsäure oder Wasserstoff/Katalysator, dabei bevorzugt Edelmetall-Katalysatoren wie Palladium oder Platin;
- bei gegebenenfalls substituierten Arylester: Natriumhydrogencarbonat, Natriumcarbonat, Kaliumcarbonat, Natriumhydroxid, Kaliumhydroxid, Natriummethylat, Chlorwasserstoff, Schwefelsäure, Ammoniak, Hydrazin oder Zink.

Das Lösungs-/Verdünnungsmittel ist bevorzugt so zu wählen, daß es gegenüber dem jeweiligen Abspaltungsreagenz inert ist. Bei Verwendung der Halogenide Trimethylsilyliodid, Bortribromid, Bortrichlorid oder Aluminiumtrichlorid sind halogenierte Lösungsmittel wie Dichlormethan, Chloroform, Tetrachlorkohlenstoff und Dichlorethan besonders bevorzugt. Bromwasserstoff wird bevorzugt in wäßriger Lösung angewandt, ganz besonders bevorzugt als eine 48 gew.%ige Lösung; Lithiumchlorid wird bevorzugt in polaren Lösungsmitteln wie niederen Alkoholen, Dimethylsulfoxid und Dimethylformamid angewendet; hydrogenolytische Methoden werden bevorzugt in niederen Alkoholen oder Carbonsäuren durchgeführt, gegebenenfalls unter Zusatz eines Wasserstoffüberträgers wie Cyclohexen und Cyclohexadien. Mineralbasen bzw. Mineralsäuren und Stickstoffbasen werden bevorzugt im wässrigen Medium angewendet. Je nach Löslichkeit der Reagenzien wird gegebenenfalls ein organisches Lösungsmittel wie z.B. ein niederer Alkohol zugesetzt.

Die Temperatur für die Abspaltungsreaktion liegt bevorzugt bei 0°C bis Siedetemperatur des jeweiligen Reaktionsgemisches.

Das Abspaltungsreagenz wird bevorzugt in etwa stöchiometrischen Mengen oder im Überschuß angewendet. Der Überschuß liegt besonders bevorzugt zwischen einem und zehn Moläquivalenten, bezogen auf die Menge an IVa.

Sowohl die Verbindungen der Formel IV als auch die Verbindungen der Formel IVa können durch nucleophilen Austausch des Fluoratoms in 2-Nitrofluorbenzolen der Formel V gegen ein Sauerstoffnucleophil R'O^{⊖} hergestellt werden. Hierin steht R' für Wasserstoff oder einen der oben definierten abspaltbaren Reste R. Ob der Weg über ein geschütztes Derivat IVa gewählt wird oder das 2-Nitrophenol direkt aus dem 2-Nitrofluorbenzol durch Austausch von Fluor gegen Hydroxid hergestellt werden kann, richtet sich dabei nach der Stabilität der Reste R¹, R² und Het in den Verbindungen V.

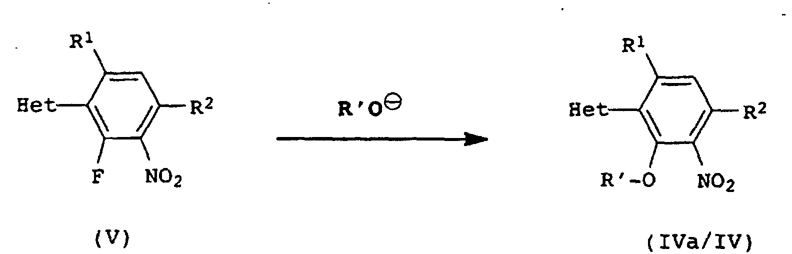

Die Austauschreaktion von aromatisch gebundenem Fluor durch Sauerstoffnukleophile R'O^{⊖}, werden auf an sich bekannte Weise durchgeführt (vgl z.B. Houben-Weyl, Methoden der organischen Chemie, Georg Thieme Verlag Stuttgart, 1976, Bd. 6/1c S. 146-202 und Band IX, 4. Auflage 1955, S. 7-18).
Folgende Reagenzien sind bei dieser Reaktion bevorzugte Reaktionspartner:
- zum Austausch F gegen OR: Kaliumbenzoat, Natriumnitrit, Natriumhydroxid, Kaliumhydroxid, Kaliumcarbonat, Benzaldoxim, Natriumacetat, Kaliumacetat, Natriummethylat, Kaliummethylat, Natriumtrimethylsilanolat, Äpfelsäuredimethylester, N-Hydroxysuccinimid.
- zum Austausch F gegen OH: Natriumhydroxid, Kaliumhydroxid, Lithiumhydroxid.

Die Reaktionen können in bestimmten Fällen ohne Lösungsmittel/Verdünnungsmittel durchgeführt werden. Bei Verwendung eines Lösungsmittels/ Verdünnungsmittels sind solche zu bevorzugen, in denen das Sauerstoff-Gruppen-übertragende Reagenz eine gute Löslichkeit besitzt. Besonders bevorzugt sind Alkohole wie Ethanol, Propanol oder tert.-Butanol, aprotisch polare Lösungsmittel wie Dimethylformamid oder Dimethylacetamid, cyclische Ether wie Dioxan oder Tetrahydrofuran oder aliphatische Ether wie Dimethoxyethan.

Die Reaktionstemperaturen liegen bevorzugt bei 20°C bis Rückflußtemperatur des jeweiligen Reaktionsgemisches, insbesondere bei 60°C bis Rückflußtemperatur.

Das Sauerstoff-übertragende Reagenz wird entweder in ca. stöchiometrischer Menge oder im Überschuß angewandt. In geeigneten Fällen kann auch ein sehr großer Überschuß eingesetzt werden. Bevorzugt sind etwa stöchiometische Mengen oder ein Überschuß von bis zu 10 Moläquivalenten, bezogen auf die Menge an V.

Die neuen 2-Nitrofluorbenzole V sind auf an sich bekannte Weise durch Fluorbenzole VI mit Nitrierungsreagenzien, d.h. NO₂⁺-Überträgern, erhältlich (vgl. z.B. Houben-Weyl, Methoden der organischen Chemie, Georg Thieme Verlag Stuttgart, Bd. 10/1, 1971, S. 479ff.):

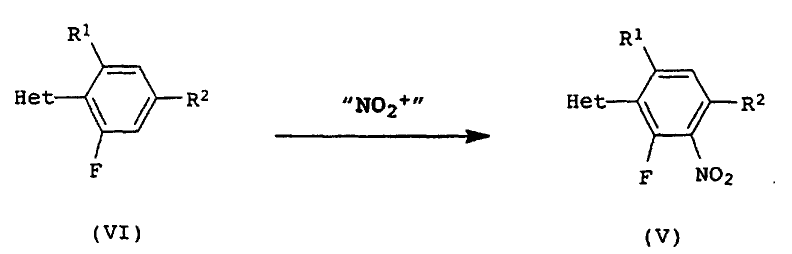

Als Nitrierungsreagenzien kommen insbesondere Salpetersäure, im Gemisch mit Schwefelsäure oder Essigsäureanhydrid, oder Nitroniumsalze, speziell Nitroniumtetrafluoroborat, in Betracht. Das Gemisch, bestehend aus Salpetersäure und Schwefelsäure, kann aus beliebigen Mengenverhältnissen der beiden Mischungspartner bestehen; bevorzugt sind solche Mischungen, bei denen der Schwefelsäure-Anteil stark überwiegt oder als Lösungsmittel dient. Für die Mischung aus Salpetersäure und Essigsäureanhydrid gilt Analoges.

Nitroniumtetrafluoroborat wird bevorzugt in aprotisch, polaren Lösungsmitteln angewendet, z.B. in Acetonitril oder Nitromethan.

Die Reaktionstemperatur liegt allgemein bei (-80) bis 80°C, insbesondere (-20)°C bis 30°C.

Bei den Nitrierungen mit dem Reagenz Salpetersäure wird bevorzugt mit einer etwa äquimolaren Menge oder besonders bevorzugt mit einem Überschuß an Nitrierungsreagenz gearbeitet. Der Überschuß kann ein Vielfaches der Menge an VI betragen. Nitroniumtetrafluoroborat wird bevorzugt äquimolar zum Substrat oder im kleinen Überschuß zwischen 1.1 und 1.5 Moläquivalenten eingesetzt.

Die substituierten Fluorbenzole der allgemeinen Formel VI können ausgehend von bekannten 2-Aminofluorbenzolen VII oder 2-Fluorphenylhydrazinen der Formel VIIa nach üblichen Synthesen für Heterocyclen oder Carbocyclen hergestellt werden hergestellt werden (Schema 1).

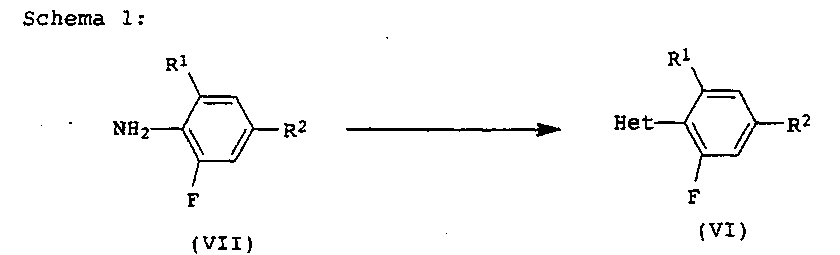

Die Verbindungen der allgemeinen Formel VI und VII sind zum Teil auch aus dem Stand der Technik bekannt, beispielsweise aus JP 0 9227535, US 4,818,272, US 4,919,708 und J. Heterocycl. Chem. 1987, 24, S.1391, oder können nach den dort beschriebenen Methoden hergestellt werden. Auf diese Schriften wird, soweit sie die Verbindungen der Formel VI und ihre Herstellung betreffen, Bezug genommen.

Selbstverständlich kann die oben aufgezeigte Synthesesequenz VII - VI - V - IV -III auch mit den entsprechenden Aminoverbindungen IIIa, IVb, IVc und Va durchgeführt werden (Schema 2).

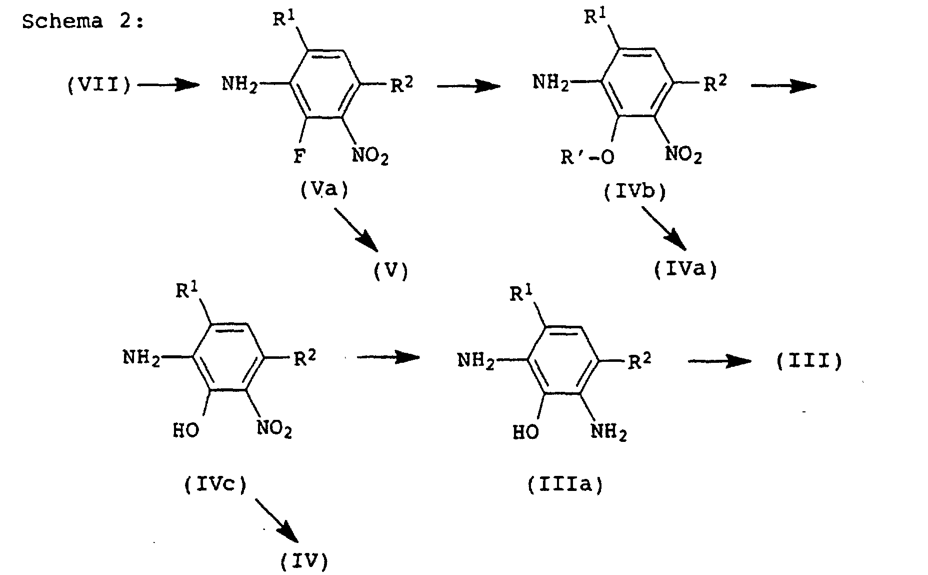

Die Aminogruppe muß gegebenenfalls auf einer oder mehreren Stufen geschützt werden. Auf jeder der Stufen dieser Synthesesequenz kann die Umwandlung der Aminogruppe in eine der Reste der allgemeinen Formel II-1 bis II-18 vorgenommen werden.

Ein Aufbau des Restes Het in den Verbindungen I ist auch ausgehend von 7-Aminobenzoxazolen der Formel VIII möglich (Schema 3). Die Verbindungen der allgemeinen Formel VIII sind beispielsweise aus der WO 97/08170 bekannt.

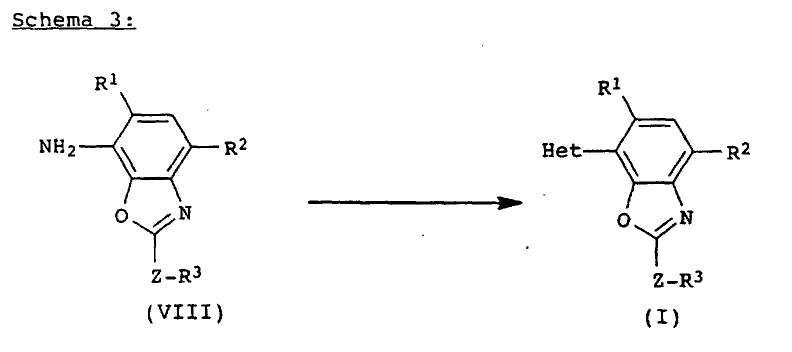

Ein weiterer Zugang zu den erfindungsgemässen Verbindungen der allgemeinen Formel I ist in Schema 4 dargestellt:

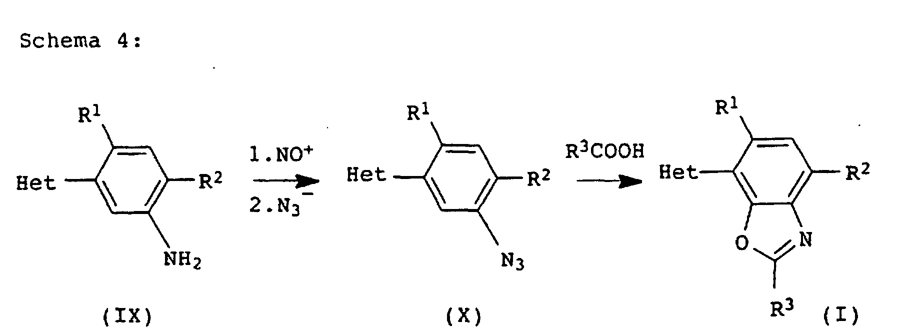

In Schema 4 haben Het, R¹, R² und R³ die zuvor genannten Bedeutungen. X steht hier für eine Einfachbindung. Gemäss Schema 4 werden zunächst die aus dem Stand der Technik, z.B. aus der WO 97/07104 bekannten Aniline der Formel IX durch sukzessive Nitrosierung zur Diazoniumverbindung und nachfolgende Umsetzung mit einem Azid in das Phenylazid der allgemeinen Formel X überführt, das anschliessend durch Umsetzung von X mit einer Carbonsäure der Formel R³COOH in das Oxazol der Formel I, worin X eine Einfachbindung bedeutet, umgewandelt wird. Die Schema 4 gezeigte Synthese erfolgt analog zu der in Houben-Weyl, Methoden der Organischen Chemie, Band E8a, 1993, S. 1087 ff. beschriebenen Umwandlung von Anilinen in Benzoxazole. Die Azide der allgemeinen Formel I sind neu und ebenfalls Gegenstand der vorliegenden Erfindung.

Die Herstellung des Diazoniumsalzes von IX erfolgt auf an sich bekannte Weise durch Umsetzung des Anilins IX mit einem Nitrit wie Natriumnitrit und Kaliumnitrit in einer wäßrigen Säurelösung, z.B. in Salzsäure, Bromwasserstoffsäure oder Schwefelsäure, oder durch Umsetzung von X mit einem Salpetrigsäureester wie tert.-Butylnitrit und Isopentylnitrit unter wasserfreien Reaktionsbedingungen, z.B. in Chlorwasserstoff haltigem Eisessig, in absolutem Alkohol, in Dioxan oder Tetrahydrofuran, in Acetonitril oder in Aceton. Die Überführung der Diazoniumverbindungen in die Arylazide erfolgt vorzugsweise durch Umsetzung der Diazoniumsalze mit einem Alkali- oder Erdalkalimetallazid wie Natriumazid oder durch Umsetzung mit Trimethylsilylazid (siehe auch Organic Synthesis, Bd. IV, S. 75 und Bd. V, S. 829).

Bei der Umsetzung der Azid-Verbindungen der Formel X mit der Carbonsäure R³-COOH arbeitet man entweder in einem inerten organischen Solvens, beispielsweise in Kohlenwasserstoffen wie Toluol oder Hexan, in halogenierten Kohlenwasserstoffen wie Dichlormethan oder Chloroform, in Ethern wie Diethylether, Dimethoxyethan, Methyl-t-butylether, Dioxan oder Tetrahydofuran, in Amiden wie Dimethylformamid, Dimethylacetamid oder N-Methylpyrrolidon, in Acetonitril oder vorzugsweise lösungsmittelfrei in einem Überschuß der Carbonsäure R¹⁹COOH. Im letzteren Fall kann der Zusatz einer Mineralsäure wie Phosphorsäure und/oder eines silylierenden Reagenzes wie ein Gemisch aus Phosphorpentoxid und Hexamethyldisiloxan hilfreich sein. Die Umsetzung erfolgt vorzugsweise bei erhöhter Temperatur, beispielsweise bei der Siedetemperatur des Gemisches.

Die im folgenden angegebenen Beispiele sollen die Erfindung erläutern, ohne sie jedoch einzuschränken.

### Beispiel 1:

### 3-(4-Chlor-6-fluorbenzoxazol-7-yl)-5-(1,1-dimethylethyl)-1,3,4-oxadiazol-2(3H)-on (Verb. I-Aa.373 aus Tabelle 1.)

1,0 g 3-(5-Amino-4-chlor-2-fluor-6-hydroxyphenyl)-5-(1,1-dimethylethyl)-1,3,4-oxadiazol-2(3H)-on (aus Vorstufe 7; enthaltend 30% des 3-Aminophenyl-Isomeren) wurden in 30 ml absolutem Ethanol vorgelegt und 1,1 g Trimethylorthoformiat wurden zugegeben. Die Mischung wurde 5,5 Stunden auf Rückfluß erhitzt. Danach wurden die flüchtigen Bestandteile am Wasserstrahlvakuum entfernt und das Produkt wurde durch Chromatographie an Kieselgel (Eluenten Cyclohexan/Ethylacetat 95/5) gereinigt. Ausbeute: 0,3 g.
¹H-NMR (270 MHz, CDCl₃): δ[ppm] = 8,20(s, 1H); 7,40(d, 1H); 1,40(s, 9H).

### Vorstufe 1: 4-Chlor-2,6-difluoranilin

30,0 g 2,6-Difluoranilin wurden mit 200 ml konzentrierter Essigsäure vermischt und auf 80°C erwärmt. 36,0 g Sulfurylchlorid wurden mit etwa der gleichen Menge konzentrierter Essigsäure vermischt und zu der erwärmten Lösung zugetropft. Danach wurde die Lösung für 6 Stunden auf Rückfluß erhitzt. Nach dem Abkühlen der Lösung wurde am Vakuum eingeengt und der Rückstand mit Wasser und Pentan verrührt. Der feste Rückstand wurde abfiltriert und mit Wasser gewaschen. Der so erhaltene Feststoff wurde mit 200 ml konzentrierter Salzsäure versetzt und für 2 Stunden auf Rückfluß erhitzt. Nach dem Abkühlen wurde die Lösung vorsichtig mit Natronlauge schwach alkalisch gestellt und dreimal mit 100 ml Ethylacetat extrahiert. Die organische Phase wurde mit Natriumsulfat getrocknet und vom Lösungsmittel befreit. Ausbeute: 34,0 g.
¹H-NMR (270 MHz, CDCl₃): δ[ppm] = 6,85(d, 2H); 3,9-3,6(br, 2H).

### Vorstufe 2: 4-Chlor-2,6-difluorphenylhydrazin

25,0 g 4-Chlor-2,6-difluoranilin wurden mit 277 ml konzentrierter Salzsäure vermischt. Die Mischung wurde kurz erhitzt bis eine feine Suspension entstanden ist. 10,6 g Natriumnitrit wurden in 60 ml Wasser gelöst und bei 0°C zu der Suspension zugetropft. Die Mischung wurde für zwei Stunden bei 0°C gerührt. In einem zweiten Kolben wurden 86,7 g Zinndichlorid-Dihydrat in 52 ml konzentrierter Salzsäure vorgelegt. Bei 0°C wurde die Diazoniumsalzlösung zugegeben, wobei ein dicker Brei entstand und die Mischung schäumte. Danach rührte man 2 Stunden bei 20°C, goß die Reaktionsmischung auf Wasser, filtrierte von unlöslichen Bestandteilen und stellte die Lösung mittels konzentrierter Natronlauge auf einen pH von 11 ein. Der ausgefallene Feststoff wurde abgesaugt und mehrmals mit Wasser gewaschen. Ausbeute: 16,2 g.
¹H-NMR (270 MHz, CDCl₃): δ[ppm] = 6,90(d; 2H); 5,2-5,0(br, 1H); 4,0-3,8 (br, 2H).

### Vorstufe 3: 1-(4-Chlor-2,6-difluorphenyl)-2-(2,2-dimethylpropionyl)hydrazin

8,0 g 4-Chlor-2,6-difluorphenylhydrazin wurden in 200 ml Toluol gelöst. Hierzu gab man 27 ml 10%ige Natronlauge. Dann tropfte man unter Kühlung auf 0°C 5,66 g Pivalinsäurechlorid zu und rührte die Mischung wurde für 3 Stunden bei 20°C. Die organische Phase wurde abgetrennt und mehrmals mit Wasser gewaschen, getrocknet und vom Lösungsmittel befreit. Ausbeute: 11,4 g.
¹H-NMR (270 MHz, CDCl₃): δ[ppm] = 7,75(br, 1H); 6,90(d, 2H); 6,30(br, 1H); 1,2(s, 9H).

### Vorstufe 4: 3-(4-Chlor-2,6-difluorphenyl)-5-(1,1-dimethylethyl)-1,3,4-oxadiazol-2(3H)-on

11,0 g 1-(4-Chlor-2,6-difluorphenyl)-2-(2,2-dimethylpropionyl)hydrazin wurden in 100 ml absolutem Toluol mit 4,6 g Diphosgen gemischt. Man erhitzte die Mischung für 3 Stunden zum Rückfluß und gab danach nochmals 5,0 g Diphosgen zu. Nach weiteren 5 Stunden Erhitzen auf Rückfluß fügte man weitere 4,0 g Diphosgen und einen Tropfen DMF zu und erhitzte für 3 Stunden zum Rückfluß. Nach dem Abkühlen der Mischung neutralisierte man mit Natriumhydrogencarbonat-Lösung und wusch anschließend die organische Phase mit Wasser. Nach Trocknung und Entfernen der flüchtigen Bestandteile erhielt man als Ausbeute 11,7 g des gewünschten Produkts.
¹H-NMR (270 MHz, CDCl₃) : δ[ppm] = 7,10(d, 2H); 1,35(s, 9H).

### Vorstufe 5: 3-(4-Chlor-2,6-difluor-3-nitrophenyl)-5-(1,1-dimethylethyl)-1,3,4-oxadiazol-2(3H)-on

11,5 g 3-(4-Chlor-2,6-difluorphenyl)-5-(1,1-dimethylethyl)-1,3,4-oxadiazol-2(3H)-on wurden in 15 ml konzentrierter Schwefelsäure gelöst. Bei 0°C gab man 9 ml 65%ige Salpetersäure zu und rührte die Mischung für 10 Minuten. Dann wurde die Mischung in Eiswasser eingerührt und mit 100 ml tert.-Butylmethylether extrahiert. Die organische Phase wurde mit Wasser gewaschen, getrocknet und von den flüchtigen Bestandteilen befreit. Ausbeute: 10,5 g.
¹H-NMR (270 MHz, CDCl₃): δ[ppm] = 7,35(d, 1H); 1,35(s, 9H).

### Vorstufe 6: 3-(4-Chlor-2-fluor-6-hydroxy-5-nitrophenyl)-5-(1,1-dimethyl-ethyl)-1,3,4-oxadiazol-2(3H)-on

8,25 g 3-(4-Chlor-2,6-difluor-3-nitrophenyl)-5-(1,1-dimethylethyl)-1,3,4-oxadiazol-2(3H)-on wurden in 250 ml absolutem Dimethylformamid gelöst. Man gab 8,25 g Natriumacetat zu und erhitzte die Mischung 45 Minuten auf 120°C. Nach dem Abkühlen gab man Wasser zu, stellte durch Zugabe von verdünnter Salzsäure einen schwach saurem pH-Wert ein und extrahierte mit tert.-Butylmethylether. Die organische Phase wurde getrocknet und von flüchtigen Bestandteilen befreit. Ausbeute: 7,8 g , enthaltend 30% das durch Austausch des zur Nitrogruppe para-ständigen Fluors entstandenen 3-Nitro-Isomers.
¹H-NMR (270 MHz, CDCl₃): δ[ppm] = 7,05(d, 1H); 1,40(s, 9H).

### Vorstufe 7: 3-(5-Amino-4-chlor-2-fluor-6-hydroxyphenyl)-5-(1,1-dimethyl-ethyl)-1,3,4-oxadiazol-2(3H)-on

7,8 g des Isomerengemischs aus Vorstufe 6 wurden in einem Gemisch aus 170 ml Wasser und 17 ml konzentrierter Salzsäure aufgenommen. Die Mischung wurde auf 65°C erwärmt. Hierzu gab man portionsweise 6,63 g Eisenpulver. Die Mischung wurde 6 Stunden auf Rückfluß erhitzt. Nach Abkühlen wurde mit Ethylacetat (150 ml) extrahiert; nach Trocknen der organischen Phase und Entfernen der flüchtigen Bestandteile erhielt man das Wertprodukt, enthaltend zu 30% das Reduktionsprodukt des 3-Nitro-Isomers aus Vorstufe 6. Ausbeute: 5,5 g Isomerengemisch.
¹H-NMR (270 MHz, CDCl₃): δ[ppm] = 6,85(d, 1H); 1,40(s, 9H).

### Beispiele 2 bis 6

In der nachfolgenden Tabelle 2 sind neben den vorstehend beschriebenen noch weitere Benzoxazol-Derivate der Formel I aufgeführt, die auf analoge weise hergestellt wurden:

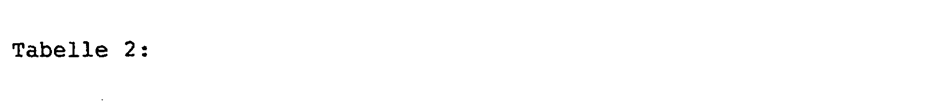

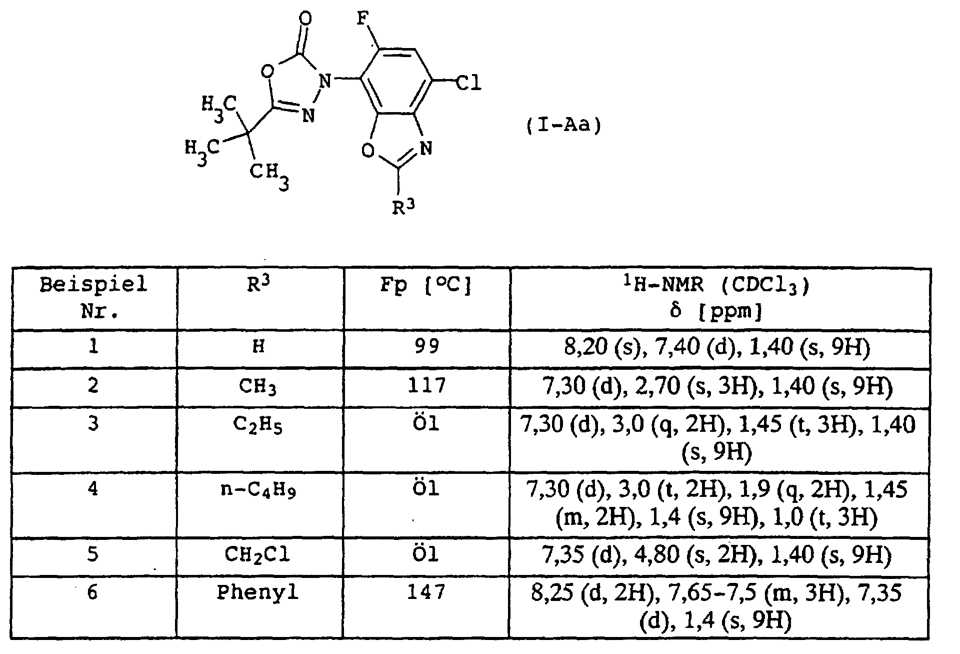

### Beispiele 7 bis 14

In analoger Weise wurde ausgehend von 1-(4-Chlor-2,6-difluorphenyl)-3-trifluormethyl-4-methyltriazol-5-on die in Tabelle 3 angegebenen Verbindungen der Beispiele 7 bis 14 hergestellt. Alle Verbindungen fielen als Öl an.

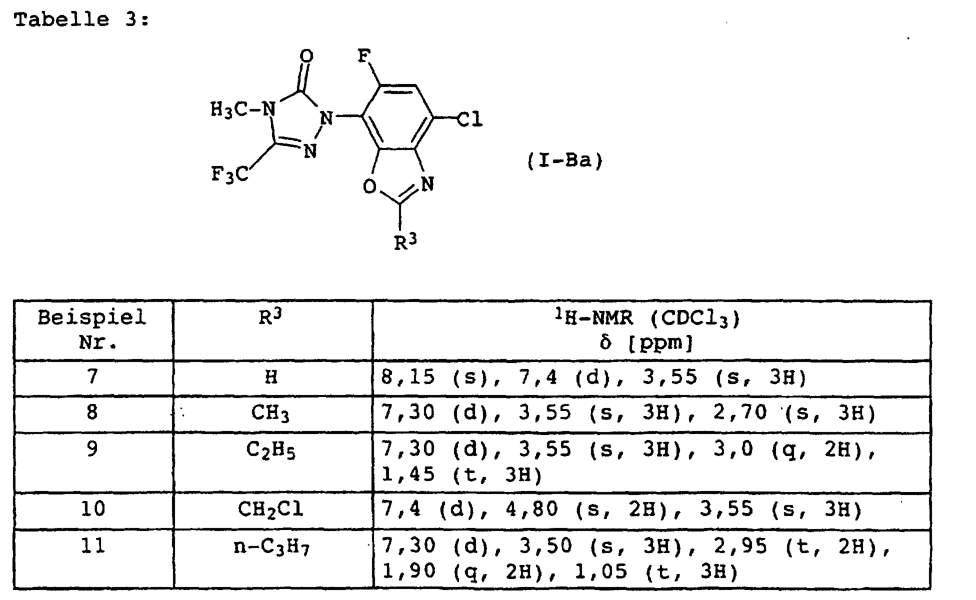

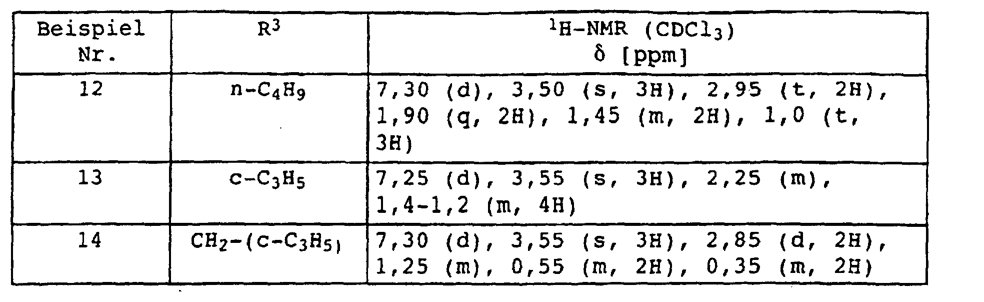

### Beispiel 15:

### N-(4-Chlor-1-cyclopropyl-6-fluorbenzoxazol-7-yl)-4,5,6,7-tetrahydrophthalimid (Verb. I-Ca.419 aus Tabelle 1)

Die Titelverbindung wurde ausgehend von 1-(4-Chlor-2,6-difluorphenyl)-3-trifluormethyl-4-methyltriazol-5-on analog Beispiel 1 hergestellt. Die Verbindung fiel als Öl an. ¹H-NMR (CDCl₃) δ [ppm] 7,2 (d), 2,50 (s, br), 2,2 (m), 1,85 (s, br), 1,35-1,2 (m).

### Beispiel 16:

### N-(4-Chlor-1-cyclopropyl-6-fluorbenzoxazol-7-yl)-4,5,6,7-tetrahydrophthalsäuredithioimid (Verb. I-C'a.419 aus Tabelle 1)

0,5 g N-(4-Chlor-1-cyclopropyl-6-fluorbenzoxazol-7-yl)-4,5,6,7-tetrahydrophthalimid wurden in 55 ml Toluol gelöst und mit 0,61 g Phosphorpentasulfid versetzt. Man erwärmte zum Rückfluss und behielt die Temperatur 46 h bei. Die Reaktionsmischung wurde im Vakumm zur Trockne eingeengt und an Kieselgel mit Cyclohexan/Essigester (100:1 bis 10:1 v/v) chromatographiert. Man erhielt so 0,8 g der Titelverbindung als Feststoff mit einem Festpunkt im Bereich von 132-135°C. ¹H-NMR (CDCl₃) δ [ppm] 7,1 (d, 1B), 2,50 (m 4H), 2,2 (m, 1H), 1,9 (m, 4H), 1,3-1,1 (m, 4H).

### Beispiel 17:

### 2-(4-Chlor-1-ethyl-6-fluorbenzoxazol-7-yl)-4-methyl-5-trifluormethylpyridazin-3-on (Verb. I-Ea.375 aus Tabelle 1)

8,9 g 2-(5-Amino-2-fluor-4-chlorphenyl)-4-methyl-5-trifluormethylpyridazin-3-on (aus WO 97/07104) wurden in 90 ml Trifluoressigsäure gelöst. Hierzu tropfte man bei 5 bis 10°C 3,0 g (0,029 mol) t-Butylnitrit. Man rührte 30 min bei 5 bis 10°C nach und gab dann 2.7 g (0.041 mol) Natriumazid portionsweise zu. Nach 1 h bei 5 bis 10°C rührte man weitere 3 h bei RT, gab die Reaktionsmidann 2.7 g (0.041 mol) Natriumazid portionsweise zu. Nach 1 h bei 5 bis 10°C rührte man weitere 3 h bei RT, gab die Reaktionsmischung auf 200 ml Eiswasser und extrahierte 3-mal mit Methylenchlorid. Die organische Phase wurde nacheinander mit Wasser, wäßriger 10 %-iger Natronlauge und nochmals mit Wasser gewaschen, über Natriumsulfat getrocknet und bei Raumtemperatur eingeengt. Man erhielt 9,2 g des Azids als hellen Feststoff, der direkt im Folgeschritt umgesetzt wurde. ¹H-NMR (CDCl₃) δ [ppm] 8,0 (s, 1 H), 7,4 (d, 1H), 7,3 (d, 1 H), 2,4 (t, 3 H).

1,63 g (0,0115 mol) P₄O₁₀ wurden 30 min bei 90°C in 6 ml Hexamethyldisiloxan gerührt und auf Raumtemperatur abgekühlt. Hierzu tropfte man eine Lösung von 1,6 g (0,0046 mol) des Azids aus der vorangegangenen Umsetzung in 20 ml Propionsäure und erwärmte die Mischung für 3 h auf 40°C. Man goss die Reaktionsmischung auf Eiswasser, stellte mit 10 %-iger, wässrige Natromlauge alkalisch und extrahiert die wässrigee Phase dreimal mit Ethylacetat. Die vereinigten organischen Phasen wurden mit Wasser gewaschen, eingeengt und an Kieselgel mit Cyclohexan/Essigester (6:1 v/v) chromatographiert. Man erhielt 0,28 g der Titelverbindung als zähes Öl.

In analoger Weise wurden die in Tabelle 4 angegebenen Verbindungen der Beispiele 18 bis 14 hergestellt.

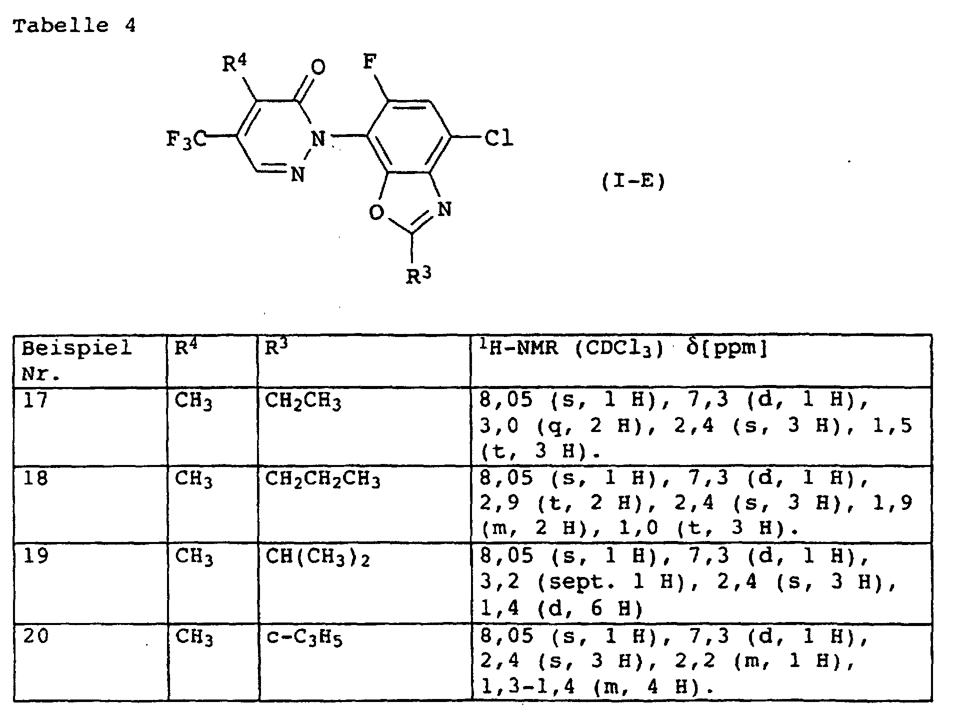

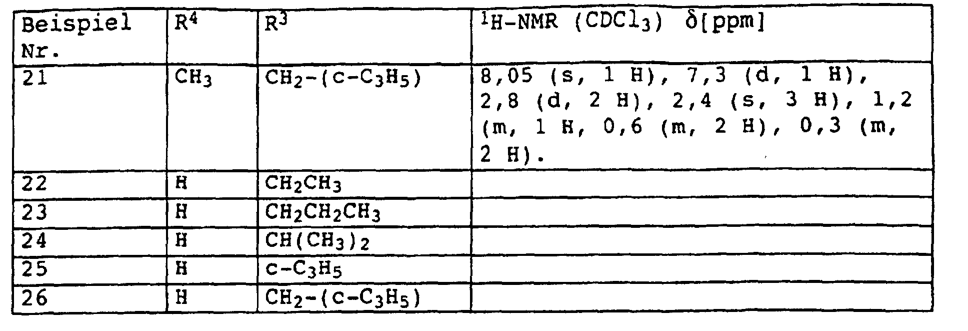

### Beispiel 27:

### 2-(4-Chlor-6-fluorbenzoxazol-7-yl)-4-amino-5-methylsulfonylpyridazin-3-on (Verb. I-E'a.373 aus Tabelle 1)

Analog zu der für Beispiel 17 beschriebenen Vorgehensweise wurde aus 2-(5-Amino-2-fluor-4-chlorphenyl)-4-methyl-5-trifluormethylpyridazin-3-on (aus WO 97/07104) die Titelverbindung hergestellt.
¹H-NMR (DMSO) δ [ppm] 9,0 (s, 1H), 8,3 (br. s, 1 H), 8,0 (s 1 H), 7,7 (br. s 1 H), 7,1 (d, 1 H).

### Beispiel 28:

### 3-(4-Chlor-1-cyclopropyl-6-fluorbenzoxazol-7-yl)-4,5-dihydro-5-(2-propyliden)-4-oxooxazol-2-on (Verb. I-Da.419 aus Tabelle 1)

Analog zu der für Beispiel 1 beschriebenen Vorgehensweise wurde aus 2-(2,6-Difluor-4-chlorphenyl)-4,5-dihydro-5-(2-propyliden)-4-oxooxazol-2-on die Titelverbindung hergestellt. Die Titelverbindung fiel als zähes Öl an.

Die Verbindungen der Formel I und deren landwirtschaftlich brauchbaren Salze eignen sich sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierten Benzoxazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden. Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:
- I: 20 Gewichtsteile des Wirkstoffs aus Beispiel 1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
- II: 20 Gewichtsteile des Wirkstoffs aus Beispiel 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III: 20 Gewichtsteile des Wirkstoffs aus Beispiel3 wurden in einer Mischung gelöst, die zu 25 Gewichtsteilen Cyclohexanon, 65 Gewichtsteilen einer Mineralölfration vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wässrige Dispersion, die 0,02 Gew.-% des Wirkstoffs enthält.
- IV: 20 Gewichtsteile des Wirkstoffs aus Beispiel4 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.
- V: 3 Gewichtsteile des Wirkstoffs aus Beispiel 1 werden mit 97 Gewichtsteilen feinteiligem Kaolin vermischt. Man erhält auf diese Weise ein Stäubemittel, das 3 Gew.-% des Wirkstoffs enthält.
- VI: 20 Gewichtsteile des Wirkstoffs aus Beispiel 5 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
- VII: 1 Gewichtsteil des Wirkstoffs aus Beispiel 6 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil des Wirkstoffs aus Beispiel 1 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die substituierten Benzoxazole I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren.Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden. Die Aufwandmengen an Wirkstoff betragen je nach Bekämpfungsziel, Jahreszeit, Zielpflanzen und Wachstumsstadium 0,001 bis 3, vorzugsweise 0,01 bis 1,0 kg/ha aktive Substanz (a. S.).

### Anwendungsbeispiele

Die herbizide Wirkung der erfindungsgemäßen Verbindungen der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 31,2, 15,6 oder 7,81 g/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Bayercode | Deutscher Name | Englischer Name |
|---|---|---|
| AMARE | Zurückgekrümmter Fuchsschwanz | redroot pigweed |
| POLPE | Flohknöterich | lady's thumb |
| ABUTH | lindenblättrige Schönmalve | velvet leaf |

Die folgenden Untersuchungen wurden im Nachlaufverfahren durchgeführt.

Die Verbindung aus Beispiel 27 (Verbindung I-E'a.373 aus Tabelle 1) zeigte eine sehr gute Herbizid-Wirkung gegen ABUTH, AMARE und POLPE bei Aufwandmengen von 31,2 und 15,6 g/ha a.S.

Die Verbindung aus Beispiel 15 (Verbindung I-Ca.419 aus Tabelle 1) zeigte eine sehr gute Herbizid-Wirkung gegen ABUTH, AMARE und POLPE bei Aufwandmengen von 15,6 und 7,81 g/ha a.S.

Die Verbindung aus Beispiel 17 (Verbindung Nr. I-Ea.375) zeigte eine sehr gute Herbizid-Wirkung gegen ABUTH, AMARE und POLPE bei Aufwandmengen von 15,6 und 7,81 g/ha a.S.

Die Verbindung aus Beispiel 13 (Verbindung Nr. I-Ba.419 zeigte eine gute bis sehr gute Herbizid-Wirkung gegen ABUTH, AMARE und POLPE bei Aufwandmengen von 31,2 und 15,6 g/ha a.S.

## Patentansprüche

1. Substituierte Benzoxazole der allgemeinen Formel I 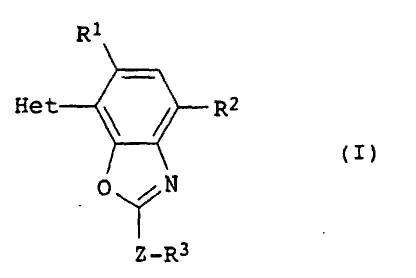 worin
Z eine chemische Bindung, O oder S bedeutet,
R¹ für Wasserstoff oder Halogen,
R² für Halogen, Cyano, C₁-C₆-Alkyl, C₁-C₆-Halogenalkyl, C₁-C₆-Alkoxy oder C₁-C₆-Halogenalkoxy, und
R³ für C₁-C₆-Alkyl, C₁-C₆-Halogenalkyl, Hydroxy-C₁-C₄-alkyl, Cyano-C₁-C₄-alkyl, C₁-C₄-Alkoxy-C₁-C₄-alkyl, C₁-C₄-Halogenalkoxy-C₁-C₄-alkyl, C₃-C₄-Alkenyloxy-C₁-C₄-alkyl, C₃-C₄-Alkinyloxy-C₁-C₄-alkyl, C₃-C₈-Cycloalkoxy-C₁-C₄-alkyl,
Amino-C₁-C₄-alkyl, C₁-C₄-Alkylamino-C₁-C₄-alkyl, Di(C₁-C₄-alkyl)amino-C₁-C₄-alkyl, C₁-C₄-Alkylthio-C₁-C₄-alkyl, C₁-C₄-Halogenalkylthio-C₁-C₄-alkyl, C₃-C₆-Alkenyl, Cyano-C₃-C₆-alkenyl, C₃-C₆-Halogenalkenyl, C₃-C₆-Alkinyl, Cyano-C₃-C₆-alkinyl, C₃-C₆-Halogenalkinyl, Hydroxycarbonyl-C₁-C₄-alkyl, (C₁-C₄-Alkoxy)carbonyl-C₁-C₄-alkyl, (C₁-C₄-Alkylthio)carbonyl-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl, Di(C₁-C₄-alkyl)phosphonyl-C₁-C₄-alkyl;
Phenyl, Phenyl-C₁-C₄-alkyl, worin die Phenylringe gegebenenfalls einen, zwei oder drei Substituenten tragen, ausgewählt unter Halogen, Cyano, C₁-C₆-Alkyl, C₁-C₆-Halogenalkyl, C₁-C₆-Alkoxy oder C₁-C₆-Halogenalkoxy;
C₃-C₈-Cycloalkyl, C₃-C₈-Cycloalkyl-C₁-C₄-alkyl, 3- bis 7-9liedriges Heterocyclyl, 3- bis 7-gliedriges Heterocyclyl-C₁-C₄-alkyl, wobei Heterocyclyl ein, zwei oder drei Heteroatome, ausgewählt unter Sauerstoff-, Schwefel- und Stickstoff-Atomen enthält,
wobei jeder Cycloalkyl- und jeder Heterocyclyl-Ring ein Carbonyl- oder Thiocarbonyl-Ringglied enthalten kann, und worin jeder Cycloalkyl- und Heterocyclyl-Ring unsubstituiert sein oder ein, zwei, drei oder vier Substituenten tragen kann, jeweils ausgewählt unter Cyano, Nitro, Amino, Hydroxy, Halogen, C₁-C₄-Alkyl, C₁-C₄-Halogenalkyl, C₁-C₄-Cyanoalkyl, C₁-C₄-Hydroxyalkyl, C₁-C₄-Aminoalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy, Di(C₁-C₄-alkyl)amino, C₃-C₆-Alkenyl, C₃-C₆-Alkinyl, C₃-C₄-Alkenyloxy, C₃-C₄-Alkenylthio, C₃-C₄-Alkinyloxy und C₃-C₄-Alkinylthio; stehen,
oder, sofern Z eine chemische Bindung bedeutet, R³ gewünschtenfalls auch für Wasserstoff, Hydroxy, Cyano, Mercapto, Amino, C₁-C₄-Alkylamino, Di-C₁-C₄-alkylamino, gesättigtes, 5- oder 6-gliedriges, N-gebundenes Stickstoffheterocyclyl, C₃-C₆-Cycloalkylamino, Halogen, -(CH₂)ₙ-CH(OH)-CH₂-R⁹, -(CH₂)ₙ-CH(Halogen)-CH₂-R⁹, -(CH₂)ₙ-CH₂-CH(Halogen)-R⁹, -(CH₂)ₙ-CH=CH-R⁹ oder -(CH₂)ₙ-CH=C(Halogen)-R⁹ stehen kann, worin R⁹ Hydroxycarbonyl, (C₁-C₄-Alkoxy)carbonyl, (C₁-C₄-Alkylthio)carbonyl, Aminocarbonyl, (C₁-C₄-Alkylamino)carbonyl oder Di(C₁-C₄-alkyl)aminocarbonyl bedeutet und n für 0 oder 1 steht;
Het für einen ungesättigten, fünf- oder sechsgliedrigen, heterocylischen Rest, der mit dem Benzoxazolteil über ein Stickstoffatom verknüpft ist und der ausgewählt ist unter Resten der allgemeinen Formeln II-1 bis II-18, 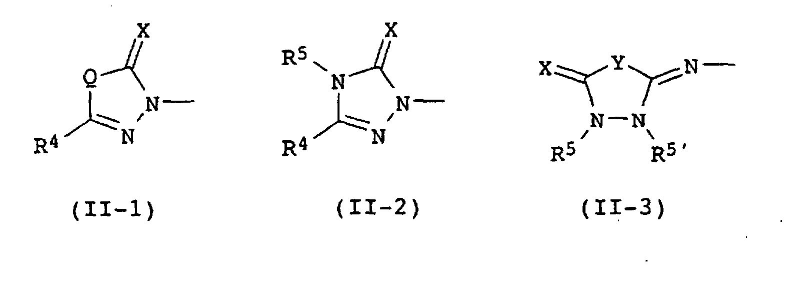 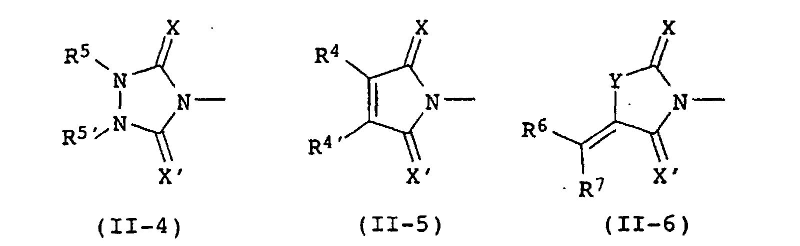 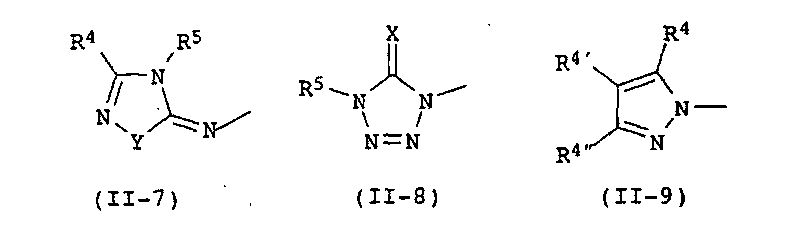 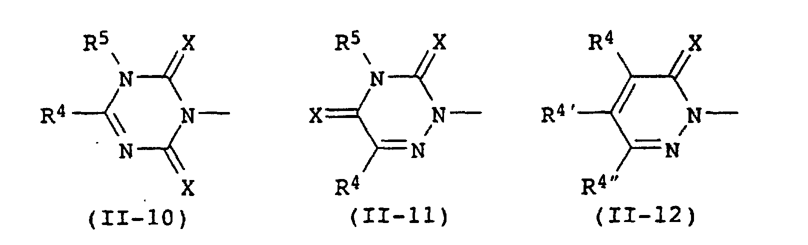 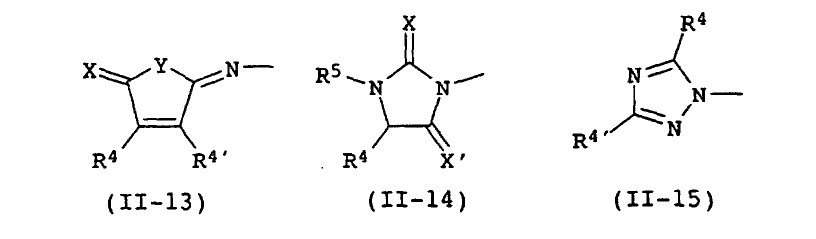 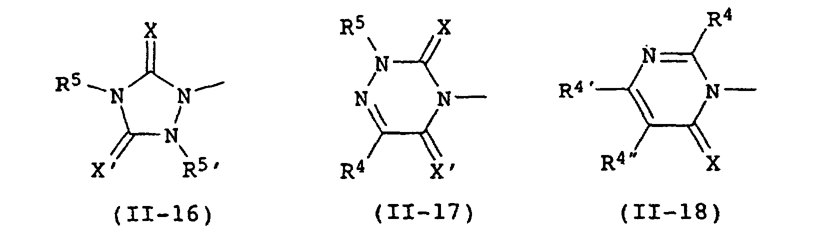
worin
R⁴, R^{4'} und R^{4"} unabhängig voneinander Wasserstoff, Halogen, Cyano, Amino, C₁-C₄-Alkylamino, Di-C₁-C₄-alkylamino, gesättigtes, 5- oder 6-gliedriges, N-gebundenes Stickstoffheterocyclyl, C₃-C₆-Cycloalkylamino, Carboxyl, C₁-C₆-Alkyl, C₁-C₆-Halogenalkyl, C₃-C₆-Alkenyl, C₃-C₆-Alkinyl, C₁-C₆-Alkoxy, C₁-C₆-Halogenalkoxy, C₁-C₆-Halogenalkylthio, C₁-C₆-Alkoxycarbonyl, C₃-C₆-Alkenyloxy, C₃-C₆-Alkinyloxy, C₁-C₆-Alkylthio, C₁-C₆-Alkylsulfinyl, C₁-C₆-Alkylsulfonyl, C₃-C₈-Cycloalkyl oder C₃-C₈-Cycloalkyl-C₁-C₄-alkyl bedeuten;
R⁵ und R^{5'} unabhängig voneinander Wasserstoff, Amino, Hydroxy, Cyano, C₁-C₆-Alkyl, C₁-C₆-Halogenalkyl, C₃-C₆-Alkenyl, C₃-C₆-Alkinyl, C₁-C₆-Alkoxycarbonyl, C₃-C₈-Cycloalkyl, C₃-C₈-Cycloalkyl-C₁-C₄-alkyl, Phenyl oder Phenyl-C₁-C₆-alkyl bedeuten;
und/oder zwei der Reste R⁴, R^{4'}, R^{4"}, R⁵ und R^{5'} gemeinsam mit dem Cyclus an den sie gebunden sind, einen 4-, 5-, 6-oder 7-gliedrigen Ring bilden, der gesättigt oder ungesättigt sein kann, der ein oder zwei Sauerstoff- und/oder Schwefelatome als Ringlied enthalten kann, und/oder der durch ein, zwei oder drei Reste ausgewählt unter C₁-C₄-Alkyl und Halogen substituiert sein kann;
R⁶ und R⁷ unabhängig voneinander Wasserstoff, C₁-C₆-Alkyl, C₁-C₆-Halogenalkyl oder C₃-C₆-Cycloalkyl bedeuten, oder gemeinsam mit dem C-Atom, an das sie gebunden sind, einen 4-, 5-, 6- oder 7-gliedrigen Ring bilden, der gesättigt oder ungesättigt sein kann, der ein oder zwei Sauerstoffund/oder Schwefelatome als Ringlied enthalten kann, und/oder durch ein, zwei oder drei Substituenten, ausgewählt unter C₁-C₄-Alkyl und Halogen substituiert sein kann;
Q in Formel II-1 für O oder S steht,
X und X' unabhängig voneinander für O oder S stehen, und
Y O, S oder eine Gruppe N-R⁸ bedeutet, worin R⁸ für Wasserstoff, C₁-C₆-Alkyl, C₁-C₆-Halogenalkyl oder C₃-C₈-Cycloalkyl steht;
sowie die landwirtschaftlich brauchbaren Salze der Verbindungen der Formel I.

2. Substituierte Benzoxazole nach Anspruch 1, worin Het für einen Stickstoffhetercyclus steht, der wenigstens eine Carbonyl- oder Thiocarbonylfunktion aufweist und der ausgewählt ist unter den Resten der allgemeinen Formel II-1, II-2, II-5, II-6 und II-12.

3. Substituierte Benzoxazole nach Anspruch 2, worin Het einen Rest der allgemeinen Formel II-1 bedeutet, in der X und Q für Sauerstoff und R⁴ für C₁-C₆-Alkyl stehen.

4. Substituierte Benzoxazole nach Anspruch 2, worin Het einen Rest der allgemeinen Formel II-2 bedeutet, in der X für Sauerstoff steht, R⁵ für Wasserstoff oder C₁-C₄-Alkyl und R⁴ für C₁-C₄-Halogenalkyl stehen.

5. Substituierte Benzoxazole nach Anspruch 2, worin Het einen Rest der allgemeinen Formel II-5 bedeutet, in der X und X' für Sauerstoff stehen, R⁴ und R^{4'} unabhängig voneinander Wasserstoff oder C₁-C₄-Alkyl bedeuten oder mit den Kohlenstoffatomen an die sie gebunden sind einen 6-gliedrigen Carbocyclus bilden.

6. Substituierte Benzoxazole nach Anspruch 2, worin Het einen Rest der allgemeinen Formel II-6 bedeutet, in der X, X' und Y für Sauerstoff stehen, R⁶ und R⁷ C₁-C₄-Alkyl bedeuten oder mit dem Kohlenstoffatom an das sie gebunden sind, einen 5- oder 6-gliedrigen, gesättigten Carbocyclus bilden.

7. Substituierte Benzoxazole nach Anspruch 2, worin Het einen Rest der allgemeinen Formel II-12 bedeutet, in der X in für Sauerstoff steht, R⁴ ausgewählt ist unter Wasserstoff, Amino und C₁-C₄-Alkyl, R^{4'} C₁-C₄-Halogenalkyl oder C₁-C₄-Alkylsulfonyl bedeutet und R^{4"} für Wasserstoff steht.

8. Substituierte Benzoxazole nach einem der vorhergehenden Ansprüche, worin R¹ für Wasserstoff, Fluor oder Chlor und R² für Halogen oder Cyano stehen.

9. Substituierte Benzoxazole nach einem der vorhergehenden Ansprüche, worin
R³ für C₁-C₄-Alkyl, C₁-C₄-Halogenalkyl, Hydroxy-C₁-C₄-alkyl, Cyano-C₁-C₄-alkyl, C₁-C₄-Alkoxy-C₁-C₄-alkyl, Amino-C₁-C₄-alkyl, C₁-C₄-Alkylamino-C₁-C₄-alkyl, Di(C₁-C₄-alkyl)amino-C₁-C₄-alkyl, C₃-C₆-Alkenyl, C₃-C₆-Alkinyl, Hydroxycarbonyl-C₁-C₄-alkyl, (C₁-C₄-Alkoxy)carbonyl-C₁-C₄-alkyl, (C₁-C₄-Alkylthio)carbonyl-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl;
Phenyl, das gegebenenfalls einen Substituenten trägt, der ausgewählt ist unter Halogen, Cyano, C₁-C₆-Alkyl, C₁-C₆-Halogenalkyl, C₁-C₆-Alkoxy oder C₁-C₆-Halogenalkoxy;
gesättigtes C₃-C₇-Cycloalkyl, 3- bis 7-gliedriges, gesättigtes Heterocyclyl, das ein, zwei oder drei Heteroatome, ausgewählt unter Sauerstoff- und Schwefel-Atomen enthält, wobei jeder Cycloalkyl- und jeder Heterocyclyl-Ring ein Carbonyl- oder Thiocarbonyl-Ringglied enthalten kann; steht,
oder, sofern Z eine chemische Bindung bedeutet, R³ gewünschtenfalls auch für Wasserstoff, Hydroxy, Cyano, Mercapto, Amino, C₁-C₄-Alkylamino, Di-C₁-C₄-alkylamino, gesättigtes, 5- oder 6-gliedriges, N-gebundenes Stickstoffheterocyclyl, C₃-C₆-Cycloalkylamino, Halogen, -(CH₂)ₙ-CH(OH)-CH₂-R⁹ , -(CH₂)ₙ-CH(Halogen)-CH₂-R⁹, -(CH₂)ₙ-CH₂-CH(Halogen)-R⁹, -(CH₂)ₙ-CH=CH-R⁹ oder -(CH₂)ₙ-CH=C(Halogen)-R⁹ stehen kann, worin R⁹ Hydroxycarbonyl, (C₁-C₄-Alkoxy)carbonyl, (C₁-C₄-Alkylthio)carbonyl, Aminocarbonyl, (C₁-C₄-Alkylamino)carbonyl oder Di(C₁-C₄-alkyl)aminocarbonyl bedeutet und n für 0 oder 1 steht.

10. Verbindungen der allgemeinen Formeln III, IV und V, 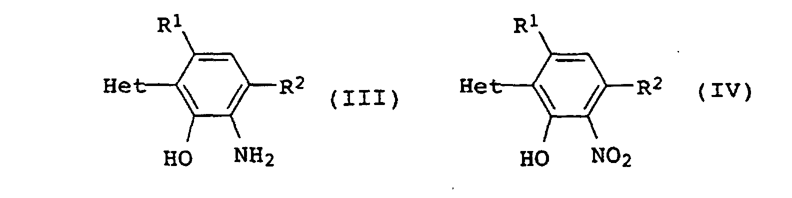 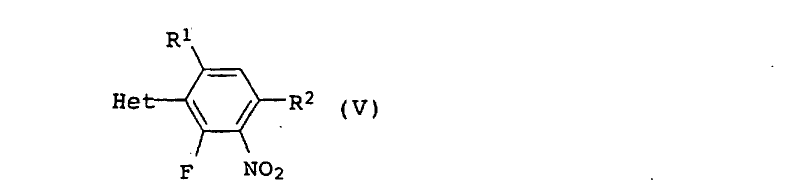 worin R¹, R² und Het die in Anspruch 1 angegebenen Bedeutungen haben.

11. Mittel, enthaltend mindestens ein substituiertes Benzoxazol der Formel I oder ein landwirtschaftlich brauchbares Salz von I gemäß einem der Ansprüche 1 bis 9, und übliche Hilfsmittel.

12. Verfahren zur Bekämpfung von unerwünschtem Pflanzenwuchs, **dadurch gekennzeichnet, daß** man eine herbizid wirksame Menge mindestens eines substituierten Benzoxazols der Formel I oder eines landwirtschaftlich brauchbaren Salzes von I gemäß einem der Ansprüche 1 bis 9, auf Pflanzen, deren Lebensraum und/oder auf Samen einwirken läßt.

13. Verwendung von substituierten Benzoxazolen der Formel I oder deren landwirtschaftlich brauchbaren Salzen gemäß einem der Ansprüche 1 bis 9 als Herbizide.

14. Verbindung der Formel X 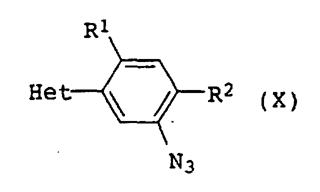 worin R¹, R² und Het die in Anspruch 1 genannten Bedeutungen haben.

## Claims

1. A substituted benzoxazole of the formula I 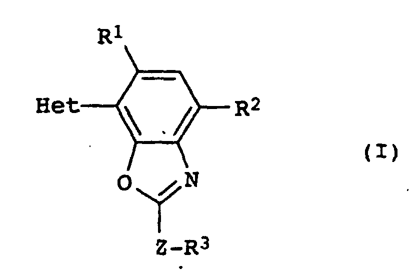 in which
Z is a chemical bond, O or S,
R¹ is hydrogen or halogen,
R² is halogen, cyano, C₁-C₆-alkyl, C₁-C₆-haloalkyl, C₁-C₆-alkoxy or C₁-C₆-haloalkoxy, and
R³ is C₁-C₆-alkyl, C₁-C₆-haloalkyl, hydroxy-C₁-C₄-alkyl, cyano-C₁-C₄-alkyl, C₁-C₄-alkoxy-C₁-C₄-alkyl, C₁-C₄-haloalkoxy-C₁-C₄-alkyl, C₃-C₄-alkenyloxy-C₁-C₄-alkyl, C₃-C₄-alkynyloxy-C₁-C₄-alkyl, C₃-C₈-cycloalkoxy-C₁-C₄-alkyl,
is amino-C₁-C₄-alkyl, C₁-C₄-alkylamino-C₁-C₄-alkyl, di(C₁-C₄-alkyl)amino-C₁-C₄-alkyl, C₁-C₄-alkylthio-C₁-C₄-alkyl,
C₁-C₄-haloalkylthio-C₁-C₄-alkyl, C₃-C₆-alkenyl, cyano-C₃-C₆-alkenyl, C₃-C₆-haloalkenyl, C₃-C₆-alkynyl, cyano-C₃-C₆-alkynyl, C₃-C₆-haloalkynyl, hydroxycarbonyl-C₁-C₄-alkyl, (C₁-C₄-alkoxy)carbonyl-C₁-C₄-alkyl, (C₁-C₄-alkylthio)carbonyl-C₁-C₄-alkyl, aminocarbonyl-C₁-C₄-alkyl, (C₁-C₄-alkylamino)carbonyl-C₁-C₄-alkyl, di(C₁-C₄-alkyl)aminocarbonyl-C₁-C₄-alkyl, di(C₁-C₄-alkyl)phosphonyl-C₁-C₄-alkyl;
is phenyl, phenyl-C₁-C₄-alkyl, where the phenyl rings may carry one, two or three substituents selected from the group consisting of halogen, cyano, C₁-C₆-alkyl, C₁-C₆-haloalkyl, C₁-C₆-alkoxy and C₁-C₆-haloalkoxy;
is C₃-C₈-cycloalkyl, C₃-C₈-cycloalkyl-C₁-C₄-alkyl, 3- to 7-membered heterocyclyl, 3- to 7-membered heterocyclyl-C₁-C₄-alkyl, where heterocyclyl contains one, two or three hetero atoms selected from oxygen, sulfur and nitrogen atoms,
where each cycloalkyl and each heterocyclyl ring may contain a carbonyl or thiocarbonyl ring member, and where each cycloalkyl and heterocyclyl ring may be unsubstituted or may carry one, two, three or four substituents, in each case selected from the group consisting of cyano, nitro, amino, hydroxyl, halogen, C₁-C₄-alkyl, C₁-C₄-haloalkyl, C₁-C₄-cyanoalkyl, C₁-C₄-hydroxyalkyl, C₁-C₄-aminoalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-haloalkylsulfinyl, C₁-C₄-alkylsulfonyl, C₁-C₄-haloalkylsulfonyl, (C₁-C₄-alkoxy)carbonyl, (C₁-C₄-alkyl)carbonyl, (C₁-C₄-haloalkyl)carbonyl, (C₁-C₄-alkyl)carbonyloxy, (C₁-C₄-haloalkyl)carbonyloxy, di(C₁-C₄-alkyl)amino, C₃-C₆-alkenyl, C₃-C₆-alkynyl, C₃-C₄-alkenyloxy, C₃-C₄-alkenylthio, C₃-C₄-alkynyloxy and C₃-C₄-alkynylthio;
or, if Z is a chemical bond, R³ may also, if desired, be hydrogen, hydroxyl, cyano, mercapto, amino, C₁-C₄-alkylamino, di-C₁-C₄-alkylamino, saturated, 5- or 6-membered, N-bonded nitrogen heterocyclyl, C₃-C₆-cycloalkylamino, halogen, -(CH₂)ₙ-CH(OH)-CH₂-R⁹, -(CH₂)ₙ-CH(halo)-CH₂-R⁹, -(CH₂)ₙ-CH₂-CH(halo)-R⁹, -(CH₂)ₙ-CH=CH-R⁹ or-(CH₂)ₙ-CH=C(halo)-R⁹, in which R⁹ is hydroxycarbonyl, (C₁-C₄-alkoxy)carbonyl, (C₁-C₄-alkylthio)carbonyl, aminocarbonyl, (C₁-C₄-alkylamino)carbonyl or di(C₁-C₄-alkyl)aminocarbonyl and n is 0 or 1;
Het is an unsaturated, five- or six-membered heterocyclic radical which is attached to the benzoxazole moiety via a nitrogen atom and which is selected from radicals of the. formulae II-1 to II-18 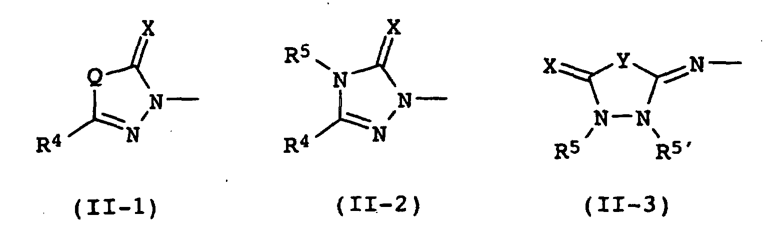 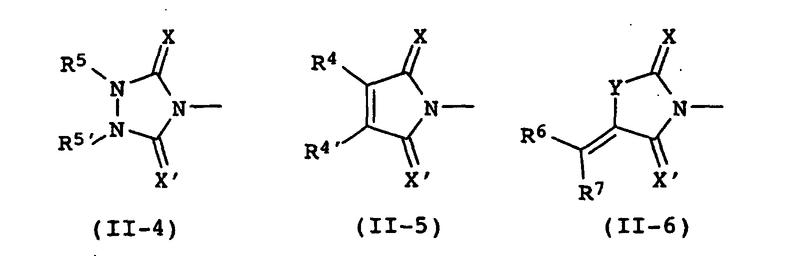 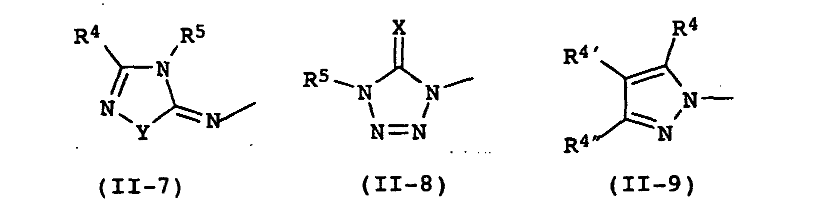 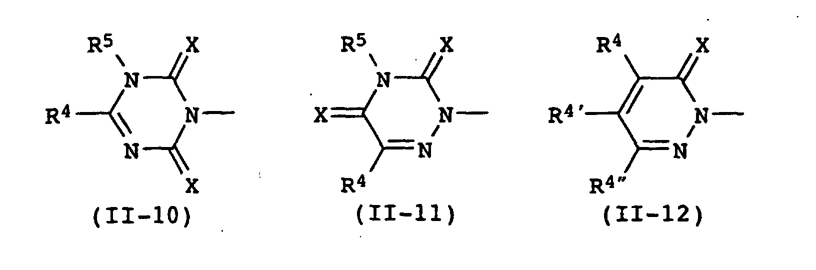 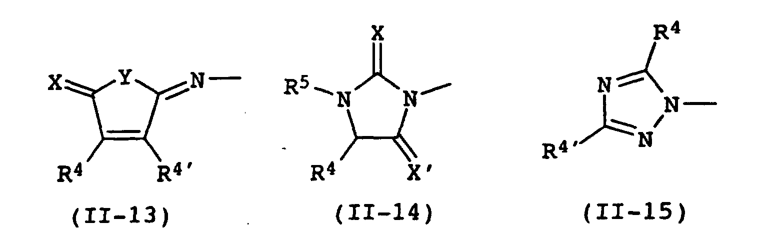 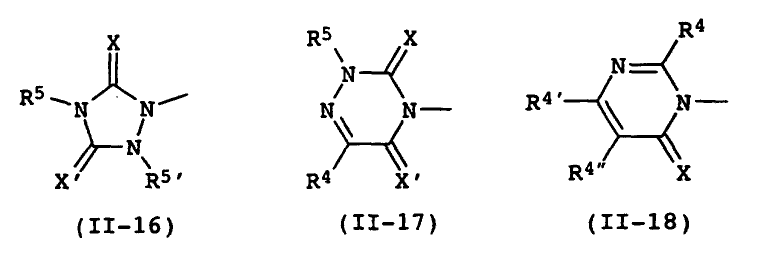
in which
R⁴, R^{4'} and R^{4"} independently of one another are hydrogen, halogen, cyano, amino, C₁-C₄-alkylamino, di-C₁-C₄-alkylamino, saturated, 5- or 6-membered N-bonded nitrogen heterocyclyl, C₃-C₆-cycloalkylamino, carboxyl, C₁-C₆-alkyl, C₁-C₆-haloalkyl, C₃-C₆-alkenyl, C₃-C₆-alkynyl, C₁-C₆-alkoxy, C₁-C₆-haloalkoxy, C₁-C₆-haloalkylthio, C₁-C₆-alkoxycarbonyl, C₃-C₆-alkenyloxy, C₃-C₆-alkynyloxy, C₁-C₆-alkylthio, C₁-C₆-alkylsulfinyl, C₁-C₆-alkylsulfonyl, C₃-C₈-cycloalkyl or C₃-C₈-cycloalkyl-C₁-C₄-alkyl;
R⁵ and R^{5'} independently of one another are hydrogen, amino, hydroxyl, cyano, C₁-C₆-alkyl, C₁-C₆-haloalkyl, C₃-C₆-alkenyl, C₃-C₆-alkynyl, C₁-C₆-alkoxycarbonyl, C₃-C₈-cycloalkyl, C₃-C₈-cycloalkyl-C₁-C₄-alkyl, phenyl or phenyl-C₁-C₆-alkyl;
and/or two of the radicals R⁴, R^{4'}, R^{4"}, R⁵ and R^{5'} together with the cycle to which they are attached form a 4-, 5-, 6- or 7-membered ring which may be saturated or unsaturated, which may contain one or two oxygen and/or sulfur atoms as ring members and/or which may be substituted by one, two or three radicals selected from the group consisting of C₁-C₄-alkyl and halogen;
R⁶ and R⁷ independently of one another are hydrogen, C₁-C₆-alkyl, C₁-C₆-haloalkyl or C₃-C₆-cycloalkyl, or together with the carbon atom to which they are attached form a 4-, 5-, 6- or 7-membered ring which may be saturated or unsaturated, which may contain one or two oxygen and/or sulfur atoms as ring members and/or which may be substituted by one, two or three substituents selected from the group consisting of C₁-C₄-alkyl and halogen;
Q in the formula II-1 is O or S,
X and X' independently of one another are O or S, and
Y is O, S or a group N-R⁸ in which R⁸ is hydrogen, C₁-C₆-alkyl, C₁-C₆-haloalkyl or C₃-C₈-cycloalkyl;
and the agriculturally useful salts of the compound of the formula I.

2. A substituted benzoxazole as claimed in claim 1 in which Het is a nitrogen heterocycle which has at least one carbonyl or thiocarbonyl function and which is selected from the radicals of the formulae II-1, II-2, II-5, II-6 and II-12.

3. A substituted benzoxazole as claimed in claim 2 in which Het is a radical of the formula II-1 in which X and Q are oxygen and R⁴ is C₁-C₆-alkyl.

4. A substituted benzoxazole as claimed in claim 2 in which Het is a radical of the formula II-2 in which X is oxygen, R⁵ is hydrogen or C₁-C₄-alkyl and R⁴ is C₁-C₄-haloalkyl.

5. A substituted benzoxazole as claimed in claim 2 in which Het is a radical of the formula II-5 in which X and X' are oxygen, R⁴ and R^{4'} independently of one another are hydrogen or C₁-C₄-alkyl or together with the carbon atoms to which they are attached form a 6-membered carbocycle.

6. A substituted benzoxazole as claimed in claim 2 in which Het is a radical of the formula II-6 in which X, X' and Y are oxygen, R⁶ and R⁷ are C₁-C₄-alkyl or together with the carbon atom to which they are attached form a 5- or 6-membered, saturated carbocylce.

7. A substituted benzoxazole as claimed in claim 2 in which Het is a radical of the formula 11-12 in which X is oxygen, R⁴ is selected from among hydorgen, amino and C₁-C₄-alkyl, R^{4'} is C₁-C₄-haloalkyl or C₁-C₄-alkylsulfonyl and R⁴" is hydrogen.

8. A substituted benzoxazole as claimed in any of the preceding claims in which R¹ is hydrogen, fluorine or chlorine and R² is halogen or cyano.

9. A substituted benzoxazole according to any of the preceding claims in which
R³ is C₁-C₄-alkyl, C₁-C₄-haloalkyl, hydroxy-C₁-C₄-alkyl, cyano-C₁-C₄-alkyl, C₁-C₄-alkoxy-C₁-C₄-alkyl, amino-C₁-C₄-alkyl, C₁-C₄-alkylamino-C₁-C₄-alkyl, di(C₁-C₄-alkyl)amino-C₁-C₄-alkyl, C₃-C₆-alkenyl, C₃-C₆-alkynyl, hydroxycarbonyl-C₁-C₄-alkyl, (C₁-C₄-alkoxy)carbonyl-C₁-C₄-alkyl, (C₁-C₄-alkylthio)carbonyl-C₁-C₄-alkyl, aminocarbonyl-C₁-C₄-alkyl, (C₁-C₄-alkylamino)carbonyl-C₁-C₄-alkyl, di(C₁-C₄-alkyl)aminocarbonyl-C₁-C₄-alkyl;
is phenyl which may carry a substituent selected from the group consisting of halogen, cyano, C₁-C₆-alkyl, C₁-C₆-haloalkyl, C₁-C₆-alkoxy and C₁-C₆-haloalkoxy;
is saturated C₃-C₇-cycloalkyl, 3- to 7-membered saturated heterocyclyl which contains one, two or three hetero atoms selected from oxygen and sulfur atoms, where each cycloalkyl and each heterocyclyl ring may contain a carbonyl or thiocarbonyl ring member;
or, if Z is a chemical bond, R³ can also, if desired, be hydrogen, hydroxyl, cyano, mercapto, amino, C₁-C₄-alkylamino, di-C₁-C₄-alkylamino, saturated, 5- or 6-membered N-bonded nitrogen heterocyclyl, C₃-C₆-cycloalkylamino, halogen, -(CH₂)ₙ-CH(OH)-CH₂-R⁹ , -(CH₂)ₙ-CH(halo)-CH₂-R⁹, -(CH₂)ₙ-CH₂-CH(halo)-R⁹, -(CH₂)ₙ-CH=CH-R⁹ or -(CH₂)ₙ-CH=C(halo)-R⁹, in which R⁹ is hydroxycarbonyl, (C₁-C₄-alkoxy)carbonyl, (C₁-C₄-alkylthio)carbonyl, aminocarbonyl, (C₁-C₄-alkylamino)carbonyl or di(C₁-C₄-alkyl)aminocarbonyl and n is 0 or 1.

10. A compound of the formula III, IV or V 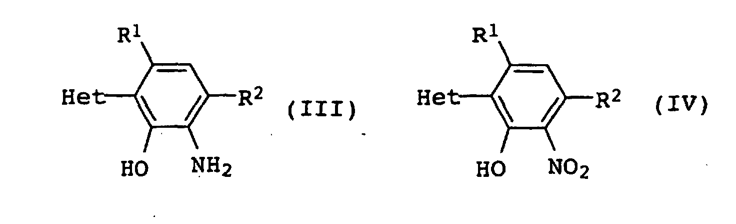 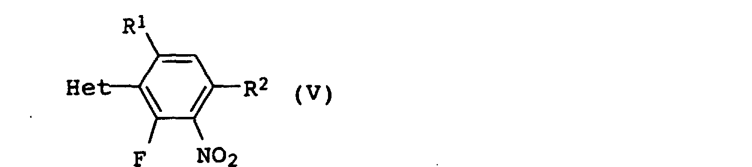 in which R¹, R² and Het are as defined in claim 1.

11. A composition comprising at least one substituted benzoxazole of the formula I or an agriculturally useful salt of I as claimed in any of claims 1 to 9, and customary auxiliaries.

12. A method for controlling undesirable vegetation, which comprises allowing a herbicidally effective amount of at least one substituted benzoxazole of the formula I or an agriculturally useful salt of I as claimed in any of claims 1 to 9 to act on plants, their habitat and/or on seeds.

13. The use of substituted benzoxazoles of the formula I or their agriculturally useful salts as claimed in any of claims 1 to 9 as herbicides.

14. A compound of the formula X 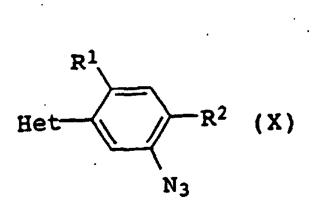 where R¹, R² and Het are as defined in claim 1.

## Revendications

1. Benzoxazoles substitués de formule générale I 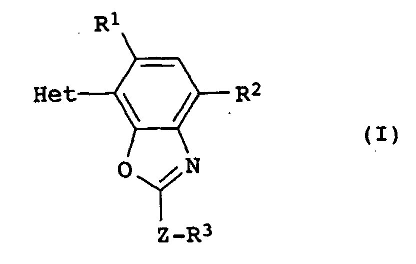 où
Z désigne une liaison chimique, O ou S,
R¹ représente l'hydrogène ou un halogéno,
R² désigne un groupe halogéno, cyano, alkyle en C₁-C₆, halogénoalkyle en C₁-C₆, alcoxy en C₁-C₆ ou halogénoalcoxy en C₁-C₆, et
R³ représente un groupe alkyle en C₁-C₆, halogénoalkyle en C₁-C₆, hydroxyalkyle en C₁-C₄, cyanoalkyle(C₁-C₄), alcoxy(C₁-C₄)-alkyle(C₁-C₄), halogénoalcoxy(C₁-C₄)-alkyle(C₁-C₄), alcényloxy-(C₃-C₄)-alkyle(C₁-C₄), alcynyloxy-(C₃-C₄)-alkyle(C₁-C₄), cycloalcoxy-(C₃-C₈)-alkyle-(C₁-C₄);
amino-alkyle en C₁-C₄, alkyl(C₁-C₄)amino-alkyle(C₁-C₄), di(alkyl en C₁-C₄)amino-alkyle(C₁-C₄), alkyl(C₁-C₄)thio-alkyle(C₁-C₄), halogénoalkyl(C₁-C₄)thio-alkyle(C₁-C₄), alcényle en C₃-C₆, cyano-alcényle(C₃-C₆), halogéno-alcényle(C₃-C₆), alcynyle en C₃-C₆, cyano-alcynyle(C₃-C₆), halogéno-alcynyle(C₃-C₆), hydroxycarbonyl-alkyle(C₁-C₄), (alcoxy en C₁-C₄)carbonyl-alkyle(C₁-C₄), (alkylthio en C₁-C₄)carbonyl-alkyle(C₁-C₄), aminocarbonyl-alkyle(C₁-C₄), (alkyl(C₁-C₄)amino)carbonyl-alkyle(C₁-C₄), di(alkyl en C₁-C₄)aminocarbonyl-alkyle(C₁-C₄), di(alkyl en C₁-C₄)phosphonyl-alkyle(C₁-C₄);
phényle, phényl-alkyle(C₁-C₄), tandis que les cycles phényle peuvent éventuellement porter un, deux ou trois substituants choisis parmi les groupes halogéno, cyano, alkyle en C₁-C₆, halogénoalkyle en C₁-C₆, alcoxy en C₁-C₆ ou halogénoalcoxy en C₁-C₆;
cycloalkyle en C₃-C₈, cycloalkyl(C₃-C₈)-alkyle(C₁-C₄), hétérocyclyle ayant 3 à 7 chaînons, (hétérocyclyle ayant 3 à 7 chaînons)-alkyle(C₁-C₄), tandis que le groupe hétérocyclyle contient un, deux ou trois hétéroatomes choisis parmi des atomes d'oxygène, de soufre et d'azote;
tandis que chaque cycle cycloalkyle et chaque cycle hétérocyclyle peut contenir un élément cyclique carbonyle ou thiocarbonyle, et tandis que chaque cycle cycloalkyle et hétérocyclyle est non-substitué ou peut porter un, deux, trois ou quatre substituants, dont chacun est choisi parmi les groupes cyano, nitro, amino, hydroxy, halogéno, alkyle en C₁-C₄, halogénoalkyle en C₁-C₄, cyanoalkyle(C₁-C₄), hydroxyalkyle en C₁-C₄, aminoalkyle en C₁-C₄, alcoxy en C₁-C₄, halogénoalcoxy en C₁-C₄, alkyl(C₁-C₄)thio, halogénoalkyl(C₁-C₄)thio, alkyl(C₁-C₄)sulfinyle, halogénoalkyl(C₁-C₄)-sulfinyle, alkyl(C₁-C₄)sulfonyle, halogéno-alkyl(C₁-C₄)sulfonyle, (alcoxy en C₁-C₄)-carbonyle, (alkyl en C₁-C₄) carbonyle, (halogénoalkyl en C₁-C₄)carbonyle, (alkyl en C₁-C₄)carbonyloxy, (halogénoalkyl en C₁-C₄)-carbonyloxy, di(alkyl en C₁-C₄)amino, alcényle en C₃-C₆, alcynyle en C₃-C₆, alcényl(C₃-C₄)oxy, alcényl(C₃-C₄)thio, alcynyl(C₃-C₄)oxy et alcynyl(C₃-C₄)thio;
ou, dans la mesure où Z représente une liaison chimique, R³ peut si on le souhaite désigner également un groupe hydrogène, hydroxy, cyano, mercapto, amino, alkyl(C₁-C₄)-amino, di(alkyl en C₁-C₄)-amino, hétérocyclyle azoté saturé, lié par l'azote et ayant 5 ou 6 chaînons, cycloalkyl(C₃-C₆)-amino, halogéno, -(CH₂)ₙ-CH(OH)-CH₂-R⁹, -(CH₂)ₙ-CH(halogéno)-CH₂-R⁹, -(CH₂)ₙ-CH₂-CH(halogéno)-R⁹, -(CH₂)ₙ-CH=CH-R⁹ ou -(CH₂)ₙ-CH=C(halogéno)-R⁹, tandis que R⁹ représente un groupe hydroxycarbonyle, (alcoxy en C₁-C₄)-carbonyle, (alkyl(C₁-C₄)thio)-carbonyle, aminocarbonyle, (alkyl(C₁-C₄)amino)-carbonyle ou di(alkyl en C₁-C₄)-aminocarbonyle, et n vaut 0 ou 1;
Het représente un reste hétérocyclique insaturé, ayant cinq ou six chaînons, qui est lié à la fraction benzoxazole par l'intermédiaire d'un atome d'azote et qui est choisi parmi les restes de formules générales II-1 à II-18, 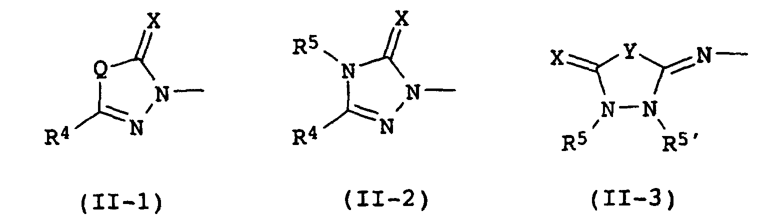 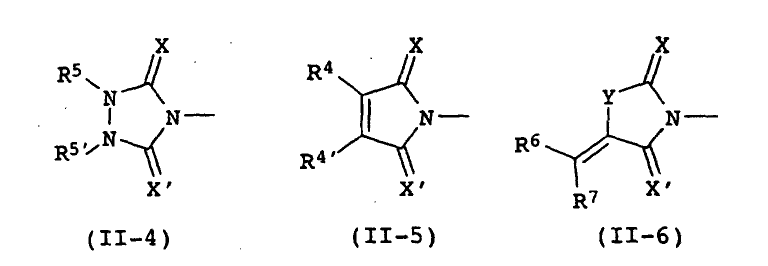 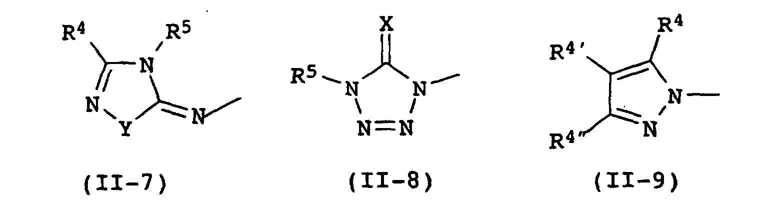 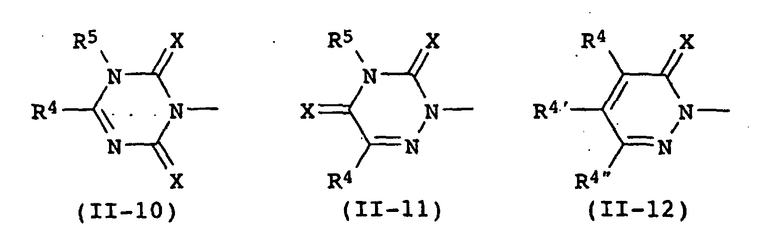 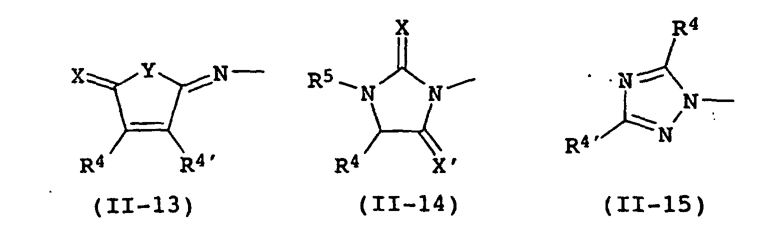 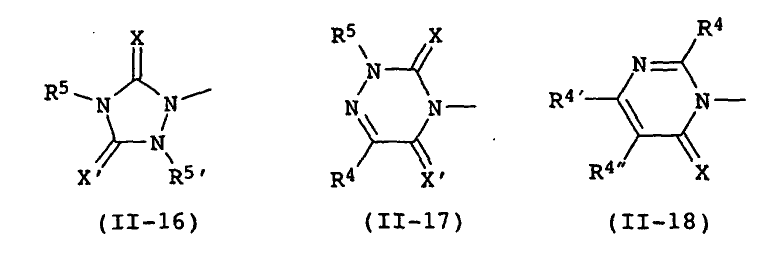
où
R⁴, R^{4'} et R^{4"} désignent, indépendamment l'un de l'autre, un groupe hydrogène, halogéno, cyano, amino, alkyl(C₁-C₄)amino, di(alkyl en C₁-C₄)amino, hétérocyclyle azoté saturé, lié par l'atome d'azote et ayant 5 ou 6 chaînons, cycloalkyl(C₃-C₆)amino, carboxyle, alkyle en C₁-C₆, halogénoalkyle en C₁-C₆, alcényle en C₃-C₆, alcynyle en C₃-C₆, alcoxy en C₁-C₆, halogénoalcoxy en C₁-C₆, halogénoalkyl(C₁-C₆)thio, alcoxy(C₁-C₆)carbonyle, alcényl(C₃-C₆)oxy, alcynyl(C₃-C₆)oxy, alkyl(C₁-C₆)thio, alkyl(C₁-C₆)sulfinyle, alkyl(C₁-C₆)sulfonyle, cycloalkyle en C₃-C₈ ou cycloalkyl(C₃-C₈)-alkyle(C₁-C₄) ;
R⁵ et R^{5'} désignent, indépendamment l'un de l'autre, un groupe hydrogène, amino, hydroxy, cyano, alkyle en C₁-C₆, halogénoalkyle en C₁-C₆, alcényle en C₃-C₆, alcynyle en C₃-C₆, alcoxy(C₁-C₆)carbonyle, cycloalkyle en C₃-C₈, cycloalkyl(C₃-C₈)-alkyle(C₁-C₄), phényle ou phényl-alkyle(C₁-C₆) ;
et/ou deux des restes R⁴, R^{4'}, R^{4"}, R⁵ et R^{5'} forment, conjointement avec le cycle auquel ils sont liés, un cycle à 4, 5, 6 ou 7 chaînons, qui peut être saturé ou insaturé, qui peut contenir comme élément du cycle un ou deux atomes d'oxygène et/ou de soufre, et/ou peut être substitué par un, deux ou trois substituants, choisis parmi les groupes alkyle en C₁-C₄ et halogéno;
Q dans la formule II-1 désigne O ou S;
X et X' représentent, indépendamment l'un de l'autre, O ou S, et
Y désigne O, S ou un groupe N-R⁸, dans lequel R8 représente un groupe hydrogène, alkyle en C₁-C₆, halogénoalkyle en C₁-C₆ ou cycloalkyle en C₃-C₈;
ainsi que les sels utilisables en agriculture des composés de formule I.

2. Benzoxazoles Substitués selon la revendication 1, dans lesquels Het représente un hétérocycle azoté, qui présente au moins une fonction carbonyle ou thiocarbonyle et qui est choisi parmi les restes de formules générales II-1, II-2, II-5, II-6 et II-12.

3. Benzoxazoles substitués selon la revendication 2, dans lesquels Het désigne un reste de formule générale II-1, où X et Q représentent l'oxygène et R⁴ représente un alkyle en C₁-C₆.

4. Benzoxazoles substitués selon la revendication 2, dans lesquels Het désigne un reste de formule générale II-2, où X représente l'oxygène, R⁵ représente l'hydrogène ou un alkyle en C₁-C₄ et R⁴ représente un halogénoalkyle en C₁-C₄.

5. Benzoxazoles substitués selon la revendication 2, dans lesquels Het désigne un reste de formule générale II-5, où X et X' représentent l'oxygène, R⁴ et R^{4'} représentent indépendamment l'un de l'autre l'hydrogène ou un alkyle en C₁-C₄ ou, conjointement avec les atomes de carbone auxquels ils sont liés, forment un carbocycle à 6 chaînons.

6. Benzoxazoles substitués selon la revendication 2, dans lesquels Het désigne un reste de formule générale II-6, où X, X' et Y représentent l'oxygène, R⁶ et R⁷ représentent un alkyle en C₁-C₄ ou, conjointement avec l'atome de carbone auquel ils sont liés, forment un carbocycle saturé, à 5 ou 6 chaînons.

7. Benzoxazoles substitués selon la revendication 2, dans lesquels Het désigne un reste de formule générale II-12, où X représente l'oxygène, R⁴ est choisi parmi l'hydrogène, un amino ou un alkyle en C₁-C₄, R^{4'} représente un halogénoalkyle en C₁-C₄ ou un alkyl(C₁-C₄)sulfonyle et R^{4"} représente l'hydrogène.

8. Benzoxazoles substitués selon l'une des revendications précédentes, dans lesquels R¹ désigne l'hydrogène, le fluor ou le chlore, et R² représente un groupe halogéno ou cyano.

9. Benzoxazoles substitués selon l'une des revendications précédentes, dans lesquels
R³ représente un groupe alkyle en C₁-C₄, halogénoalkyle en C₁-C₄, hydroxyalkyle en C₁-C₄, cyano-alkyle(C₁-C₄), alcoxy(C₁-C₄)-alkyle(C₁-C₄), amino-alkyle en C₁-C₄, alkyl(C₁-C₄)amino alkyle(C₁-C₄), di(alkyl en C₁-C₄)aminoalkyle(C₁-C₄), alcényle en C₃-C₆, alcynyle en C₃-C₆, hydroxycarbonyl-alkyle(C₁-C₄), (alcoxy en C₁-C₄)carbonyl-alkyle(C₁-C₄), (alkylthio en C₁-C₄)carbonyl-alkyle(C₁-C₄), aminocarbonylalkyle(C₁-C₄), (alkyl(C₁-C₄)amino)carbonylalkyle(C₁-C₄), di(alkyl en C₁-C₄)aminocarbonylalkyle(C₁-C₄);
phényle, qui porte éventuellement un substituant choisi parmi les groupes halogéno, cyano, alkyle en C₁-C₆, halogénoalkyle en C₁-C₆, alcoxy en C₁-C₆ ou halogénoalcoxy en C₁-C₆; cycloalkyle en C₃-C₇ saturé, hétérocyclyle saturé ayant 3 à 7 chaînons, qui contient un, deux ou trois hétéroatomes choisis parmi des atomes d'oxygène et de soufre, tandis que chaque cycle cycloalkyle et chaque cycle hétérocyclyle peut contenir un élément cyclique carbonyle ou thiocarbonyle;
ou, dans la mesure où Z représente une liaison chimique, R³ peut si on le souhaite désigner également un groupe hydrogène, hydroxy, cyano, mercapto, amino, alkyl(C₁-C₄)-amino, di(alkyl en C₁-C₄)-amino, hétérocyclyle azoté saturé, lié par l'azote et ayant 5 ou 6 chaînons, cycloalkyl(C₃-C₆)-amino, halogéno, -(CH₂)ₙ-CH(OH)-CH₂-R⁹, -(CH₂)ₙ-CH(halogéno)-CH₂-R⁹, - (CH₂)ₙ-CH₂-CH(halogéno)-R⁹, -(CH₂)ₙ-CH=CH-R⁹ ou -(CH₂)ₙ-CH=C(halogéno)-R⁹, tandis que R⁹ représente un groupe hydroxycarbonyle, (alcoxy en C₁-C₄)-carbonyle, (alkyl(C₁-C₄)thio)-carbonyle, aminocarbonyle, (alkyl(C₁-C₄)amino)-carbonyle ou di(alkyl en C₁-C₄)-aminocarbonyle, et n vaut 0 ou 1.

10. Composés de formules générales III, IV et V, 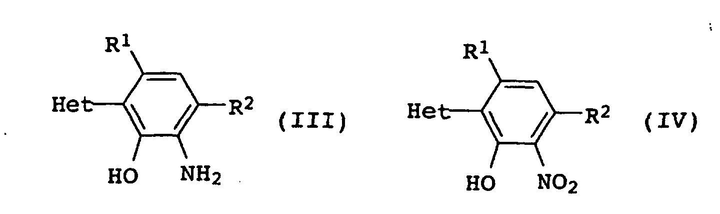 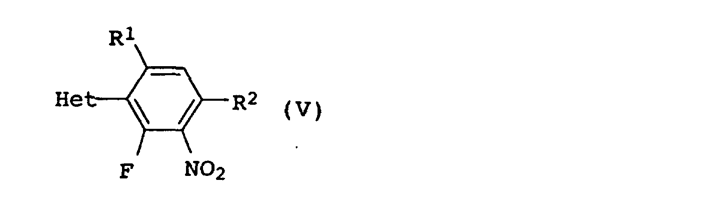 où R¹, R² et Het ont les significations indiquées dans la revendication 1.

11. Produit, contenant au moins un benzoxazole substitué de formule I ou un sel utilisable en agriculture de I selon l'une des revendications 1 à 9, et des adjuvants classiques.

12. Procédé pour la lutte contre la croissance non-souhaitée des plantes, **caractérisé par le fait qu'**on fait agir une quantité à efficacité herbicide d'au moins un benzoxazole substitué de formule I ou d'un sel utilisable en agriculture de I selon l'une des revendications 1 à 9, sur des plantes leur biotope et/ou des semences.

13. Utilisation de benzoxazoles substitués de formule I ou de leurs sels utilisables en agriculture selon l'une des revendications 1 à 9 comme herbicides.

14. Composé de formule X 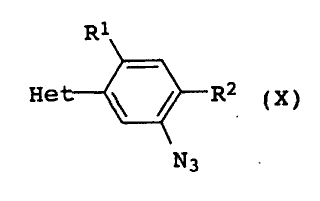 où R¹, R² et Het ont les significations indiquées dans la revendication 1.
